# EUROPÄISCHE PATENTSCHRIFT

(11) **EP 3 377 592 B1**
(45) Veröffentlichungstag und Bekanntmachung des Hinweises auf die Patenterteilung: **24.03.2021**
(21) Anmeldenummer: 16805323.9
(22) Anmeldetag: 16.11.2016
(51) Int. Cl.: C09K 11/06, G01N 33/00, G01N 33/22

(54) **FLUORESZIERENDE FARBSTOFFFILME ZUR DETEKTION VON EXPLOSIVSTOFFEN AUF NOX-BASIS IN DER LUFT IN LÖSUNGEN UND AUS WISCHPROBEN**
FLUORESCENT DYE FILMS FOR DETECTING NOX-BASED EXPLOSIVES IN THE AIR IN SOLUTIONS AND FROM WIPE SAMPLES
FILMS COLORÉS FLUORESCENTS POUR LA DÉTECTION DE SUBSTANCES EXPLOSIVES À BASE DE NOX DANS L'AIR DANS DES SOLUTIONS ET DES PRÉLÈVEMENTS PAR ESSUYAGE

(30) Priorität: 16.11.2015 DE 102015119765
(43) Veröffentlichungstag der Anmeldung: 26.09.2018
(73) Patentinhaber: Institut Dr. Foerster GmbH & Co. KG, 72766 Reutlingen (DE)
(72) Erfinder: BIYIKAL, Mustafa, 12053 Berlin (DE); RURACK, Knut, 12101 Berlin (DE)
(74) Vertreter: Zimmermann & Partner Patentanwälte mbB
(86) Internationale Anmeldenummer: PCT/EP2016/077888
(87) Internationale Veröffentlichungsnummer: WO 2017/085137

(56) Entgegenhaltungen:
- WO-A2-2008/140635
- JP-A- 2001 192 359
- JP-A- 2003 192 652
- US-A1- 2015 308 956
- YAN-DUO LIN ET AL: "meta versus para substituent effect of organic dyes for sensitized solar cells", JOURNAL OF PHOTOCHEMISTRY AND PHOTOBIOLOGY, A: CHEMISTRY, ELSEVIER SEQUOIA, LAUSANNE, CH, Bd. 222, Nr. 1, 29. Mai 2011 (2011-05-29), Seiten 192-202, XP028261388, ISSN: 1010-6030, DOI: 10.1016/J.JPHOTOCHEM.2011.05.022 [gefunden am 2011-06-03]
- DABIN SHI ET AL: "Synthesis, Structures, and Properties of Two Three-Dimensional Metal-Organic Frameworks, Based on Concurrent Ligand Extension", INORGANIC CHEMISTRY, Bd. 51, Nr. 12, 18. Juni 2012 (2012-06-18) , Seiten 6498-6506, XP055348260, EASTON, US ISSN: 0020-1669, DOI: 10.1021/ic202624e
- BRYCE W. DAVIS ET AL: "FRET Detection of Proteins Using Fluorescently Doped Electrospun Nanofibers and Pattern Recognition", LANGMUIR, Bd. 27, Nr. 10, 17. Mai 2011 (2011-05-17), Seiten 6401-6408, XP055348311, US ISSN: 0743-7463, DOI: 10.1021/la2006925
- ETIENNE J. DONCKELE ET AL: "The [2 + 2] Cycloaddition-Retroelectrocyclization and [4 + 2] Hetero-Diels-Alder Reactions of 2-(Dicyanomethylene)indan-1,3-dione with Electron-Rich Alkynes: Influence of Lewis Acids on Reactivity", ORGANIC LETTERS , 14(23), 6012-6015 CODEN: ORLEF7; ISSN: 1523-7052, Bd. 17, Nr. 14, 17. Juli 2015 (2015-07-17) , Seiten 3506-3509, XP055348318, ISSN: 1523-7060, DOI: 10.1021/acs.orglett.5b01598
- DONG-GYU CHO ET AL: "The Benzil-Cyanide Reaction and Its Application to the Development of a Selective Cyanide Anion Indicator", JOURNAL OF THE AMERICAN CHEMICAL SOCIETY, Bd. 130, Nr. 36, 10. September 2008 (2008-09-10), Seiten 12163-12167, XP055348321, US ISSN: 0002-7863, DOI: 10.1021/ja8039025
- JYE-SHANE YANG ET AL: "Zn(II)-Induced Ground-State [pi]-Deconjugation and Excited-State Electron Transfer in N,N -Bis(2-pyridyl)amino-Substituted Arenes", THE JOURNAL OF ORGANIC CHEMISTRY, Bd. 69, Nr. 10, 16. April 2004 (2004-04-16), Seiten 3517-3525, XP055348326, ISSN: 0022-3263, DOI: 10.1021/jo049902z
- DORA B BOGGIÁN ET AL: "Efficient alkene synthesis on solid support using the Julia-Kocienski coupling", MOLECULAR DIVERSITY, KLUWER ACADEMIC PUBLISHERS, DO, Bd. 14, Nr. 4, 17. April 2010 (2010-04-17) , Seiten 847-853, XP019864580, ISSN: 1573-501X, DOI: 10.1007/S11030-010-9251-8
- YANKE CHE ET AL: "Diffusion-Controlled Detection of Trinitrotoluene: Interior Nanoporous Structure and Low Highest Occupied Molecular Orbital Level of Building Blocks Enhance Selectivity and Sensitivity", JOURNAL OF THE AMERICAN CHEMICAL SOCIETY, Bd. 134, Nr. 10, 14. März 2012 (2012-03-14), Seiten 4978-4982, XP055348605, US ISSN: 0002-7863, DOI: 10.1021/ja300306e
- MUNGKARNDEE RADEEMADA ET AL: "Fluorescence sensor array for identification of commercial milk samples according to their thermal treatments", FOOD CHEMISTRY, ELSEVIER LTD, NL, vol. 197, 19 October 2015 (2015-10-19), pages 198-204, XP029312113, ISSN: 0308-8146, DOI: 10.1016/J.FOODCHEM.2015.10.083
- Sean P. Mcilroy ET AL: "Two-Photon Photosensitized Production of Singlet Oxygen:? Sensitizers with Phenylene-Ethynylene-Based Chromophores", The Journal of Organic Chemistry, vol. 70, no. 4, 1 February 2005 (2005-02-01), pages 1134-1146, XP055020669, ISSN: 0022-3263, DOI: 10.1021/jo0482099

## Beschreibung

Die Erfindung liegt auf dem Gebiet des Nachweises von zumindest eine NOx-Gruppe umfassenden Analyten und betrifft insbesondere den Nachweis von Explosivstoffen und Markersubstanzen für Explosivstoffe mit Hilfe von optisch vermessbaren Indikatorschichten.

Praxisrelevante Explosivstoffe und zu deren Markierung verwendete Markersubstanzen umfassen Verbindungen auf NOx-Basis. Für die Spuren-Analytik relevante Verbindungen sind beispielsweise TNT (2,4,6-Trinitrotoluol), DNT (2,4-Dinitrotoluol und 2,6-Dinitrotoluol), Tetryl (2,4,6-Trinitrophenylmethylnitramin), PETN (Nitropenta), NG (Nitroglycerin), EGDN (Ethylenglycoldinitrat), RDX (Hexahydro-1,3,5- trinitro-1,3,5-triazin), HMX (Octahydro-1,3,5,7-tetranitro-1,3,5,7-tetrazocin), NH₄NO₃ (Ammoniumnitrat) und DMDNB (2,3-Dimethyl-2,3-dinitrobutan - eine Markersubstanz). Im Bereich Sicherheit, Militär und Umwelt ist der Vor-Ort-Nachweis dieser Verbindungen von größter praktischer Bedeutung. Die meisten, derzeit auf dem Markt angebotenen Systeme zum Nachweis von Explosivstoffen basieren auf Ionen-Mobilitätsspektrometrie (IMS), Gaschromatographie (GC) bzw. Raman- und Infrarot- (IR) spektroskopischer Messtechnik. Kommerzielle Bedeutung haben vor allem IMS- (z.B. SABRE 4000, Smiths Detection/USA) und Raman-Geräte (z.B. FirstDefender™, Ahura /USA) erlangt. Weiterhin ist zum Explosivstoffnachweis die Verwendung chemischer Methoden auf der Basis von Chemilumineszenz-Assays oder molekular wechselwirkenden Sensoren, wie fluoreszierenden konjugierten Polymeren, bekannt als "amplifying fluorescent polymers" (AFPs), beschrieben worden. Andere für die Spuren-Analytik relevante Verbindungen auf NOx-Basis sind beispielsweise Pestizide, deren Rückstände und Abbauprodukte (Metabolite) Aus dem Stand der Technik sind z.B. aus der US2015/308956 triarylamin-basierte Nachweisreagenzien für NOx-Gruppen umfassende Analyten bekannt.

Neben dem Platzbedarf der typischerweise stationär arbeitenden und somit bestimmte Bedingungen voraussetzende Geräte haben die bekannten Verfahren weitere Nachteile:
(i) IMS basieren auf einer radioaktiven Quelle und weisen oft ein nachteiliges Driftverhalten auf.
(ii) GC-Techniken benötigen ein Trägergasreservoir.
(iii) Raman-Spektrometer setzen typischerweise einen Anschluss an das Stromnetz voraus, bzw. sind nicht batteriebetrieben und anfällig gegen unspezifische Fluoreszenz.
(iv) Laser-basierte Verfahren sind zumeist ebenso nicht batteriebetrieben und unterliegen häufig starken Matrixeffekten.

Vor diesem Hintergrund wird ein Nachweisreagenz nach Anspruch 1, ein Verfahren zum Nachweis eines eine NOx-Gruppe umfassenden Analyten nach Anspruch 7, ein Herstellungsverfahren für eine Analyt-sensitive Schicht nach Anspruch 12, eine Analyt-sensitive Schicht nach Anspruch 14 und die Verwendung eines Nachweisreagenz zur Überwachung eines Grenzwertes eines Explosivstoffes nach Anspruch 15 vorgeschlagen. Weitere Ausführungsformen, Modifikationen und Verbesserungen ergeben sich anhand der folgenden Beschreibung und der beigefügten Ansprüche.

Gemäß einem ersten Aspekt wird als Nachweisreagenz ein Farbstoff vorgeschlagen, dessen Grundstruktur ausgewählt ist unter einem 4-(Phenylethinyl)-phenyl-amin, einem 4-(Phenylethenyl)-phenyl-amin und/oder einem Biphenylamin-Derivat. Damit weist der als Nachweisreagenz für Nitroaromate, Nitroalkane, Nitroamine, Nitrate, Salpetersäure, salpetrige Säure, Stickoxide, sowie zusätzlich für Schwefeldioxid (das bei der Zersetzung von Schwarzpulver auftritt) einsetzbare Farbstoff eine Grundstruktur gemäß dem Formelbild 1 auf: 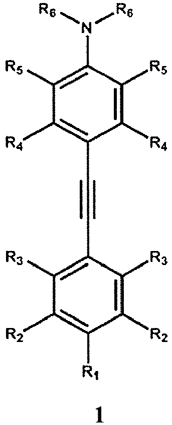
Dabei sind: R₁ = CO₂X oder PhCO₂X mit (X = Vinyl, Allyl, Homoallyl, Aryl) oder
R₁ = C(O)NX₂ oder PhC(O)NX₂ mit (X = H, Alkyl, Perfluoralkyl, Vinyl, Allyl, Homoallyl, Aryl);
R₂, R₃, R₄ und R₅ unabhängig voneinander = H, F, Alkyl, Aryl; und
R₆ = Aryl.

Gemäß bevorzugten Ausführungsformen steht R₁ für CO₂Me, für C(O)N(Me)₂, für C(O)N(*i*-Pr)₂, oder für PhC(O)N(Me)₂, insbesondere für PhC(O)N(*i*-Pr)₂. Beispielsweise ist weiterhin bevorzugt, wenn R₂-R₅ jeweils für H stehen, insbesondere in Kombination mit einer der vorstehend beschriebenen bevorzugten Ausführungsformen. Weiterhin ist bevorzugt, wenn R₆ für eine Phenyl-Gruppe steht, insbesondere in Kombination mit den zuvor beschriebenen bevorzugten Ausführungsformen. Weiterhin ist bevorzugt, wenn eine Triaryl-Gruppe mit der *para*-substituierten Phenyl-Gruppe über eine Dreifachbindung kovalent gebunden ist.

Besonders bevorzugt wird vorgeschlagen, zur Detektion von Nitroaromaten, Nitroalkanen, Nitroaminen, Nitraten, Salpetersäure, salpetriger Säure, nitrosen Gasen und Schwefeloxid vorgeschlagen, eine 4-(Phenylethinyl)-triphenylamin-Verbindung bzw. (Biphenylethinyl)-triphenylamin-Farbstoff gemäß den nachfolgend gezeigten Formeln 4, 5 oder 6 - also ein asymmetrisches Triphenylamin-Derivat einzusetzen: 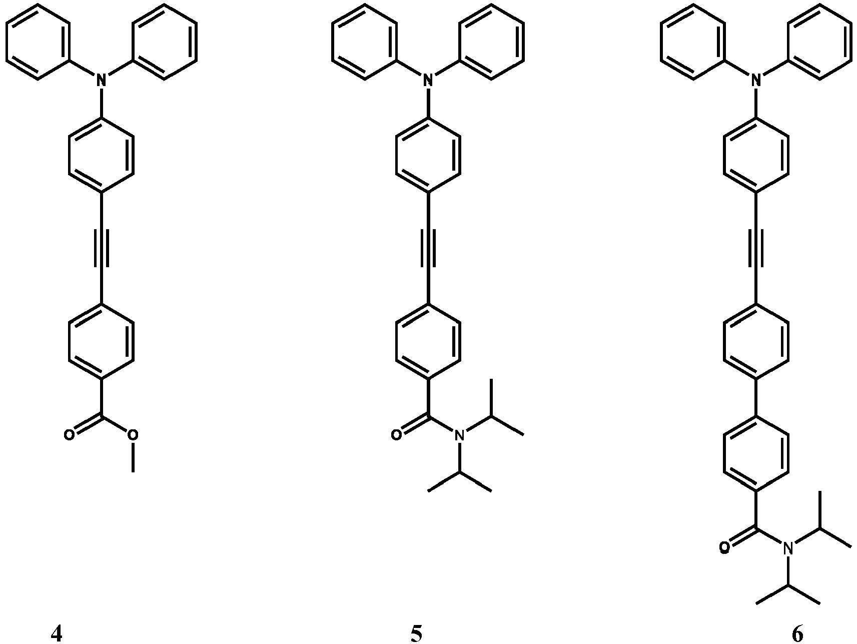 Dabei ist anzumerken, dass die Formel 4 nicht unter den Schutzumfang der Ansprüche fällt und alle Bezüge zu dieser Formel außerhalb des Schutzumfangs der Ansprüche stehen.

Eine der drei Triphenylamino-Gruppen dieser Nachweisreagenzien ist über eine Dreifachbindung mit einer Phenylgruppe kovalent gebunden, während zwei Phenylreste der Phenylamino-Gruppe unsubstituiert bleiben. Die über eine Dreifachbindung mit einer Phenylgruppe kovalent gebundene Triphenylamino-Gruppe wiederum ist bevorzugt in der *para*-Position mit einer elektronenziehenden Gruppe substituiert. Hieraus resultiert die angesprochene Asymmetrie der Verbindung, die zu einer hier analytisch genutzten Fluoreszenzlöschung bei Elektronenabstraktion führt.

Elektronenreiche Gruppen wie Amine (vgl. Formel 5) erhöhen die Elektronendichte im konjugierten System, verschieben das Emissionssignal einer betreffenden Verbindung in den nahen UV-Bereich und erhöhen zugleich deren Oxidations- bzw. Luftempfindlichkeit durch Luftsauerstoff. In Gegenwart polarer (d.h. gut mit Wasser mischbarer) Lösungsmittel, wie z.B. Acetonitril (ACN), ist ein Fluoreszenzsignal dieser Verbindungen in den längerwelligen Bereich verschoben, gegenüber einem Fluoreszenzsignal der gleichen Verbindung in einem Lösungsmittel von geringer Polarität (hoher Hydrophobizität, geringer Mischbarkeit mit Wasser) wie Hexan (vgl. Tab. 1, 2).

Bei stark elektronenziehenden Gruppen, wie z.B. bei einer Nitril-Gruppe (CN), nimmt die Elektronendichte am Triphenylamin-Stickstoff ab. Das hat zur Folge, dass die Energie des Akzeptors (wie den nachzuweisenden Explosivstoff) nicht zur Abstraktion eines Elektrons des Triphenylamin-Stickstoffs ausreicht und deshalb eine deutlich geringere Sensitivität des Indikators gegenüber den Explosivstoffen bzw. nur eine schwache Löschung des sonst bei geeigneter Anregung messbaren Fluoreszenzsignals zu beobachten ist.

Aus diesem Grund ist die elektronenziehende Gruppe R₁ für Verbindungen gemäß den Formeln 4 und 5, im weitesten Sinne also gemäß Strukturformel 1 vorteilhaft so ausgewählt,
dass die Abstraktion eines Elektrons des Triphenylamin-Stickstoffs in Gegenwart der NOx-Verbindungen energetisch begünstigt ist. Dadurch tritt bei Wechselwirkung des Farbstoffs mit dem eine NOx-Gruppe umfassenden Analyten eine Fluoreszenzlöschung auf. Das ermöglicht es, z.B. die benannten Explosivstoffe (und deren Markerstoffe) durch ein spektroskopisches Messverfahren qualitativ und - nach Kalibrierung im relevanten Temperatur- und Luftfeuchte-Bereich - quantitativ sicher zu detektieren. Unter den hier in Betracht kommenden Messverfahren ist die Kolorimetrie als eines der unempfindlichsten Formate für die Spurenanalytik eher ungeeignet. Hingegen sind fluoreszenzbasierte Messverfahren typischerweise mindestens um den Faktor 1000 empfindlicher. Aus diesem Grund wird nachfolgend diesem Messprinzip der Vorzug gegeben.

Hinzu kommt, dass eine spektroskopische Messung unter Verwendung einer flüssigen Phase einen störenden Einfluss des Lösungsmittels auf den Analyten ausschließen muss. Für die hier interessierenden Explosivstoffe (vorrangig aromatische Nitroverbindungen) ist die Ausbildung von selbst stark absorbierenden Meisenheimer-Komplexen des Lösungsmittels (z.B. DMF, ACN) mit dem Analyten (z.B. TNT) bekannt. Vor diesem Hintergrund wird nachfolgend weiterhin einem Festphasen-gestützten Nachweisverfahren der Vorzug gegeben.

Neben der Sensitivität gegenüber den eingangs genannten Explosivstoffen hat die elektronenziehende Gruppe R₁ des Indikators 1 in Verbindungen gemäß den Formeln 4, 5 und 6 auch einen starken Einfluss auf die molekulare Beweglichkeit der Fluoreszenzindikators, wenn diese an einer Phasengrenze Feststoff / Luft, z.B. an einer Polymer-Luftgrenze vorliegt. Vermutlich aggregieren die Moleküle an derartigen Phasengrenzen unter dem Einfluss der als mobile Phase wirkenden Luftfeuchtigkeit auf der als stationäre Phase wirkenden (Polymer-)Oberfläche, sodass ihre Fluoreszenz auf Grund von Selbstlöschung vermindert wird.

Werden die Indikatoren **4, 5** und **6** direkt auf ein zur Fluoreszenzmessung geeignetes Substrat, z.B. auf eine Glasoberfläche aufgetragen, ist die Intensität messbarer Emissionssignale nicht konstant, sondern fällt stetig ab. Ursache dafür scheint eine Selbstlöschung zu sein. Das Problem der Selbstlöschung wurde im Falle der vorbekannten AFPs mit Hilfe von sterisch anspruchsvollen Iptycen-Einheiten gelöst [3, 4], welche eine intermolekulare Wechselwirkungen zwischen apolaren konjugierten Polymeren auf polaren Oberflächen verhindern. Vorbekannten Konzepte basieren also auf der Verwendung eines fluoreszierenden (konjugierten) Polymers.

Gemäß einem zweiten Aspekt wird im Gegensatz zu diesem vorbekannten Ansatz hier vorgeschlagen, das Emissionssignal eines fluoreszierenden Moleküls, nämlich einer Verbindung **1, 4, 5** und/oder **6,** auf einem festen Substrat durch einen nicht fluoreszierenden Polymerfilm zu stabilisieren. Gemäß typischen praktischen Ausführungsformen erfolgt das, indem Polymerfilme mit einer angepassten Polarität und Schichtdicke, z.B. auf kommerziell erhältlichen Trägerglasmaterialien, beispielsweise auf Mikroskopie-Deckgläsern aufgebaut werden. Die Größe oder Stärke des gewählten Substrates kann der jeweils verwendeten Messanordnung, beispielsweise einem Handgerät, angepasst werden. Die Polarität, z.B. die Benetzbarkeit mit Wasser, kann dabei durch die Wahl funktioneller Gruppen des Polymers eingestellt werden. Die Schichtdicke wiederum kann in weiten Grenzen variiert werden. Bevorzugt wird zumindest eine einfache Moleküllage aufgebracht, wobei die Dicke der Polymerschicht von 1 nm bis 5 nm zur Detektion der schwerflüchtigen Explosivstoffe und von 1 nm bis 150 nm zur Detektion von flüchtigen Nitroverbindungen, wie dem Marker DMDNB, beträgt.

Gemäß einer typischen Ausführungsform wird vorgeschlagen, einen dünnen und nicht fluoreszierenden Polymerfilm mit einem Nachweisreagenz auszurüsten, das spezifisch mit einer nachzuweisenden Nitro-Verbindung wechselwirkt und dabei zumindest eine Fluoreszenzeigenschaft ändert. Der mit dem Nachweisreagenz versehene Polymerfilm ist zweckmäßig unter den Messbedingungen für schwerflüchtige Explosivstoffe beständig und auf einem hier als Substrat bezeichneten Festkörper, beispielsweise auf Glas, aufgebracht. In Gegenwart der NOx-Verbindung ändert sich eine fluoreszenzoptisch messbare Eigenschaft des Nachweisreagenz, bzw. des damit ausgerüsteten Polymerfilms. Insbesondere wird vorgeschlagen, dass das Nachweisreagenz ein fluoreszierender Farbstoff, umfassend ein mit zumindest einem Aryl verknüpftes tertiäres Amin gemäß den zuvor dargestellten Formeln ist. Demgemäß werden nachfolgend die Begriffe Nachweisreagenz, Farbstoff, (Fluoreszenz-) Indikator und molekulare Sonde im betreffenden Zusammenhang synonym verwendet. Eine auf einem Substrat angeordnete und mit zumindest einem der Nachweisreagenzien ausgerüstete Polymerschicht wird nachfolgend als Analyt-sensitive Schicht bezeichnet.

Für den Nachweis eines oder mehrerer schwerflüchtiger Explosivstoffe und/oder optional eines oder mehrerer der als Markerstoffe von Explosivstoffen eingesetzten Verbindungen oder eines anderen NOx-haltigen Analyten wird vorgeschlagen, den zeitlichen Verlauf einer Fluoreszenzlöschung des Nachweisreagenz zu bestimmen. Die Anwesenheit der Explosivstoffe und damit das Vorhandensein eines Gefährdungspotentials wird also an Hand einer Löschung eines Fluoreszenzsignals synchron zu einer reversiblen (physikalischchemischen) Wechselwirkung der Explosivstoffe (Markerstoffe) mit dem Nachweisreagenz und/oder an Hand einer erneuten Fluoreszenzzunahme bei Desorption des Explosivstoffs (Markerstoffs) nach vorheriger Löschung der Fluoreszenz des Nachweisreagenz (also während einer Regeneration) nachgewiesen. Messtechnisch erfasst wird hierzu ein zeitlicher Verlauf einer Fluoreszenzintensität in einem spezifischen Wellenlängenbereich.

Der NOx-haltige Analyt (Explosivstoff, Markerstoff, Pestizid) kann vorliegen in der Luft, auf der Oberfläche eines Gegenstandes (Gegenstandsoberfläche), in wässriger oder organischer Flüssigkeit oder dem Extrakt einer Probe, z.B. einer Bodenprobe. Auch thermisch induzierte Sublimation, z.B. nach Zersetzung des Analyten zu Stickoxiden, kann verfahrensgemäß zum spezifischen Nachweis dienen. Das Überschreiten einer kritischen Konzentration (Grenzwert) des Analyten in/auf einer Probe weist verfahrensgemäß auf eine Gefährdung hin. Hierzu ist verfahrensgemäß auch allein der qualitative Nachweis des NOx-Analyten nutzbar.

Von einer Gegenstandsoberfläche kann die Probe auf die Analyt-sensitive Schicht entweder direkt durch Strömung(-sübertragung) über von der Oberfläche abgegebene Explosiv- und /oder Markerstoffdämpfe übertragen werden, oder zuerst von der Oberfläche erfasst und mit Hilfe eines Übertragungsgegenstands auf die Analyt-sensitive Schicht appliziert werden. Letztgenanntes Prinzip ist als Wischprobenverfahren bekannt.

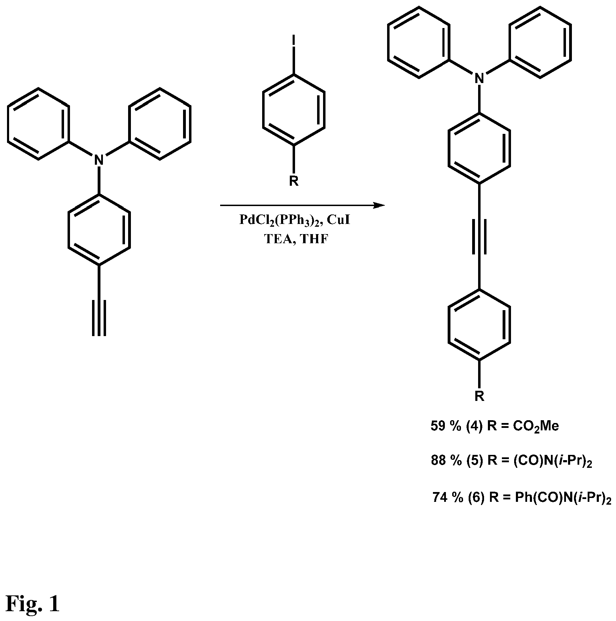

Der Aufbau der Polymerfilme auf dem Substrat kann dabei beispielsweise mittels Spin-Coating auf einer unbehandelten Substratoberfläche ausgehend von einer Polymerlösung oder durch radikalische Polymerisation von Comonomeren und Quervernetzern auf einer zuvor mit 3-(Trimethoxysilyl)propyl-methacrylat kovalent modifizierten Oberfläche, z.B. auf einer Glasoberfläche erfolgen. Im letztgenannten Fall wird "verlinktes" Glas erhalten. Den hier beispielhaft beschriebenen Ausführungsformen liegen kovalent am Glas gebundene Polymerketten als molekulare Monolagen oder als Polymerfilme, umfassend kovalent am Glas gebundene und zumindest teilweise untereinander quervernetzte Polymerketten, zu Grunde. Das Nachweisreagenz wird dann adsorptiv am Polymerfilm gebunden (Fign. 1-3). Als ein Beleg der überwiegend auf physiko-chemischen Wechselwirkungen beruhenden Bindung des Nachweisreagenz an den Polymerfilm wird angesehen, dass sich ein mit dem Nachweisreagenz ausgerüsteter Polymerfilm durch Waschen mit organischen Lösungsmitteln wieder vollständig entfärben lässt, sodass keine Fluoreszenz mehr vorliegt, das zum Waschen verwendete Lösungsmittel jedoch fluoresziert.

Vorteile dieser Ausführungsform bestehen darin, dass ein hinsichtlich seiner Polarität an die Polarität des jeweiligen Nachweisreagenz angepasster Polymerfilm bzw. eine resultierende starke Wechselwirkungen zwischen dem Polymermaterial und den Molekülen des Nachweisreagenz (Farbstoff) die Beweglichkeit letzterer an der Luft-Polymer Grenze reduziert. Dadurch ist bei einer an das jeweilige Nachweisreagenz angepassten optischen Anregung in Gegenwart eines Analyten (Explosivstoff oder Markerstoff) ein stabiles Emissionssignal messbar.

Ein weiteres technisches Merkmal für das Einstellen einer hohen Empfindlichkeit der Analyt-sensitiven Schicht ist die Anpassung der Schichtdicke des die molekulare Sonde tragenden Polymerfilms. Weiterhin ist das Polymermaterial hinsichtlich seiner Polarität, einer mittleren Kettenlänge, und einer Länge der zur Verbrückung von benachbarten Monomeren eingesetzten Linker (Quervernetzer) an die chemischen und physikalischen Eigenschaften des Explosivstoffs anpassbar. Diese Anpassung dient zur Anreicherung von leichtflüchtigen Analyten (Explosivstoffe, leichtflüchtige Zerfallsprodukte von (schwerflüchtigen) Explosivstoffen und leichtflüchtige Marker). Über eine verlängerte Verweilzeit wird eine zuverlässige Wechselwirkung (Fluoreszenzlöschung) und daraus resultierend eine verbesserten Detektion der betreffenden Substanz erreicht. Das molare Verhältnis des Monomeren und eines ggf. zu dessen Vernetzung verwendeten bifunktionalen Reagenz bestimmt für eine gegebene Konzentration des zur Polymerisation verwendeten Radikalbildners bei sonst gleichen Reaktionsbedingungen eine mittlere Porosität der erhaltenen Polymerschicht. Für praktische Ausführungsbeispiele, betreffend die Herstellung verschiedener, hier beispielhaft verwendeter Polymerfilme werden weiter unten noch detailliert Angaben gemacht.

Als geeignete Polymere erwiesen sich insbesondere Polymethacrylate. Lediglich als praktische Ausführungsbeispiele (vgl. nachfolgend noch beschriebene **SM1** und **SM2)** werden die ausgehend von Benzylacrylat und Benzylmethacrylat erhaltenen Poly(benzylacrylate), bzw. Poly(benzylmethylacrylate) angeführt. Die Verwendung anderer Monomere ist ebenso möglich. Bei der genutzten radikalischen Polymerisation können benachbarte Polymerketten direkt miteinander vernetzt werden. Wie beispielhaft für die ebenfalls später noch beschriebenen Sensormaterialien **SM3** und **SM4** gezeigt, können jedoch auch untereinander, mit Linkern - beispielsweise mit Bisacrylamid, vernetzte Polymerketten zur Ausbildung einer Polymerschicht verwendet werden. Wie bereits zuvor können auch hier verschiedenste Acrylate, Acrylamid, bzw. Acrylamide, Arylacrylate, alkyl-substituierte Arylacrylate, Arylacrylamide oder alkylsubstituierte Arylacrylamide mit beliebigen Quervernetzern, beispielsweise einem Piperazinderivat oder einem anderen bifunktionellen Linker vernetzt werden.

Gemäß einem praktischen Ausführungsbeispiel wird zur Detektion einer schwerflüchtigen NOx-Verbindung, ausgehend von reinem Benzylmethacrylat (BMA), Benzylacrylat (BA), Styrol oder deren Derivaten, ein dünner, wasserabweisender Polymerfilm auf einer homogenen Glasoberfläche hergestellt. So erhaltene Polymerfilme wurden gemäß nachfolgend noch detailliert beschriebenen Ausführungsbeispielen mit den Nachweisreagenzien **4** und **5** beschichtet. Wie nachfolgend noch an konkreten Beispielen erläutert, wurden dazu Poly(benzylmethacrylat)-, Poly(benzylacrylat)- bzw. Polystyrol-Lösungen in apolaren Lösungsmitteln wie Toluol auf einer unbehandelten Glasoberfläche als homogene Schicht aufgebracht. Im zweiten Schritt wird das Nachweisreagenz gemäß Formel **4** oder **5** in einem polaren Lösungsmittel (z.B. Isopropanol) homogen auf den Polymerfilm aufgetragen. Nachdem das organische Lösungsmittel verdampft ist, liegt eine homogen fluoreszierende Analyt-sensitive Schicht vor.

Vorteilhaft ist hierbei die Möglichkeit einer kostengünstigen, einfachen und schnellen Herstellung von dünnen, zur fluoreszenzoptischen Detektion von Explosivstoffen geeigneten Polymerfilmen; eine hohe Oxidations-, Photo- und Langzeitstabilität der resultierenden Analyt-sensitiven Schichten, umfassend eine Polymerschicht und daran adsorptiv gebundenes Nachweisreagenz; und eine hohe Empfindlichkeit der Analyt-sensitiven Schichten gegenüber Explosivstoffen auf NOx-Basis. Insbesondere ändert sich ein an der Analyt-sensitiven Schicht (Polymerfilm + Nachweisreagenz) gemessenes Fluoreszenzsignal bei Anwesenheit des Explosivstoffes. Wie nachfolgend noch weiter erläutert, erwies sich ein dünner Polymerfilm, der ohne einen zusätzlichen Quervernetzer auf dem Substrat aufgebaut wurde, für schwerflüchtige Explosivstoffe als besonders geeignet. Nitro-Verbindungen mit höherem Dampfdruck lassen sich hingegen vorteilhaft mit Hilfe von Analyt-sensitiven Schichten nachweisen, die eine dickere und poröse Polymerschicht umfassen. Hierfür geeignete dickere Polymerschichten sind bevorzugt Copolymerschichten, die aus einer ersten Art Moleküle (ersten Monomer) und einer zweiten Art Moleküle, beispielsweise zwei endständige reaktive Gruppen aufweisenden Molekülen (Quervernetzer), ausgebildet werden. Durch die vorhandenen Poren erscheinen diese Polymerschichten optisch opak, bzw. weiß.

Die erhaltenen Polymere, also Poly(benzylmethacrylat), Poly(benzylacrylat) und Polystyrol können auch kovalent auf der Substratoberfläche verankert werden. Dazu wird die Glasoberfläche zuvor z.B. mit 2-(Trimethylsilyloxy)ethylmethacrylat modifiziert ("verlinktes" Glas) und das Polymer mittels radikalischer Polymerisation kovalent gebunden. Im dritten Schritt wird der Farbstoff **4** oder **5** auf den Polymerfilm sodann gleichmäßig, z.B. mittels Spin-coating, aufgetragen.

Vorteile dieser Ausführungsform sind die kostengünstige, einfache und schnelle Herstellung von kovalent am Substrat verankerten Polymerketten mit der zusätzlichen Möglichkeit, funktionelle Comonomeren einzubauen und so die Polarität der Polymerketten an die Polarität des jeweiligen Farbstoffs anzupassen. Hierbei wird vorteilhaft eine solche Polarität eingestellt, dass eine starke Wechselwirkung zwischen den Polymerketten und dem Farbstoff erreicht und damit die Beweglichkeit der Farbstoffmoleküle, welche zur Bildung von Aggregaten führt, einschränkt und somit die Selbstlöschung (self-quenching) der Fluoreszenz minimiert wird. Diese Methode gewährleistet eine hohe Luft-, Photo- und Langzeitstabilität der Farbstoffe auf dem Polymerfilm und eine hohe Empfindlichkeit der resultierenden Analyt-sensitiven Schicht zum Nachweis von schwerflüchtigen Explosivstoffen auf NOx-Basis.

Weitere Vorteile der Verwendung von Poly(benzylmethacrylat), Poly(benzylacrylat) und Polystyrol als Polymermatrix auf Glas ergeben sich daraus, dass die entsprechenden hydrophoben Polymerfilme wasserabweisend sind und sich daher in Gegenwart von Wasser zusammenziehen. Durch das dabei resultierende einander Näherrücken der Polymerketten wird die Umgebung der Farbstoffmoleküle apolarer, wodurch deren Fluoreszenzintensität in Gegenwart eines Analyten zunimmt und sich das Fluoreszenzmaximum in den blauen Bereich des Spektrums verschiebt (vgl. Tabelle 1). Ist eine schwerflüchtige NOx-Verbindung, wie z.B. TNT, im Wasserdampf vorhanden, der auf die Analyt-sensitive Schicht geleitet wird, so verbleibt diese auf dem Polymerfilm und eine deutliche Fluoreszenzlöschung ist messbar.

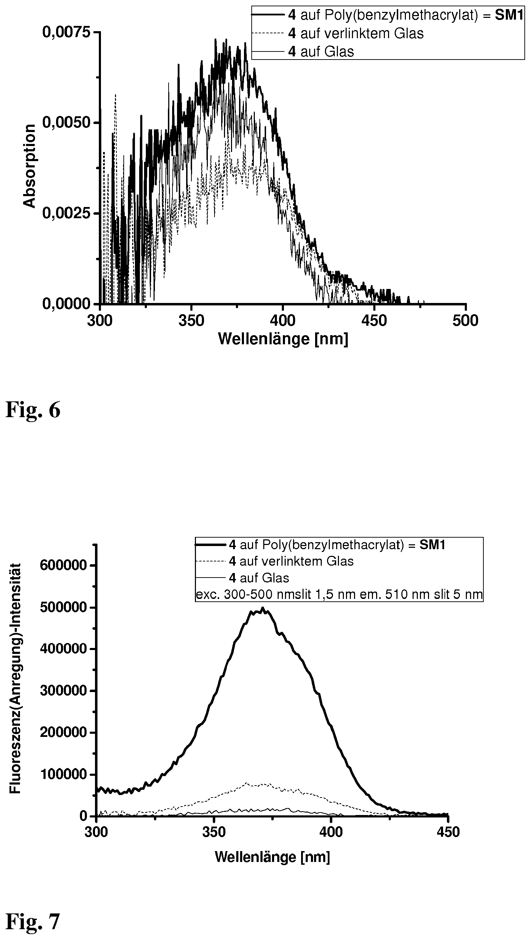

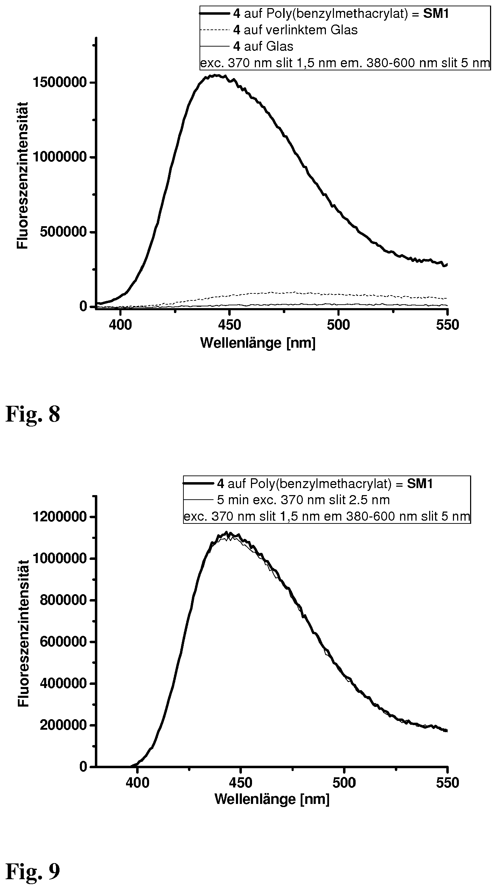

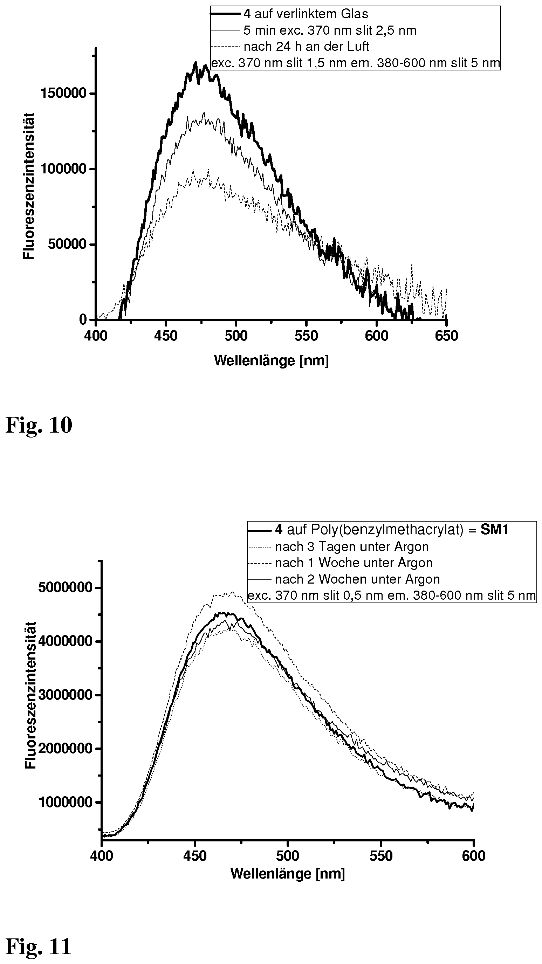

Im Übrigen kann die Verschiebung und die Intensität der Fluoreszenz der reinen Nachweisreagenzien auf polymermodifizierten Glassubstraten auch als Beleg des Vorhandenseins der Polymerschicht auf dem Glas gewertet werden: Das auf reinem, chemisch nicht modifiziertem Glas aufgebrachte Nachweisreagenz 4 verliert nach wenigen Stunden an der Luft seine Fluoreszenz. Das auf das "verlinkte" Glas aufgebrachte Nachweisreagenz **4** fluoresziert vergleichsweise schwach (λₘₐₓ = 470 nm), während dasselbe Nachweisreagenz **4** in gleicher Menge/Fläche auf ein polymermodifiziertes Glassubstrat aufgebracht, eine ausgeprägte und dauerhafte Fluoreszenz zeigt (λₘₐₓ = 445 nm) (vgl. Fign. 7-8). Aufgrund der ähnlichen Absorptionsintensitäten bei λₘₐₓ = 370 nm des Nachweisreagenz **4** auf den drei unterschiedlich modifizierten Glassubstratoberflächen ist dessen Konzentration auf nicht modifiziertem Glassubstrat im Vergleich zum polymermodifizierten Glassubstrat nur um das 1,2-fache geringer (Fig. 6). Zudem verblasst die Fluoreszenz auf "verlinktem" Glas unter Dauerbelichtung (λ = 370 nm) um 23 % (Fig. 10), während sie sich auf einer Polymerschicht unter sonst gleichen Bedingungen (Temperatur, Feuchte) als stabil erwies (3 % Fluoreszenzlöschung). (Fig. 9).

**Tabelle 1**

| | | | | |
|---|---|---|---|---|
| Die nachfolgend angegebenen Fluoreszenzquantenausbeuten des Fluoreszenzindikators **4** wurden unter Verwendung der Verbindung 2,2'-p-Phenylenbis-(5-phenyloxazol) (POPOP) (ϕ_{f}= 0,91 in EtOH) als Standard ermittelt [8]. | | | | |

| Polarität | Lösungsmittel | λ_{abs}/nm | λₑₘ/nm | φ_{f} |
|---|---|---|---|---|
| 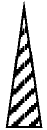 | Hexan | 367 | 394/410 | 0,67 |
| | Toluol | 375 | 425 | 1,00 |
| | CHCl₃ | 375 | 470 | 1,00 |
| | EtOH | 370 | 535 | 0,22 |
| | ACN | 367 | 545 | 0,56 |

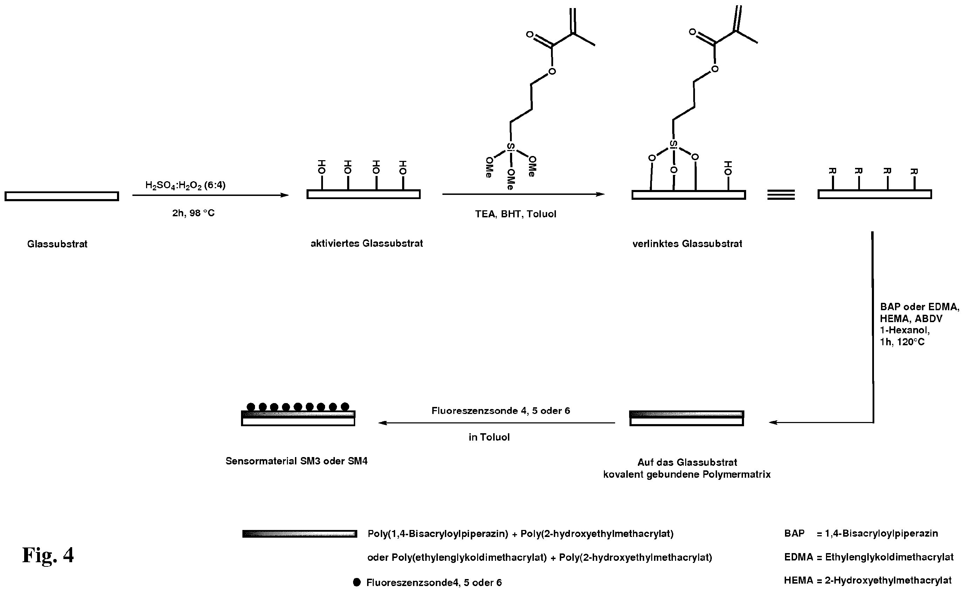

Gemäß einem weiteren praktischen Ausführungsbeispiel wurde zur Detektion des Markerstoffs DMDNB eine Mischung aus 1,4-Bisacryloylpiperazin (BAP) mit oder ohne 2-Hydroxyethylmethacrylat (HEMA) auf verlinktem Glas polymerisiert. Die auf die Glasoberfläche kovalent gebundene Polymermatrix wird im zweiten Schritt mit dem Farbstoff **5** oder **6** beschichtet (Fig. 4).

Vorteile ergeben sich aus der Bereitstellung eines einfachen und schnellen Herstellungsverfahrens der dünnen Polymerfilme, einer hohen Luft-, Photo- und Langzeitstabilität der Nachweisreagenzien auf dem Polymerfilm und einer hohen Empfindlichkeit gegenüber DMDNB durch dessen Anreicherung im Polymermaterial. Als Langzeitstabilität wird hierbei die Stabilität der Verbindung bei Lagerung unter Schutzgas und Lichtausschluss bei Raumtemperatur verstanden.

Weitere Vorteile ergeben sich aus der Transparenz der Polymerfilme zur Messung der Fluoreszenz von der unbeschichteten Seite der Gläser her, sowie aus den nicht fluoreszierenden und nicht absorbierenden Eigenschaften der Polymere Poly(benzylmethacrylat), Poly(benzylacrylat) und Polystyrol im sichtbaren Bereich des Spektrums, sowie aus der gleichmäßig guten Benetzbarkeit der Glasoberfläche für die Polymere und der hohen Stabilität dieser Polymere auf der Glasoberfläche an der Luft.

Ähnlich vorteilhaft ist, dass die Hauptabsorptions- und Fluoreszenzbanden der beschriebenen Triphenylaminoalkine im sichtbaren Spektralbereich liegen. Aus diesem Grund kann auf eine UV-Anregung der Sonde zur Erzeugung eines Fluoreszenzsignals verzichtet werden. Hieraus ergeben sich reduzierte Kosten und die Möglichkeit der Realisierung eines portablen Messgerätes, da UV-Anregungsquellen (jedenfalls heutzutage noch) deutlich teurer, instabiler und meist auch dimensional größer sind, als z.B. mit LED ausgestattete Lesegeräte.

Da sowohl das Polymermaterial als auch das Glas UV-Strahlung absorbiert, wird hierdurch die Anregung der Farbstoffe auf dem Polymerfilm erleichtert.

Vorteile der Detektion von NOx-Verbindung, wie TNT in der Luft, im Wasser, in organischen Lösungsmitteln und auf Oberflächen sind offensichtlich. Vorteile des vorgeschlagenen Nachweisverfahrens und der hierbei genutzten Nachweisreagenzien betreffen eine unkomplizierte Probenaufbereitung, die einen direkten und schnellen Nachweis eines Gefährdungspotentials unmittelbar vor Ort erlaubt.

Weitere Vorteile ergeben sich aus der erreichten Einfachheit der Anwendung und dem geringen Einfluss von Umwelteinflüssen auf die Messung. Die Vorteile der mit fluoreszierenden Farbstoffen beschichteten Polymerfilme liegen auf der Hand: Genaue Messungen können mit dem entsprechenden Messgerät auch durch ungeübte Anwender fehlerfrei ausgeführt werden. Die Polymerfilme sind reproduzierbar in großer Stückzahl herstellbar, an der Luft beständig und können unter Schutzgas auf unbestimmte Zeit gelagert werden. Der geringe Einfluss von Wasser und organischen Lösungsmitteln sichert im Verein mit dem entsprechenden Messgerät eine zuverlässige Messung von Luft, Wasser- und Wischproben bei unterschiedlichsten Messsituationen.

Gemäß einem weiteren Aspekt wird ein Verfahren zum Nachweis einer NOx-Verbindung vorgeschlagen, wobei der Nachweis mit einem portablen, bevorzugt einhändig tragbaren Lesegerät erfolgt, das eine bei zumindest einer definierten Wellenlänge messende Scan-Vorrichtung umfasst.

Vorteilhafterweise sind geeignete Lesegeräte kommerziell verfügbar und werden bereits für verschiedene Tests zum Nachweis von umweltrelevanten Chemikalien verwendet. Es kann mit einer stetigen Weiterentwicklung derartiger portabler Geräte gerechnet werden, sodass zum einen ggf. die Empfindlichkeit der realisierbaren Messverfahren; zum anderen aber auch die Palette der reproduzierbar und sicher auswertbaren spektroskopischen Parameter erweitert werden dürfte.

Demgemäß wird ein Verfahren zum Nachweis einer NOx-Verbindung in der Luft vorgeschlagen, wobei das Verfahren umfasst:
- Bereitstellen einer Analyt-sensitiven fluoreszierenden Schicht, umfassend ein als feste Phase wirkendes Trägermaterial (Molekülteppich, Polymer) mit einer der zuvor beschriebenen Fluoreszenzsonden;
- Echtzeitmessung der Fluoreszenz und Erfassen einer Fluoreszenzeigenschaft, insbesondere einer Fluoreszenzabnahme der Analyt-sensitiven Schicht bei Wechselwirkung des Analyten mit der Analyt-sensitiven Schicht.
Das Verfahren kann optional weiter zumindest einen der nachfolgenden Schritte umfassen:
- Leiten einer potentiell mit einer Nitro-Verbindung kontaminierten Gasströmung (z.B. Luftströmung) auf die fluoreszierende Schicht, sodass der mit der Fluoreszenzsonde befrachtete Abschnitt des Trägermaterials von der Luftströmung vollständig benetzt wird;
- Qualitative Analyse der NOx-Verbindung an Hand von Vergleichswerten und/oder Kurvenverläufen und/oder zumindest einer zuvor erfassten Regenerierphase bei Anströmen der Analyt-sensitiven Schicht mit Analytfreier Luft oder mit Analyt-freiem Wasserdampf;
- Ermitteln einer Konzentration oder eines Konzentrationsbereichs des Analyten (z.B. Explosivstoffes) in der Gasströmung an Hand eines Vergleichswertes und/oder einer Kalibrierkurve, wobei der Vergleichswert und/oder die Kalibrierkurve nach Wechselwirkung der Fluoreszenzsonde mit einer bekannten Konzentration des Analyten, z.B. in Luft, ermittelt wurden.

Die beschriebene Messung der Fluoreszenzlöschung kann beispielsweise von der Rückseite des Substrates (also von der nicht beschichteten Seite des Substrates aus) erfolgen. Eine entsprechende Messanordnung setzt ein optisch transparentes Substratmaterial voraus. Ebenso kann von der beschichteten Seite aus gemessen werden. Das Substrat (Trägermaterial) und auch der darauf aufgebrachte Polymerfilm müssen also nicht transparent sein. Wird ein geeignetes transparentes Substrat verwendet, beispielsweise Glas, kann auf Grund der Lichtleitereigenschaften eines solchen Substrates bei einer reproduzierbaren Einkopplung des Fluoreszenzlichtes in das Substrat die Fluoreszenzmessung auch von einem Außenrand des Substrates aus erfolgen. Hieraus ergibt sich beispielsweise eine vorteilhaft kompakte Messanordnung.

Entsprechend dem in der Rückstandsanalytik üblichen Vorgehen lässt sich nach einer Kalibrierung des Messsignals für den betreffenden Konzentrationsbereich mit einem aktuell ermittelten Messwert (Fluoreszenzlöschung) aus den Mengenverhältnissen des eingesetzten Probenvolumens (Aliquot) auf die Original-Konzentration des betreffenden Analyten in der jeweiligen Originalprobe (Matrix + Analyt) folgern.

Demgemäß wird ein Verfahren zum Nachweis einer NOx-Verbindung (beispielsweise eines Explosivstoffs) aus einer Lösung, insbesondere aus einer wässrigen oder einer organischen Lösung vorgeschlagen, wobei das Verfahren umfasst:
- Bereitstellen einer Analyt-sensitiven Schicht, umfassend ein als feste Phase wirkender Polymerfilm auf einem Trägermaterial mit einer der zuvor beschriebenen und adsorptiv am Polymerfilm gebunden Fluoreszenzsonden;
- Echtzeitmessung einer Fluoreszenzeigenschaft, beispielsweise einer Fluoreszenzabnahme zumindest eines Abschnitts der Analyt-sensitiven Schicht nach Wechselwirkung der Analyt-sensitiven Schicht mit dem die NOx-Gruppe umfassenden Analyten mit einer geeigneten Messanordnung.
Das Verfahren umfasst weiter die nachfolgenden Schritte:
- Erwärmen und/oder Verdampfen der potentiell mit dem nachzuweisenden Analyten kontaminierten Lösung, beispielsweise an einem geheizten Lufteinlass, und Leiten der dabei entstehenden Dämpfe mit einem Luftstrom auf die fluoreszierende Analyt-sensitive Schicht, sodass ein mit einer Fluoreszenzsonde befrachteter Abschnitt des Trägermaterials von der Luftströmung vollständig benetzt wird, bzw. der Analyt in und/oder an der Analytsensitiven Schicht angereichert wird;
- Qualitative und/oder quantitative Analyse der NOx-Verbindung an Hand von Vergleichswerten und/oder Kurvenverläufen und Regenerierphasen, d.h. unter Verwendung von gespeicherten Messdaten einer Vergleichsmessung;
- Ermitteln einer Konzentration oder eines Konzentrationsbereichs des Analyten (Explosivstoffes) in der Lösung an Hand eines Vergleichswertes und/oder einer Kalibrierkurve, wobei der Vergleichswert und/oder die Kalibrierkurve nach Wechselwirkung der Fluoreszenzsonde mit einer bekannten Konzentration des Analyten ermittelt wurden.

Gemäß einer beispielhaft angeführten Ausführungsform kann ein Verfahren zum Nachweis einer NOx-Verbindung, insbesondere eines nitro-aromatischen Sprengstoffes auf einer Oberfläche die folgenden Schritte umfassen:
- Befrachten eines als feste Phase wirkenden Trägermaterials mit einer der zuvor beschriebenen Fluoreszenzsonden und Erhalten einer fluoreszierenden Analytsensitiven Schicht (Indikatorschicht). Vorteilhaft ist diese Indikatorschicht auf einem starren Substrat angeordnet und widersteht einer Anströmung mit einem Fluid (insbesondere einem aufgeheizten Gas).
- Erfassen eines zeitlichen Verlaufs eines Fluoreszenzsignals zumindest eines Abschnitts der Indikatorschicht bei Wechselwirkung des Analyten mit der Analyt-sensitiven Schicht, insbesondere einer Abnahme einer Fluoreszenz der Indikatorschicht. Wie auch zuvor kann die Fluoreszenzmessung sowohl im Transmissionsmodus, als auch als Epifluoreszenzmessung erfolgen.
Das oben beispielhaft angeführte Verfahren kann optional weiter zumindest einen der nachfolgenden Schritte umfassen:
- Aufnehmen von Analysematerial von einer Oberfläche mit einem Wischprobenmaterial, sodass auf der Oberfläche vorliegendes Analysematerial auf das Wischprobenmaterial übertragen wird.
- Heizen des Wischprobenmaterials auf eine Temperatur >150°C. Leiten von hierbei freigesetzten thermischen Sublimations- und/oder Zersetzungsprodukten des Wischprobenmaterials mit einem Trägergasstrom (z.B. mit einem Edelgas, einem trockenen Gas, oder Luft) auf die fluoreszierende Indikatorschicht. Hierbei wird der mit dem Nachweisreagenz befrachtete Abschnitt des Trägermaterials von der Trägergasströmung vollständig benetzt. Im Ergebnis dessen kann eine Wechselwirkung der im Trägergasstrom herangeführten Analyte mit der Fluoreszenzsonde erfolgen.
- Qualitatives Analysieren der NOx-Verbindung an Hand von zuvor aufgezeichneten oder in einer Datenbank gespeicherten Vergleichswerten und/oder Kurvenverläufen. Ebenso kann eine Regenerierphase, d.h. ein Erholen einer zunächst zumindest teilweise gelöschten Fluoreszenz unter Einwirkung eines reinen Trägergases (z.B. Luft) oder mit Wasserdampf zur Beurteilung der stofflichen Natur und/oder Konzentration des Analyten im Trägergasstrom herangezogen werden.
- Ermitteln einer Konzentration oder eines Konzentrationsbereichs des Analyten (Explosivstoffes) auf dem Probenträger an Hand eines Vergleichswertes und/oder einer Kalibrierkurve, wobei der Vergleichswert und/oder die Kalibrierkurve nach Wechselwirkung der Fluoreszenzsonde mit einer bekannten Konzentration des NOx-haltigen Analyten, bspw. des Sprengstoffes oder einer Markersubstanz von Explosivstoffen ermittelt wurden.

Unabhängig von der Art der jeweiligen Probe und der jeweiligen Analyt-sensitiven Schicht kann eine Fluoreszenzmessung eine synchrone Anregung einer oder mehrerer Analyt-sensitiver Schichten bei unterschiedlichen Anregungswellenlängen und/oder bei unterschiedlichen Emissionswellenlängen umfassen. Zur Anregung können eine oder mehrere Lichtquellen verwendet werden, beispielsweise Laser, LED, OLED, Glühfadenlampe.

Der Einsatz von fluoreszierenden konjugierten Polymeren zur Detektion von Explosivstoffen mittels Fluoreszenzlöschung ist beispielsweise aus US 8,287,811 B1; US 8,323,576 B2; US 8557595 B2; US 8,557,596 B2; CN 101787112 und [3 - 5] bekannt. Die Mehrzahl bekannter Detektionsmethoden für Explosivstoffe auf NOx-Basis basiert auf der spezifischen Wechselwirkung zwischen dem AFP und dem Analyten mit einem hohen Oxidationspotential. Um eine hohe Empfindlichkeit zu erreichen, werden dünne Schichten der AFPs direkt auf das verwendete Substrat, beispielsweise auf ein Glas aufgetragen.

Die Nachteile bekannter Nachweisverfahren auf der Basis konjugierter Polymere (AFPs) zur Detektion von Explosivstoffen lassen sich stichpunktartig wie folgt zusammenfassen:
- Die bestehenden Querempfindlichkeiten der derzeit als technologisch führend betrachteten fluoreszierenden Sensormaterialien können zu Fehlalarmen führen z.B. auch durch plötzliche Änderungen der Luftfeuchtigkeit oder durch Wechselwirkung mit Stoffen mit einem hohen Oxidationspotential, die nicht zur Gruppe der Explosivstoffe oder Markerstoffe zählen. Die bestehenden Querempfindlichkeiten beeinträchtigen die Gesamtwirksamkeit bzw. Marktakzeptanz der Sensormaterialien.
- Dies hat wiederrum den Nachteil, dass die Empfindlichkeit für den jeweiligen Explosivstoff abnimmt. Daher ist das Einsatzgebiet solcher AFPs als Sensoren für Explosivstoffe auf NOx-Basis auf relativ konstante Witterungs- und Umweltbedingungen beschränkt.
- Neben einer erhöhten Luftfeuchtigkeit und Wasser können auch mit Wasser mischbare organische polare Lösungsmittel, sowie thermisch Wasserdampf produzierende Stoffe wie Kristallwasser enthaltende Salze oder thermisch induzierte Kondensationsreaktionen die Querempfindlichkeit des jeweiligen Sensormaterials erhöhen, bzw. die spezifische Empfindlichkeit für den Analyten senken.
- Um eine hohe Empfindlichkeit gegenüber den Explosivstoffen zu erzielen, sind monomolekulare Schichten der konjugierten Polymere erforderlich.
- Die Signalbildung beruht nur auf der Wechselwirkung zwischen dem konjugierten Polymer und dem Analyten. Das verwendete Trägermaterial, z.B. Glas, zeigt mit flüchtigen Analyten nur eine schwache Wechselwirkung.
- Der verpflichtend seit 1991 in kommerziell verfügbaren Explosivstoffen enthaltene Markerstoff DMDNB kann nur in wenigen Fällen mit Hilfe von AFPs [7] detektiert werden.

Aufgrund der großen Vielfalt an Stoffklassen mit ihren elektronischen bzw. sterischen Unterschieden in der Molekülstruktur und unterschiedlichen Eigenschaften (z.B. Flüchtigkeit / Dampfdruck) ist für einen Breitbandnachweis - also für den parallel erfolgenden Nachweis unterschiedlicher Analyten in einem Probenmaterial - der Einsatz spezifisch angepasster Analyt-sensitiver Schichten, die eine selektive Detektion unterschiedlicher Explosivstoffe ermöglichen, notwendig.

Eine hier vorgeschlagene Anpassung umfasst die Dicke und/oder Benetzbarkeit der Polymerschicht, welche die molekulare Sonde trägt, an die Flüchtigkeit (Dampfdruck) eines Analyten, ausgewählt unter einem oder mehreren Explosivstoffen und dem zu deren Markierung eingesetzten Marker, z.B. DMDNB.

Demgemäß werden beispielsweise mindestens zwei funktionalisierte, spezifische Analyt-sensitive Schichten mit unterschiedlicher Komposition eingesetzt, um aus den relativen Fluoreszenzsignalen beider Schichten über eine Mustererkennung, durch Messung bestimmter Charakteristiken und/oder über die absoluten bzw. relativen Geschwindigkeit der Regenerierung der Analyt-sensitiven Schichten eine Eingruppierung (Kategorisierung) der in der jeweils untersuchten Probe enthaltenen Explosivstoffe vornehmen zu können.

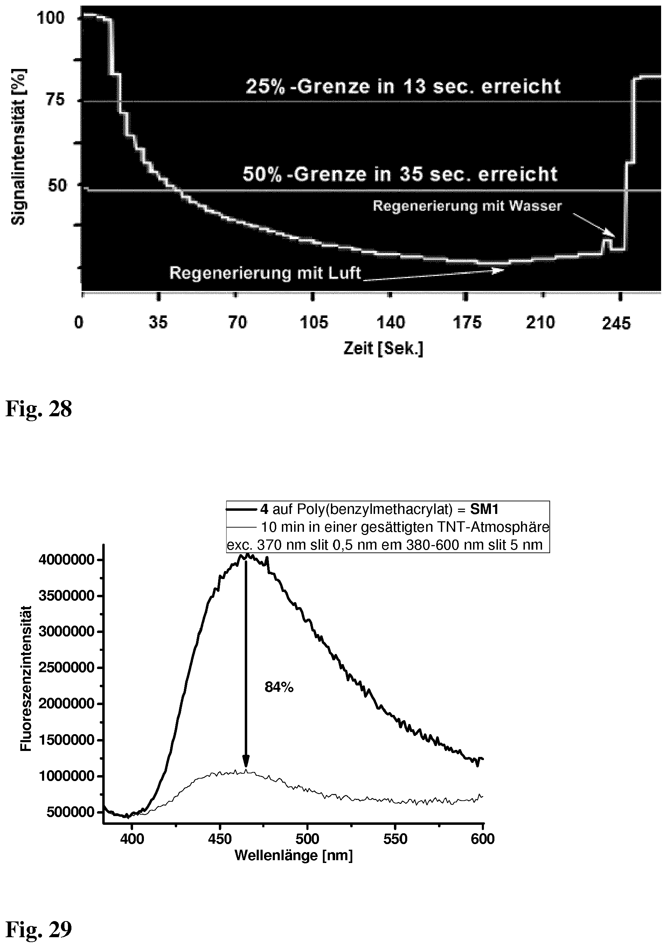

Bei schwerflüchtigen Explosivstoffen wie TNT und RDX ist die Sensitivität der Analyt-sensitiven Schicht vor allem von deren Schichtdicke abhängig. (Fig. 29 bis 31 zeigen die Fluoreszenzspektren von **SM1** vor und nach Begasung mit TNT bei Raumtemperatur sowie die Schichtdicken der entsprechenden Polymerfilme).

Die Schichtdicke von **SM1** und **SM2** beträgt bevorzugt zwischen 1 und 5 nm. Zur Sichtbarmachung der Schicht unter dem Elektronenmikroskop wurde der Polymerfilm mit Gold bedampft und die Bruchkante beobachtet (Fig. 30). Für **SM3** und **SM4** zur Detektion des Markers DMDNB werden zur Anreicherung Polymerfilme mit einer porösen, dickeren Schicht (150-350 nm) eingesetzt. Die Aufnahmen unter dem Elektronenmikroskop zeigen, dass die Oberfläche von **SM3** Poren in der Größenordnung von 50 nm enthält, die die Durchlässigkeit dieses Materials für flüchtige Analyten wie DMDNB ermöglichen (Fig. 33 und 34). Aufgrund der Schichtdicke von 150 nm können daher flüchtige Analyten in diesem Material angereichert werden (Fig. 35). Im Falle von **SM4** umfasst die Polymeroberfläche nach den Aufnahmen unter dem Elektronenmikroskop beurteilt Polymerpartikel. Gemessene Schichtdicken lagen im Bereich von 160-350 nm (vgl. Fig. 36).

Um die Farbstoffdichte auf den Polymerfilmen zu bestimmen wurde in einer Verdünnungsreihe (in Toluol) der Farbstoffe **4, 5** und **6** mit bekannter Konzentration die Absorption gemessen. Aufgrund des linearen Verhaltens der Absorptionsintensität zur Farbstoffkonzentration konnte dadurch die Farbstoffkonzentration auf den Polymerfilmen bestimmt werden. Dazu wurden die mit dem entsprechenden Farbstoff beschichteten Polymerfilme mit einer bestimmten Menge Toluol versetzt und anschließend die Absorption der Lösung gemessen. Daraus ergeben sich für Glassubstrate mit einer Fläche von 2,8 cm² folgende Farbstoffkonzentrationen für die Sensormaterialien **SM1** - **SM4:** Für **SM1** und **SM2** bzw. Poly(benzylmethacrylat) beschichtet mit dem Farbstoff **4** (75 pmol / cm²); Für **SM1** und **SM2** bzw. Poly(benzylmethacrylat) beschichtet mit dem Farbstoff **5** (30 pmol / cm²); Für **SM3** bzw. Poly(1,4-bisacryloylpiperazin) / Poly(2-hydroxyethylmethacrylat) beschichtet mit dem Farbstoff **6** (70 pmol / cm²); Für **SM4** bzw. Poly(ethylenglykoldimethacrylat) / Poly(2-hydroxyethylmethacrylat) beschichtet mit dem Farbstoff **5** (30 pmol / cm²).

Für das schwerflüchtige TNT wurde eine untere Nachweisgrenze von 1,9 ng aus einer Wischprobe erreicht. Hierbei löste ein senkrecht von der Wischprobe auf die Analyt-sensitive Schicht **SM1** geleiteter Luftstrom einer Temperatur von 120-150°C eine mittlere Fluoreszenzlöschung von 20 % aus. Jedoch ist bereits eine Fluoreszenzlöschung von 5 bis 10 % sicher feststellbar. Daraus ergibt sich, dass die tatsächlich erreichbare untere Nachweisgrenze dieser Analyt-sensitiven Schicht für TNT im Pikogramm-Bereich liegt. Mit Hilfe einer optimierten Probenführung (Führung des Luftstroms mit dem Analyten auf die Analyt-sensitive Schicht) kann die Sensitivität optimiert werden.

Die geringste nachweisbare Konzentration von TNT im Trägergasstrom (Luft, 120 - 150°C) beträgt 5 ppb. Das wurde mit Hilfe eines Exsikkators, der eine konstante TNT-Konzentration einer Luftprobe bereitstellt, verifiziert. Die Konstanz dieser Konzentration wurde massenspektroskopisch überwacht.

Weitere schwerflüchtige Explosivstoffe wie PETN und Ammoniumnitrat bilden beim Erhitzen flüchtigere Nitrate sowie Stickoxide. Diese zeigen mit den Analyt-sensitiven Schichten ebenfalls spezifische Wechselwirkungen und erlauben so einen spezifischen Nachweis.

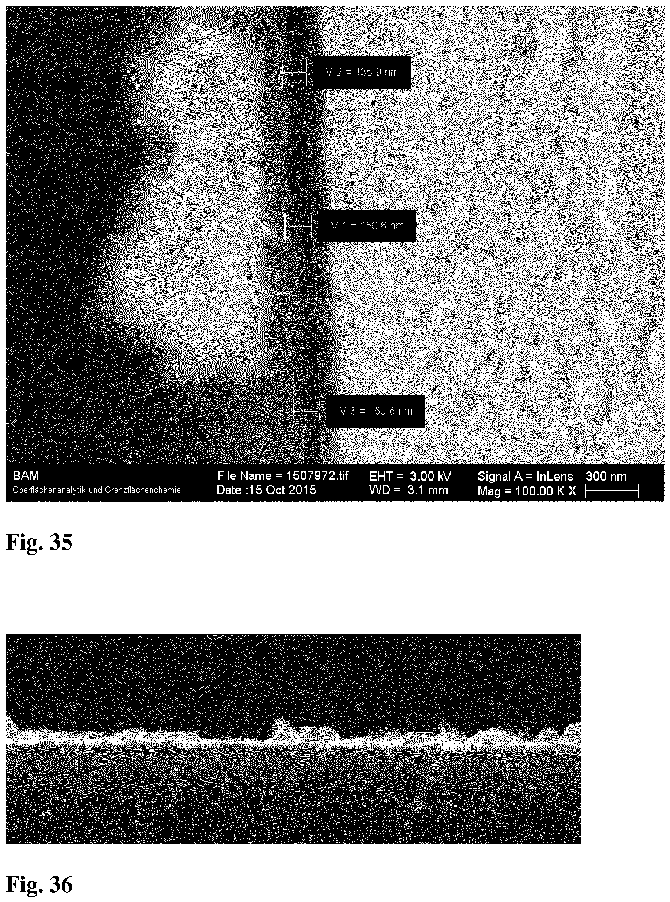

Der Markerstoff DMDNB ist im Vergleich zu TNT eine flüchtige Verbindung. Der Einsatz der vorgeschlagenen Polymere als Trägerschicht des Nachweisreagenz ermöglicht es, durch eine Anreicherung des jeweiligen flüchtigen Analyten die Verweilzeit dieser Verbindung auf der Analyt-sensitiven Schicht zu erhöhen und so die Detektionsgrenze in den ppb-Bereich herabzusetzen. Die Schichtdicke und die Komposition des Polymerfilms spielen dabei eine wichtige Rolle. Bei zunehmender Schichtdicke der Analyt-sensitiven Schicht nimmt die Sensitivität gegenüber dem Marker ab, weil das Nachweisreagenz in den unteren Schichten für den Marker nicht mehr erreichbar ist. Bei dünnen Analyt-sensitiven Schichten dagegen nimmt die Verweildauer des flüchtigen Markerstoffs im Polymer ab, wodurch die Sensitivität der betreffenden Analyt-sensitiven Schicht ebenfalls abnimmt. Die optimale Schichtdicke der Analyt-sensitiven Schicht zur Detektion des Markers DMDNB beträgt 130-170 nm (vgl. Fig. 35).

### Detaillierte Beschreibung der Synthese verwendeter Nachweisreagenzien

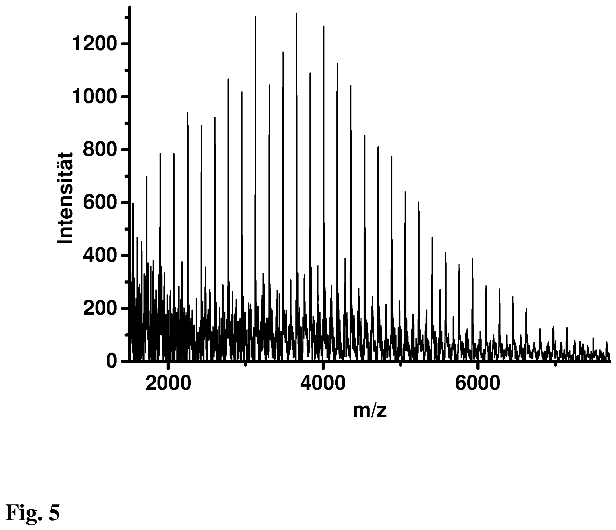

Die Synthese der Nachweisreagenzien **4, 5** und **6** erfolgt wie schematisch in Fig. 1 dargestellt. Alle Reagenzien stammen von kommerziellen Herstellern und wurden ohne weitere Aufreinigung eingesetzt. Alle luft- und feuchtigkeitsempfindlichen Reaktionen wurden unter Schutzgas (Argon) in trockenen Glasapparaturen durchgeführt. Triethylamin, Diisopropylamin, Dichlormethan und Tetrahydrofuran wurden über Molekularsieb (4Ä) getrocknet. Die Monomere zur Herstellung der Polymerfilme wurden vor ihrem Einsatz im Hochvakuum destilliert oder umkristallisiert. Für dünnschichtchromatographische Analysen (DC) wurden Merck DC Fertigplatten, Kieselgel 60 F254 verwendet. Die Sichtbarmachung der Substanzen erfolgte im UV-Licht bei 254 nm und 365nm. NMR-Messungen erfolgten unter Verwendung der Signale von Resten protonierter Lösungsmittel als internem Standard mit einem Gerät des Typs Bruker AV 400 oder Bruker 600. Chemische Verschiebungen werden hierbei in ppm und Kopplungskonstanten in Hz angegeben. Massenspektren zur Charakterisierung der Nachweisreagenzien wurden mit einem Orbitrap "Exactive" Mass Spektrometer erfasst. Die MALDI-Messung zur Charakterisierung der Polymerschichten von **SM1** bzw. **SM2** werde mit dem Autoflex III Smartbeam Bruker erfasst (Fig. 5). UV-vis Absorptionsspektren wurden mit einem Specord 210 Plus Spektrophotometer (Analytik, Jena) erfasst. Fluoreszenz-Messungen in Lösung und an Polymerfilmen wurden mit einem FluoroMax-4P Spektrofluorometer (Horiba Jobin-Yvon, Bensheim) erfasst. Für spektroskopische Messungen verwendete Lösungsmittel waren entsprechend rein (UV spectroscopic grade - Aldrich).

Das Ausgangsmaterial 4-Ethinyl-triphenylamin zur Herstellung der Nachweisreagenzien bzw. Farbstoffe **4** und **5** wird gemäß einer hier ausdrücklich einbezogenen Druckschrift [1], bzw. der darin beschriebenen Synthesevorschrift hergestellt.

### Herstellung des Nachweisreagenz (Referenz-Beispiel)

4-Ethinyl-triphenylamin (40 mg, 0,148 mmol) und Methyl-4-iodobenzoat (50 mg, 0,191 mmol) wurden in trockenem THF / TEA (4 mL, 1:1) unter Schutzgas gelöst und mit *trans*-Dichlorobis(triphenylphosphine)palladium (12 mg, 0,016 mmol) und CuI (12 mg, 0,063 mmol) versetzt und bei Raumtemperatur gerührt. Nach 16 h wurde die Reaktionsmischung mit Ethylacetat verdünnt und mit HCl saurem Wasser (10 %) versetzt und das Rohprodukt extrahiert, über Na₂SO₄ getrocknet und unter vermindertem Druck aufkonzentriert. Das Rohprodukt wurde durch Säulenchromatographie an Silicagel (Cyclohexan : Ethylacetat, 8 : 1) aufgereinigt. Es wurde ein gelblicher Feststoff erhalten (35 mg, 0,087 mmol, 59 % Ausbeute).

¹H-NMR (500 MHz, CDCl₃) δ: 3.85 (s, 3H, CH₃), 6.93 (d, *J* = 8.5Hz, 2H), 7.00 (t, *J* = 7.5Hz, 2H), 7.05 (d, *J* = 7.5Hz, 4H), 7.21 (t, *J* = 8.0Hz, 4H), 7.30 (d, *J* = 9.0Hz, 2H), 7.48 (d, *J* = 8.5Hz, 2H), 7.93 (d, *J* = 8.5Hz, 2H). ¹³C-NMR (CDCl₃) δ: 52.2, 88.0 (q), 92.9 (q), 115.2 (q), 121.9, 123.7, 125.1, 128.4 (q), 129.0 (q), 129.4, 129.5, 131.3, 132.7, 147.0 (q), 148.3 (q), 166.6 (q). HRMS m/e 404,1650 (M+H)⁺ (berechnet für C₂₈H₂₁NO₂ 404,1645). Herstellung von 4-Iodo-N,N-diisopropylbenzamid

4-Iodbenzoylchlorid (500 mg, 1,88 mmol) wurde in Dichlormethan unter Schutzgas bei 0°C tropfenweise mit Diisopropylamin (0,8 mL, 5,69 mmol) versetzt und bei Raumtemperatur gerührt. Nach 16 h wurde die Reaktionsmischung mit Ethylacetat verdünnt und mit HCl saurem Wasser (10 %) versetzt und das Rohprodukt extrahiert, über Na₂SO₄ getrocknet und unter vermindertem Druck aufkonzentriert. Das Rohprodukt wurde durch Säulenchromatographie an Silicagel (Cyclohexan : Ethylacetat, 5 : 1) aufgereinigt. Es wurde ein weißer Feststoff erhalten (511 mg, 1,54 mmol, 82 % Ausbeute). Die Spektren des aufgereinigten Produkts stimmen mit publizierten Daten [2] überein.

### Herstellung des Nachweisreagenz 5

4-Ethinyl-triphenylamin (54 mg, 0,200 mmol) und 4-Iodo-*N,N*-diisopropylbenzamid (62 mg, 0,187 mmol) wurden in trockenem THF / TEA (4 mL, 1:1) unter Schutzgas gelöst und mit trans-Dichlorobis(triphenylphosphine)palladium (8 mg, 0,011 mmol) und CuI (8 mg, 0,042 mmol) versetzt und bei Raumtemperatur gerührt. Nach 16h wurde die Reaktionsmischung mit Ethylacetat verdünnt und mit HCl saurem Wasser (10 %) versetzt und das Rohprodukt extrahiert, über Na₂SO₄ getrocknet und unter vermindertem Druck aufkonzentriert. Das Rohprodukt wurde durch Säulenchromatographie an Silicagel (Cyclohexan : Ethylacetat, ) aufgereinigt. Es wurde ein gelblicher Feststoff erhalten (87 mg, 0,184 mmol, 98 % Ausbeute).

¹H-NMR (500 MHz, CDCl₃) δ: 1.10 (br. s, 6H, CH₃), 1.44 (br. s, 6H, CH₃), 3.46 (br. s, 1H, CH), 3.74 (br. s, 1H, CH), 6.93 (d, *J* = 8.5Hz, 2H), 6.99 (t, *J* = 7.5Hz, 2H), 7.04 (d, *J* = 7.5Hz, 4H), 7.20 (m, 6H), 7.29 (d, *J* = 8.5Hz, 2H), 7.44 (d, *J* = 8.5Hz, 2H). ¹³C-NMR (CDCl₃) δ: 20.7, 45.9, 50.9, 88.0 (q), 90.7 (q), 115.7 (q), 122.2, 123.6, 124.0 (q), 125.0, 125.7, 129.4, 131.5, 132.5, 138.1 (q), 147.1 (q), 148.1 (q), 170.4 (q). HRMS m/e 473,2585 (M+H)⁺ (berechnet für C₃₃H₃₂N₂O 473,2587).

### Herstellung des Nachweisreagenz 6

4-((4-(Diphenylamino)phenyl) ethynyl)phenylboronsäure* (48 mg, 0,123 mmol) und 4-Iodo-*N,N*-diisopropylbenzamid (40 mg, 0,121 mmol) wurden in THF / Wasser (2,6 mL / 0,9 mL) unter Schutzgas gelöst und mit *trans*-Dichlorobis(triphenylphosphine)palladium (8 mg, 0,011 mmol) und Na₂CO₃ (20 mg, 0,189 mmol) versetzt und 2 h bei Raumtemperatur gerührt. Nach der Extraktion mit Ethylacetat und Wasser wurde das Rohprodukt über Na₂SO₄ getrocknet und unter vermindertem Druck aufkonzentriert. Das Rohprodukt wurde durch Säulenchromatographie an Silicagel (Cyclohexan : Ethylacetat, 5:0,5) aufgereinigt. Es wurde ein gelbbrauner Feststoff erhalten (49 mg, 0,089 mmol, 74 % Ausbeute).
*Tetrahedron 67 (2011) 6804-6811

¹H-NMR (500 MHz, CDCl₃) δ: 1.00 (br. s, 6H, CH₃), 1.35 (br. s, 6H, CH₃), 3.36 (br. s, 1H, CH), 3.73 (br. s, 1H, CH), 6.82 (d, *J* = 9.0Hz, 2H,), 6.87 (t, *J* = 7.25Hz, 2H), 6.93 (d, *J* = 7.5Hz, 4H), 7.09 (t, *J* = 8.0Hz, 4H), 7.19 (d, *J* = 2.5Hz, 2H), 7.21 (d, *J* = 2.0Hz, 2H), 7.39 (s, 4H), 7.42 (d, *J* = 8.5Hz, 2H). ¹³C-NMR (CDCl₃) δ: 20.7, 45.9, 50.9, 88.4 (q), 90.6 (q), 115.9 (q), 122.2, 122.9 (q), 123.5, 124.9, 126.2, 126.9, 127.0, 129.4, 131.9, 132.5, 137.9 (q), 139.7 (q), 140.6 (q), 147.1 (q), 147.9 (q), 170.7 (q). HRMS m/e 549,2908 (M+H)⁺ (berechnet für C₃₉H₃₆N₂O₁ 549,2900).

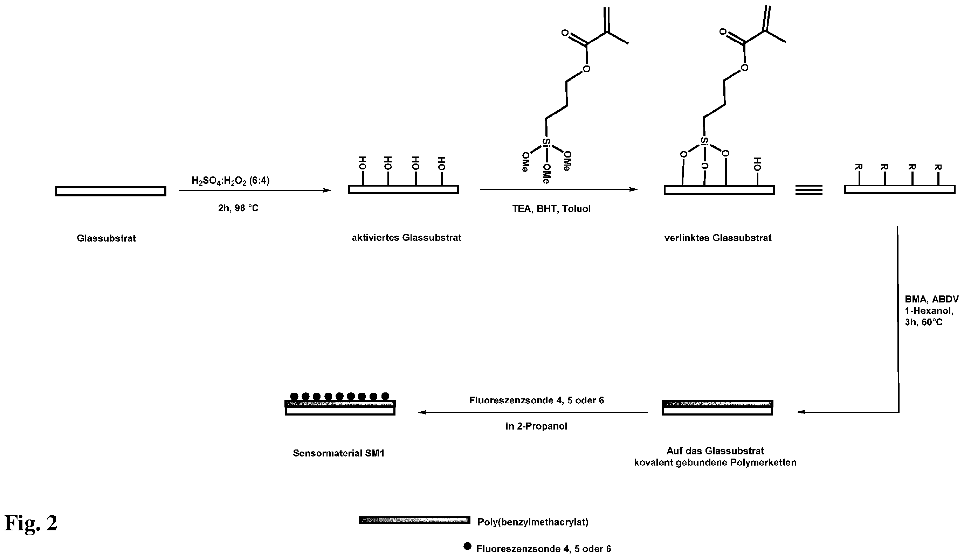

Die Herstellung von Polymerfilmen, die mit den Nachweisreagenzien **4, 5** oder **6** ausgerüstet sind, erfolgte gemäß den schematisch in den Fign. 2 bis 4 dargestellten Schritten.

### Aktivierung und Funktionalisierung bzw. Verlinkung der Glassubstratoberfläche

Die Glassubstrate wurden in Piranhasäure (40 mL H₂O₂ (30 %) und 60 mL H₂SO₄) 2 h auf 98°C geheizt. Nach dem Waschen mit deionisiertem Wasser wurden die Glassubstrate 3h in einer Soxhlet-Apparatur mit Aceton gewaschen. Nach dem Trocknen (1 h bei 150°C) wurden die Deckgläschen in Toluol (100mL) mit 3-(Trimethoxysilyl)propylmethacrylat (6 mL, 25,2 mmol), Triethylamin (1 mL, 7,2 mmol) und mit dem Radikalinhibitor 2,6-Di-tert-butyl-4-methyl-phenol (50 mg, 0,23 mmol) zwei Tage bei Raumtemperatur umgesetzt. Die Glassubstrate wurden dann 3h in der Soxhlet-Apparatur mit Ethylacetat gewaschen und anschließend unter Schutzgas gelagert. Das vorstehend beschriebene Verfahren der Aktivierung, Funktionalisierung und Verlinkung ist schematisch in Fig. 2 dargestellt.

### Herstellung von Poly(benzylmethacrylat)-, Poly(benzylacrylat)- bzw. Polystyrolfilmen auf der Glasoberfläche mittels Spin-Coating-Verfahren

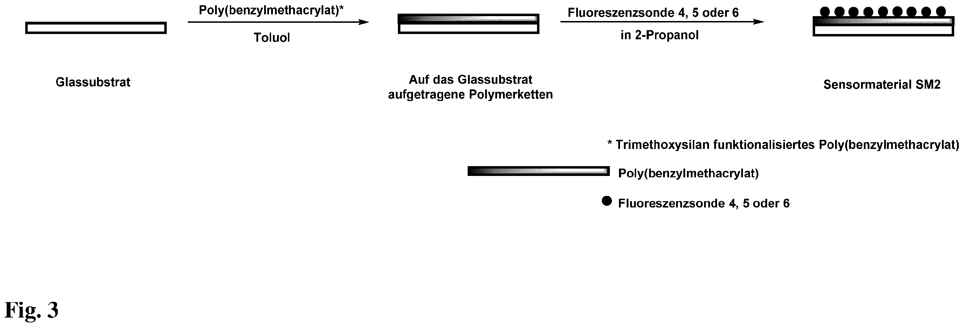

Benzylmethacrylat (600 µL, 3,5 mmol) wurde mit 3-(Trimethoxysilyl)propylmethacrylat (1µL, 0,0042 mmol) in 1-Hexanol (6 mL) mit 2,2'-Azobis(2,4-dimethylvaleronitril) (ABDV) (12mg, 0,05 mmol) versetzt und 17 h bei 60°C gerührt. Nach dem Abdampfen des Lösungsmittels wurde das Polymer mit Methanol gewaschen und im Vakuum getrocknet. 200 mg des Polymers wurden in 2 mL Toluol gelöst und 10 µL der Lösung wurden auf ein chemisch unbehandeltes Glassubstrat mittels Spin-Coating (312 rps) aufgetragen. Anschließend wurde der Polymerfilm bei 312 rps mit Methanol (250 µL) gewaschen und unter Schutzgas gelagert. Dieses Herstellungsverfahren ist aus der schematischen Darstellung in Fig. 3 ersichtlich.

### Herstellung von kovalent gebundenen Poly(benzylmethacrylat)- , Poly(benzylacrylat)- bzw. Polystyrol-Ketten auf der Glasoberfläche

Die verlinkten Glassubstrate wurden in 1-Hexanol (6 mL) mit Benzylmethacrylat (600 µL, 3,5 mmol) und mit 2,2'-Azobis(2,4-dimethylvaleronitril) (ABDV) (12mg, 0,05 mmol) versetzt und 3 h auf 60°C geheizt. Bei den eingesetzten hohen Temperaturen können jedoch auch andere Radikalstarter, wie z.B. 2,2'-Azobis(2-methylpropionitril) (AIBN) oder 2,2,6,6-Tetramethylpiperidin-1-oxyl (TEMPO) eingesetzt werden. Nach der Polymerisation wurden die Glassubstrate 1h in einer Soxhlet-Apparatur mit Ethylacetat gewaschen und anschließend unter Schutzgas gelagert. Das Verfahren ist schematisch in Fig. 2 dargestellt.

### Herstellung von Polymerfilmen aus 1,4-Bisacryloylpiperazin und 2-Hydroxyethylmethacrylat auf der Glasoberfläche

1,4-Bisacryloylpiperazin (520 mg, 2,68 mmol) wurde in 1-Hexanol (2081 µL) bei 60°C gelöst. 28 µL der Lösung wurden anschließend mit 1-Hexanol (12 µL), HEMA (3 µL, 0,025 mmol) und 2,2'-Azobis(2,4-dimethylvaleronitril) (ABDV) (0,4 mg, 0,0016 mmol) versetzt. Nach der Begasung mit Argon wurde 10 µL der Polymerisationsmischung zwischen zwei verlinkten Glassubstraten (Ø 19 mm, Stärke No. 5 bzw. 0,5 mm Schichtdicke) mit planer Oberfläche aufgetragen und die gesamte Glasoberfläche mit dem Gemisch benetzt. Anschließend wurden die Glassubstrate in 1-Hexanol gesättigter Atmosphäre unter Schutzgas 1 h auf 120°C geheizt. Nach dem Trennen der Glassubstrate wurde das untere Glassubstrat 0,5 h in der Soxhlet-Apparatur mit Ethylacetat gewaschen. Nach dem Entfernen der Polymerpartikel von der Polymerfilmoberfläche wurden diese erneut 0,5h in der Soxhlet-Apparatur mit Ethylacetat gewaschen und anschließend an der Luft getrocknet. Die schematische Darstellung dieser Arbeitsschritte ist Bestandteil der Fig. 4.

### Herstellung von Polymerfilmen aus Ethylenglykoldimethacrylat und 2-Hydroxyethylmethacrylat auf der Glasoberfläche

Ethylenglykoldimethacrylat (3 µL, 0,016 mmol) und 2-Hydroxyethylmethacrylat (7 µL, 0,058 mmol) in 1-Hexanol (40 µL) wurden mit 2,2'-Azobis(2,4-dimethylvaleronitril) (ABDV) (0,4 mg, 0,0016 mmol) versetzt. Nach der Begasung mit Argon wurde 1 µL der Polymerisationsmischung zwischen zwei verlinkten Glassubstraten (Ø 19 mm, Stärke No. 5 bzw. 0,5 mm Schichtdicke) mit planer Oberfläche aufgetragen und die gesamte Glasoberfläche mit dem Gemisch benetzt. Anschließend wurden die Glassubstrate in 1-Hexanol gesättigter Atmosphäre unter Schutzgas 1 h auf 120°C geheizt. Nach dem Trennen der Glassubstrate wurde das untere Glassubstrat 0,5 h in der Soxhlet-Apparatur mit Ethylacetat gewaschen. Nach dem Entfernen der Polymerpartikel von der Polymerfilmoberfläche wurden diese erneut 2 h in der Soxhlet-Apparatur mit Ethylacetat gewaschen und anschließend an der Luft getrocknet. Die schematische Darstellung dieser Arbeitsschritte ist Bestandteil der Fig. 4.

Nachfolgend wird die Herstellung, spezifische Eigenschaften und die Anwendung von vier Sensormaterialien **SM1- SM4** beschrieben.

### Herstellung des Sensormaterials SM1 zur Detektion von schwerflüchtigen Explosivstoffen und Nitroaromaten

Zur Herstellung des Sensormaterials **SM1** wurde der Farbstoff **4** bzw. **5** in 2-Propanol (c = 0,56 mM) im Ultraschallbad und anschließend bei 60 °C gelöst. Die mit Poly(benzylmethacrylat), Poly(benzylacrylat) bzw. Polystyrol kovalent gebundenen Glassubstrate (Ø 3-19 mm, 0,13-0,5 mm Schichtdicke) wurden mit dem Spin-Coater auf 312 rps beschleunigt, mit 100 µL 2-Propanol gewaschen und anschließend 5 µL der Farbstofflösung aufgetragen und das Glassubstrat eine Minute mit Hilfe des Spin-Coaters getrocknet. Das Sensormaterial wurde anschließend 24 h an der Luft getrocknet und anschließend Schutzgas und Lichtausschluss gelagert.

### Herstellung des Sensormaterials SM2 zur Detektion von schwerflüchtigen Explosivstoffen und Nitroaromaten

Zur Herstellung des Sensormaterials **SM2** wurde der Farbstoff **4** bzw. **5** in Methanol (c = 0,56 mM) im Ultraschallbad und anschließend bei 60°C gelöst. Die mit Poly(benzylmethacrylat), Poly(benzylacrylat) bzw. Polystyrol mittels Spin-Coating beschichteten Glassubstrate wurden auf dem Spin-Coater auf 312 rps beschleunigt, mit 250 µL Methanol gewaschen und anschließend 5µL der Farbstofflösung aufgetragen und das Glassubstrat eine Minute mit Hilfe des Spin-Coaters getrocknet. Das Sensormaterial wurde anschließend 24 h an der Luft getrocknet und unter Schutzgas und Lichtausschluss gelagert.

### Herstellung des Sensormaterials SM3 zur Detektion von DMDNB und Nitroaromaten

Zur Herstellung des Sensormaterials **SM3** wurde der Farbstoff **5** bzw. **6** in Toluol (c = 0,56 mM) gelöst. Die mit einem Poly(1,4-bisacryloylpiperazin)-Film (und Poly(2-hydroxyethylmethacrylat)) beschichteten Glassubstrate wurden auf dem Spin-Coater auf 312 rps beschleunigt, und 5 µL der Farbstofflösung aufgetragen und das Glassubstrat eine Minute mit Hilfe des Spin-Coaters getrocknet. Das Sensormaterial wurde anschließend 24 h an der Luft getrocknet und unter Schutzgas und Lichtausschluss gelagert.

### Herstellung des Sensormaterials SM4 zur Detektion von DMDNB, TNT und Nitroaromaten.

Zur Herstellung des Sensormaterials **SM4** wurde der Farbstoff **5** bzw. **6** in Toluol (c = 0,56 mM) gelöst. Die mit Poly(ethylenglykoldimethacrylat) und Poly(2-hydroxyethylmethacrylat) beschichteten Glassubstrate wurden auf dem Spin-Coater auf 312 rps beschleunigt, und 5 µL der Farbstofflösung aufgetragen und das Glassubstrat eine Minute mit Hilfe des Spin-Coaters getrocknet. Das Sensormaterial wurde anschließend 24 h an der Luft getrocknet und unter Schutzgas und Lichtausschluss gelagert.

### Herstellung des Referenzmaterials für SM1

Zur Herstellung des Referenzmaterials wurde 1 µL der Farbstofflösung **4** in 2-Propanol (c = 5,6 mM) mit Benzylmethacrylat (20 µL, 0,117 mmol), Ethylenglykoldimethacrylat (30 µL, 0,16 mmol) und mit 2,2'-Azobis(2,4-dimethylvaleronitril) (ABDV) (0,75mg, 0,003 mmol) versetzt. 0,5 µL der Lösung wurde zwischen zwei verlinkte Glassubstrate (Glas 1 = Ø 19 mm, 0,13-0,5 mm Schichtdicke; Glas 2 = Ø 5 mm, 0,13 mm Schichtdicke) aufgetragen und 20 Minuten bei 150°C polymerisiert.

### Untersuchungen zur Photostabilität der Sensormaterialien SM1-SM4 während der Messung.

Untersuchungen zur Photostabilität der Sensormaterialien **SM1** bis **SM4** erfolgten an der Luft unter Zuhilfenahme eines FluoroMax-4P Spektrofluorometers. Nach Aufnahme des Fluoreszenzspektrums (Anregung λ_{exc} = 365 nm, Spaltbreite 1,5 nm, Emission λₑₘ = 375-600 nm, Spaltbreite 5 nm) wurden die Sensormaterialien bei einer Anregungswellenlänge von λ_{exc} = 365 nm mit einer Spaltbreite von 2,5 nm 5 Minuten bestrahlt und die Änderungen des Emissionsmaximums aufgenommen. Abschließend wurde erneut das Fluoreszenzspektrum (Anregung λ_{exc} = 365 nm, Spaltbreite 1,5 nm, Emission λₑₘ = 375-600 nm, Spaltbreite 5 nm) aufgenommen (vgl. Fign. 6 und 7).

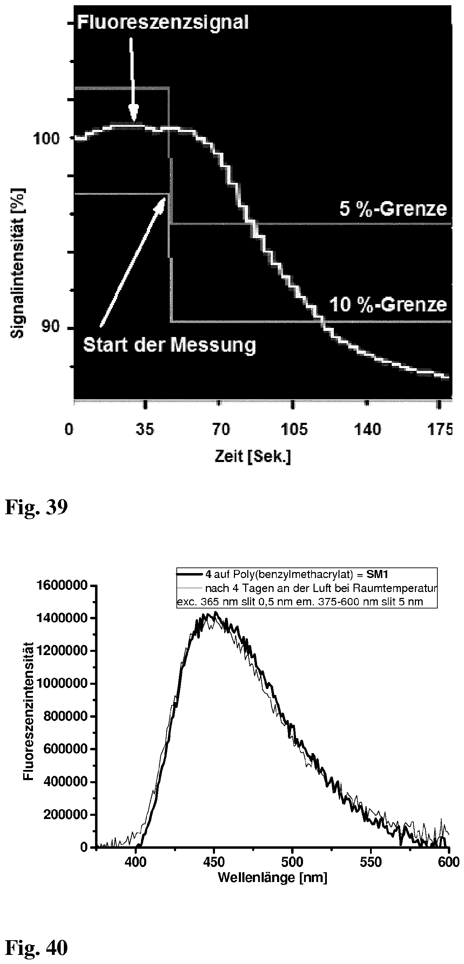

Die hervorragende Photostabilität der Nachweisreagenzien auf den entsprechenden Polymerfilmen zeigt sich insbesondere durch den Abfall des Emissionsmaximums nach 5 Minuten Dauerbestrahlung um 1,5 - 3 % mit dem Fluoreszenzspektrometer mit einer höheren Fluoreszenzanregungsintensität bei einer Wellenlänge von 365 nm bzw. 370 nm bei einer Spaltbreite von 2,5 nm (Fig. 9, 15, 18, 22, 25). Im Falle von **SM1**bleibt das Emissionssignal des Nachweisreagenz 4 auf dem Poly(benzylmethacrylat)-Film auch über mehrere Tage an der Luft bei Raumtemperatur konstant (Fig. 40)..

Die hohe Photostabilität des Nachweisreagenz **4** auf hydrophoben Oberflächen und des Nachweisreagenz **5** auf polaren Oberflächen wird durch die hohen Quantenausbeuten dieser molekularen Sonden ergänzt.

Repräsentative Spektren und spektroskopische Daten von **4** und **5** in Lösungsmitteln und Polymeren unterschiedlicher Polarität sind in den Tabellen 1 und 2 und den Fign. 6-25, 29 und 31 zusammengestellt.

**Tabelle 2**

| | | | | |
|---|---|---|---|---|
| Die Fluoreszenzquantenausbeuten des Fluoreszenzindikators **5** wurden unter Verwendung der Verbindung 2,2'-p-Phenylenebis-(5-phenyloxazol), abgekürzt auch als **POPOP** bezeichnet, (ϕ_{f}= 0,91 in EtOH)als Standard ermittelt [8]. | | | | |

| Polarität | Lösungsmittel | λ_{abs}/nm | λₑₘ/nm | φ_{f} |
|---|---|---|---|---|
| 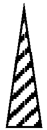 | Hexan | 353 | 382/400 | 0,32 |
| | Toluol | 358 | 404 | 0,49 |
| | CHCl₃ | 358 | 428 | 0,67 |
| | EtOH | 355 | 470 | 0,65 |
| | ACN | 353 | 472 | 0,69 |

### Untersuchungen zur Langzeitstabilität der Sensormaterialien SM1-SM4 an der Luft und unter Argon

Untersuchungen zur Langzeitstabilität der Sensormaterialien **SM1-SM4** erfolgten unter Zuhilfenahme des FluoroMax-4P Spektrofluorometers. Nach Aufnahme des Fluoreszenzspektrums (Anregung λ_{exc} = 365 nm, Spaltbreite 0,5 nm, Emission λₑₘ = 375-600 nm, Spaltbreite 5 nm) der Sensormaterialien wurden diese bei Raumtemperatur an der Luft (Fig. 9) oder Argon (Fig. 11) unter Lichtausschluss gelagert. Nach bestimmten Zeitabständen wurde die Messung wiederholt.

An Hand der vorliegenden experimentellen Befunde kann eine Aggregat-Bildung der Sonden oder Oxidation der Sonden durch Luftsauerstoff in der Matrix ausgeschlossen werden, da sich die Spektren nach 24h an der Luft oder mit Bestrahlung und Aufheizung an der Luft nicht verändern (Fig. 9). Dennoch werden, um die Passivierung der sensitiven Schicht zu vermeiden, die Sensormaterialien bis zu ihrem Einsatzzeitpunkt vorteilhaft unter einem Inertgas, beispielsweise unter Argon gelagert (Fig. 11).

Untersuchungen zur Wechselwirkungen der Sensormaterialien **SM1** - **SM4** mit Luftfeuchtigkeit und Wasser und/oder Wasserdampf.

Die Detektion von NOₓ-Explosivstoffen mit AFPs wird bekanntermaßen durch plötzliche Änderung der Luftfeuchtigkeit und durch feuchte Wischproben äußerst erschwert. Um die mit den Explosivstoffen konkurrierende Wirkung des Wassers zu umgehen, wird vorgeschlagen, hydrophobe Polymere wie Poly(benzylmethacrylat), Poly(benzylacrylat) und Polystyrol im Sensormaterial **SM1** und **SM2** einzusetzen. Über eine Komplexierung der Nachweisreagenzien durch die Polymerketten gelingt es, die Beweglichkeit der hier vorgeschlagenen Fluoreszenzsonden 4 und 5 sogar in Gegenwart von Wasserdampf bei einer Wassertemperatur von 40 - 90 °C einzuschränken und damit das Erreichen der TNT-Nachweisgrenze von 5 % Fluoreszenzlöschung zu verhindern (siehe Tabelle in[0084]).

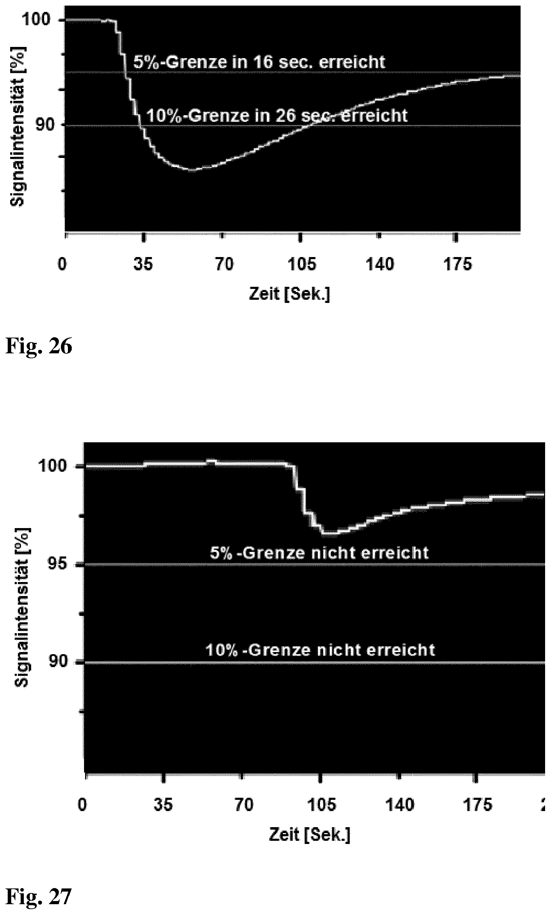

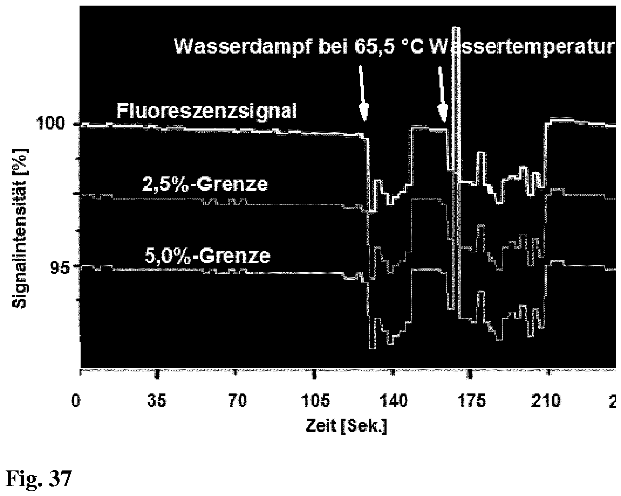

Als Ergebnis wird beim Einleiten von Wasserdampf von der Oberfläche einer 40-60°C Wasserprobe auf das Sensormaterial, gemessen 1 cm über der Wasseroberfläche, eine maximale Signalabnahme von 2,7 % beobachtet, die die untere Nachweisgrenze (5 % Signalabnahme) nicht erreicht und im Gegensatz zur Erholungsdauer der Sensormaterialien von TNT (Fig. 26) sich schnell wieder erholt (vgl. Fig. 37). Ergebnisse sind in der nachfolgenden Tab. 3 zusammengefasst.

**Tab. 3**

| **Wassertemperatur [°C]** | **Signalabnahme [%]** |
|---|---|
| 40 | 0,3 - 0,8 |
| 50 | 1,3 - 2,2 |
| 60 | 1,6 - 3,4 |
| 65,5 | 1,0 - 2,7 |
| 67,3 | 0,2 - 2,5 |
| 70 - 90 | Fluoreszenzzunahme |

Beim Einleiten von Wasserdampf von der Oberfläche einer 70-90°C Wasserprobe auf das Sensormaterial, gemessen 1 cm über der Wasseroberfläche, wird dagegen nur eine Signalzunahme beobachtet. In Kombination mit der Verdünnung des Nachweisreagenz 4 auf der Polymeroberfläche von **SM1** (75 pmol / cm²) wird dadurch der "Self-quenching"-Effekt ausgeschlossen. Für die empfindliche Detektion von Explosivstoffen sind aber für diesen Konzentrationsbereich hohe Quantenausbeuten des Nachweisreagenz nötig. Genau diese bietet die Fluoreszenzsonde **4** in hydrophober Umgebung (vgl. Tabelle 1 und 2).

Das Sensormaterial **SM3** zeigt nur im Falle einer vorübergehenden Luftfeuchtigkeitserhöhung, z.B. beim Verdampfen von reinem Wasser in unmittelbarer Nachbarschaft zur Analyt-sensitiven Schicht **SM3,** eine Löschung des Messsignals, das sich sehr schnell wieder erholt. In Gegenwart des Markerstoffes DMDNB zeigt das Sensormaterial **SM3** ebenfalls eine Fluoreszenzlöschung, die sich jedoch abhängig von der Polymermaterialzusammensetzung langsam erholt (Fig. 28).

Diese ungewöhnlich lange Erholungsphase des Sensormaterial **SM3** von einer flüchtigen Verbindung wie DMDNB ist vermutlich darauf zurückzuführen, dass das Polymer Poren enthält, die den Markerstoff DMDNB aufnehmen können. Wird aber während der Erholungsphase Wasserdampf auf das mit DMDNB kontaminierte Sensormaterial **SM3** geleitet, so erholt sich das Messsignal in wenigen Sekunden und erreicht erneut die Basislinie. Sodann kann das regenerierte Sensormaterial wieder für die nächste Messung eingesetzt werden (vgl. Fig. 28).

Die vorliegenden Ergebnisse rechtfertigen die Schlussfolgerung, dass der Wasserdampf den Markerstoff DMDNB aus den Poren des Sensormaterials **SM3** verdrängt. Aufgrund der hohen Temperaturen (>150°C) im Luftdurchlasssystem verdampft das Wasser im Polymermaterial, was eine Regenration des Messsignals bewirkt.

Vor diesem Hintergrund bieten die vorgeschlagenen Fluoreszenzfarbstoffe auf den entsprechend ihrer Polarität angepassten Polymerfilmen eine Möglichkeit für den selektiven Nachweis von Explosivstoffen auf NOₓ-Basis in der Luft, im Wasser, in organischen Lösungsmitteln und auf Oberflächen. Insbesondere wird die Verwendung von hydrophoben Poly(benzylmethacrylat)- bzw. Polystyrol-Filmen auf Glassubstraten, die mit den benannten molekularen Sonden **4** oder **5** ausgestattet sind, zur Verwendung in einem robusten Handgerät zum Nachweis von Explosivstoffen, wie TNT, Tetryl, PETN, NG, EGDN, RDX, HMX, NH₄NO₃ und des Markers DNT vorgeschlagen. Weiter wird die Verwendung des hydrophilen Poly(1,4-Bisacryloylpiperazin)/ 2-Hydroxyethylmethacrylat -Films auf einem Glassubstrat, das mit der molekular Sonde **6** ausgestattet ist, in einem robusten Handgerät zum Nachweis der leichtflüchtigen Marker DMDNB, DNT und zum Nachweis von Explosivstoffen, wie PETN, NG, EGDN, NH₄NO₃ vorgeschlagen.

Die beiliegenden Figuren veranschaulichen Ausführungsformen und dienen zusammen mit der Beschreibung der Erläuterung der Prinzipien der Erfindung. Die Elemente der Zeichnungen sind relativ zueinander und nicht notwendigerweise maßstabsgetreu. Gleiche Bezugszeichen bezeichnen entsprechend ähnliche Teile.
- Fig. 1: zeigt ein Syntheseschema zur Darstellung der Fluoreszenzsonden **4, 5** und **6;**
- Fig. 2: zeigt schematisch eine Methode zur Herstellung des Sensormaterials **SM1**, die kovalente Bindung der Polymerketten mit den verlinkten Glassubstraten und die Beschichtung der auf das Glassubstrat kovalent gebundenen Polymerketten mit den Fluoreszenzsonden **4, 5** und **6;**
- Fig. 3: zeigt schematisch eine Methode zur Herstellung des Sensormaterials **SM2,** die Herstellung der mit Poly(benzylmethacrylat) beschichteten Glassubstrate mittels Spin-Coating, und die Beschichtung der auf das Glassubstrat aufgetragenen Polymerketten mit den Fluoreszenzsonden **4, 5 und 6;**
- Fig. 4: zeigt schematisch eine Methode zur Herstellung der Sensormaterialien **SM3** und **SM4,** umfassend die Herstellung der verlinkten Glassubstrate, die kovalente Bindung des quervernetzten Polymers mit den verlinkten Glassubstraten und die Beschichtung der auf das Glassubstrat kovalent gebundenen Polymermatrix mit den Fluoreszenzsonden **4, 5** und **6;**
- Fig. 5: zeigt Maldi-Spektren von Poly(benzylmethacrylat) aus der 1-Hexanol Lösung des bei der Substratbehandlung ausgefallenen Polymers.
- Fig. 6: zeigt das Absorptionsspektrum vom Nachweisreagenz **4** auf chemisch unbehandeltem Glas, auf "verlinktem" Glas und auf Poly(benzylmethacrylat) beschichtetem Glas (**SM1**).
- Fig. 7: zeigt das Fluoreszenzanregungsspektrum vom Nachweisreagenz 4 auf chemisch unbehandeltem Glas, auf "verlinktem" Glas und auf Poly(benzylmethacrylat) beschichtetem Glas (**SM1**).
- Fig. 8: zeigt das Fluoreszenzspektrum vom Nachweisreagenz 4 auf chemisch unbehandeltem Glas, auf "verlinktem" Glas und auf Poly(benzylmethacrylat) beschichtetem Glas (**SM1**).
- Fig. 9: zeigt Ergebnisse bzw. das Fluoreszenzspektrum von Untersuchungen zur Photostabilität des Nachweisreagenz **4** auf Poly(benzylmethacrylat) beschichtetem Glas (**SM1**) vor und nach Dauerbestrahlung bei höherer Anregungsintensität;
- Fig. 10: zeigt Ergebnisse bzw. das Fluoreszenzspektrum von Untersuchungen zur Photo- und Luftstabilität des Nachweisreagenz **4** auf "verlinktem" Glas vor und nach Dauerbestrahlung bei höherer Anregungsintensität und nach anschließender Lagerung an der Luft für 24 Stunden;
- Fig. 11: zeigt Ergebnisse bzw. das Fluoreszenzspektrum von Untersuchungen zur Langzeitstabilität des Nachweisreagenz **4** auf Poly(benzylmethacrylat) beschichtetem Glas (**SM1**) unter Argon-Schutzgas nach drei Tagen, nach einer Woche und nach zwei Wochen;

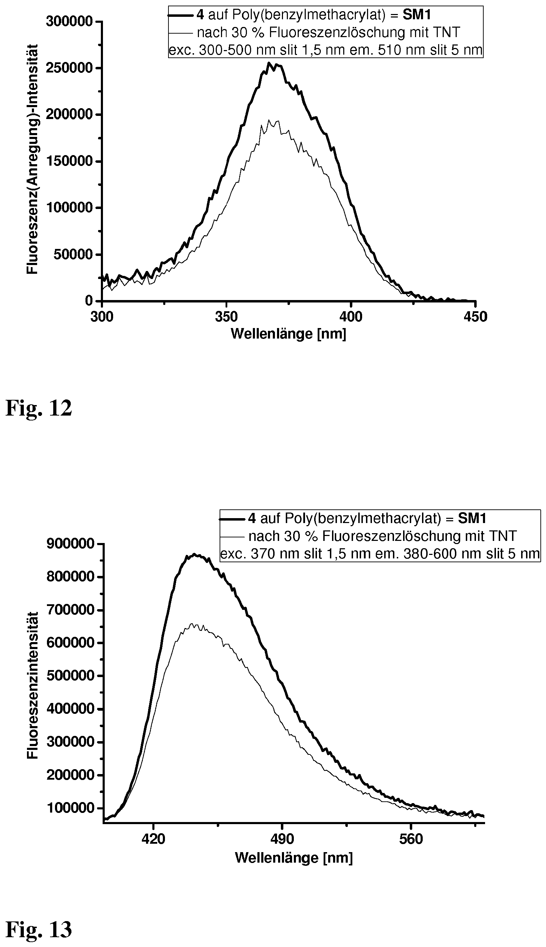

- Fig. 12: zeigt das Fluoreszenzanregungsspektrum des Nachweisreagenz **4** auf Poly(benzylmethacrylat) beschichtetem Glas (**SM1**) vor und nach der 30 %-igen Lösung der Fluoreszenz durch TNT in der Luft bei Raumtemperatur;
- Fig. 13: zeigt das Fluoreszenzspektrum des Nachweisreagenz **4** auf Poly(benzylmethacrylat) beschichtetem Glas (**SM1**) vor und nach der 30 %-igen Lösung der Fluoreszenz durch TNT in der Luft bei Raumtemperatur;

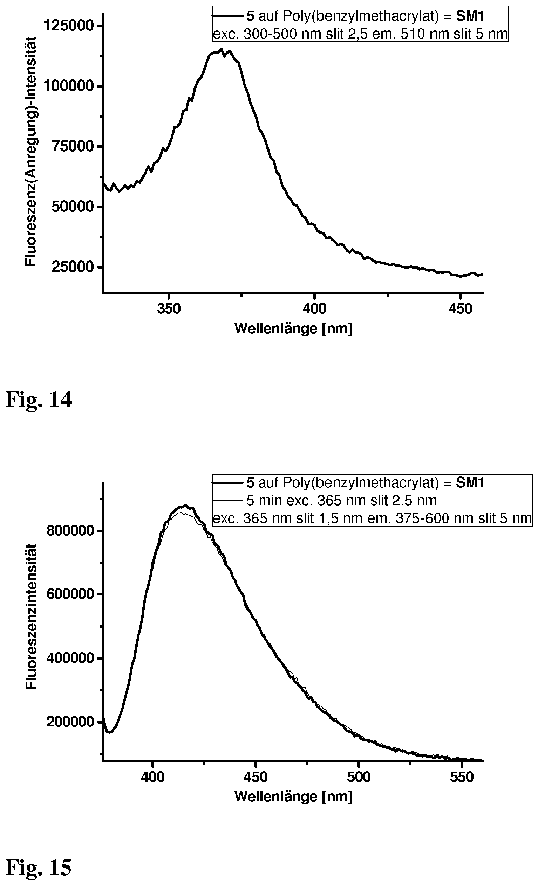

- Fig. 14: zeigt das Fluoreszenzanregungsspektrum vom Nachweisreagenz **5** auf Poly(benzylmethacrylat) beschichtetem Glas (**SM1**).
- Fig. 15: zeigt Ergebnisse bzw. das Fluoreszenzspektrum von Untersuchungen zur Photostabilität des Nachweisreagenz **5** auf Poly(benzylmethacrylat) beschichtetem Glas (**SM1**) vor und nach Dauerbestrahlung bei höherer Anregungsintensität;

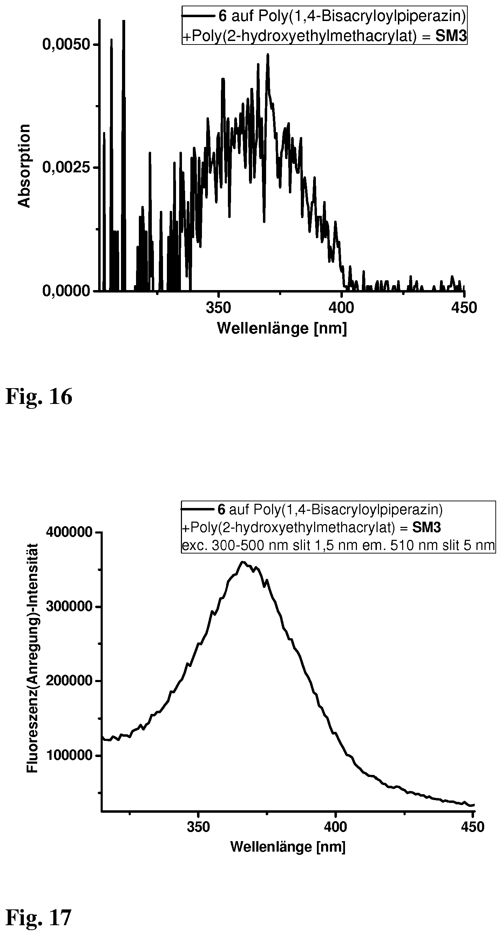

- Fig. 16: zeigt das Absorptionsspektrum vom Nachweisreagenz **6** auf 1,4-Bisacryloylpiperazin / 2-hydroxyethylmethacrylat polymerisiertem Glas (**SM3**).
- Fig. 17: zeigt das Fluoreszenz-Anregungsspektrum vom Nachweisreagenz 6 auf 1,4-Bisacryloylpiperazin / 2-hydroxyethylmethacrylat polymerisiertem Glas (**SM3**).

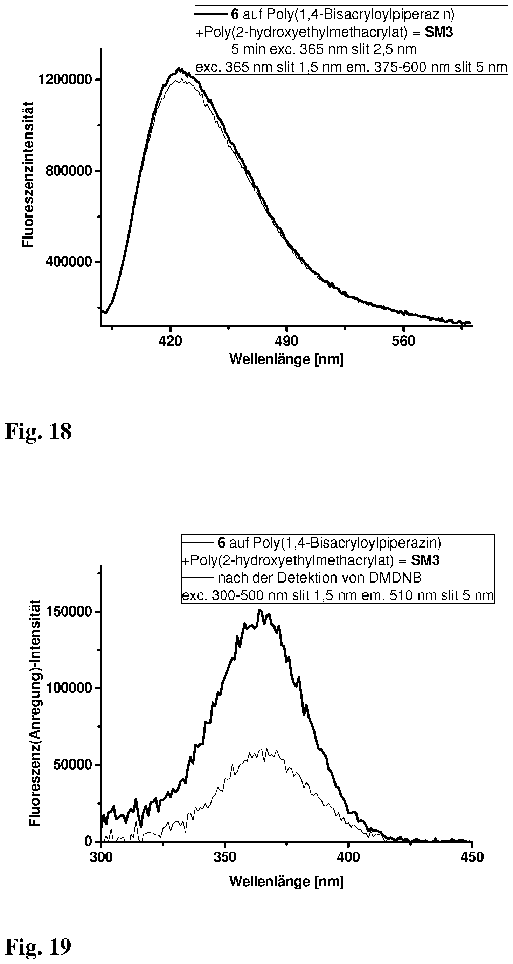

- Fig. 18: zeigt Ergebnisse bzw. das Fluoreszenzspektrum von Untersuchungen zur Photostabilität des Nachweisreagenz **6** auf 1,4-Bisacryloylpiperazin / 2-hydroxyethylmethacrylat polymerisiertem Glas (**SM3**) vor und nach Dauerbestrahlung bei höherer Anregungsintensität;
- Fig. 19: zeigt das Fluoreszenzanregungsspektrum vom Nachweisreagenz **6** auf 1,4-Bisacryloylpiperazin / 2-hydroxyethylmethacrylat polymerisiertem Glas (**SM3**) vor und nach der >50 %-igen Löschung der Fluoreszenz durch DMDNB in der Luft bei Raumtemperatur;

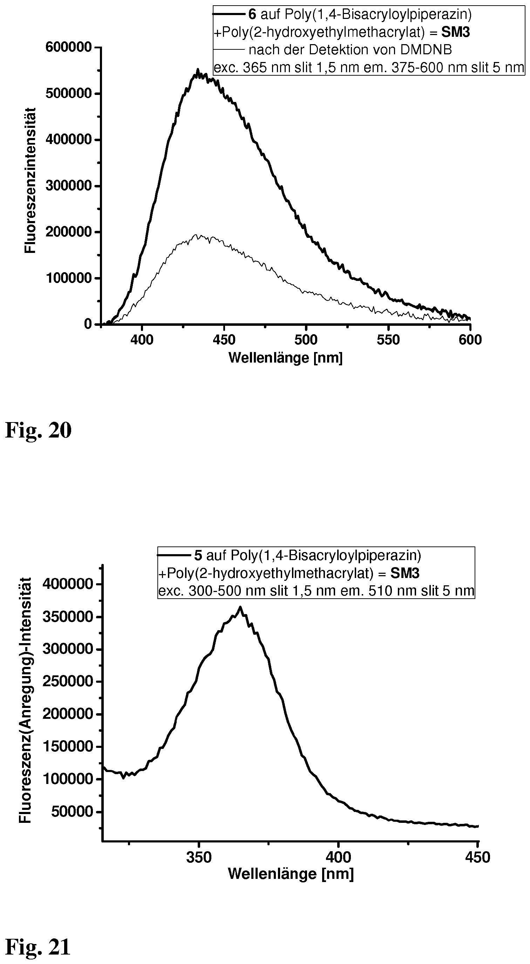

- Fig. 20: zeigt das Fluoreszenzspektrum vom Nachweisreagenz **6** auf 1,4-Bisacryloylpiperazin / 2-hydroxyethylmethacrylat polymerisiertem Glas (**SM3**) vor und nach der >50 %-igen Löschung der Fluoreszenz durch DMDNB in der Luft bei Raumtemperatur;
- Fig. 21: zeigt das Fluoreszenzanregungsspektrum vom Nachweisreagenz **5** auf 1,4-Bisacryloylpiperazin / 2-hydroxyethylmethacrylat polymerisiertem Glas (**SM3**);

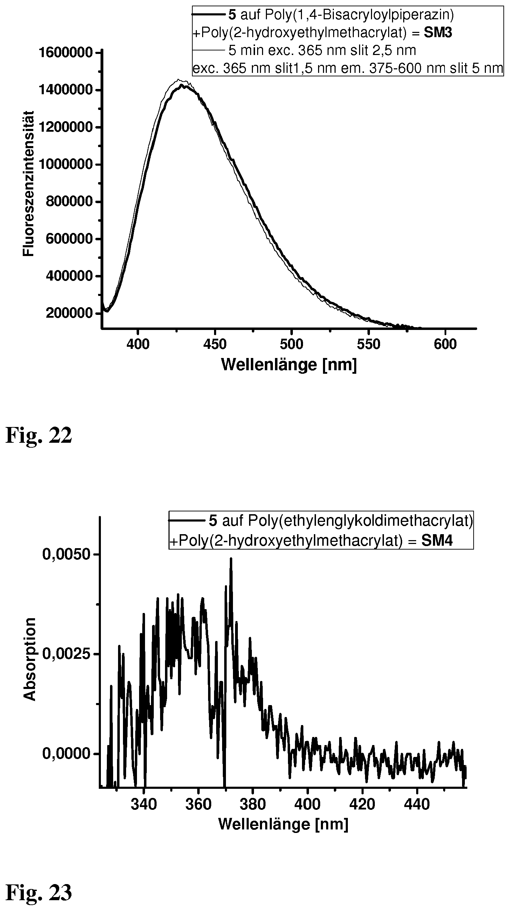

- Fig. 22: zeigt das Fluoreszenzspektrum vom Nachweisreagenz **5** auf 1,4-Bisacryloylpiperazin / 2-hydroxyethylmethacrylat polymerisiertem Glas (**SM3**) vor und nach Dauerbestrahlung bei höherer Anregungsintensität;
- Fig. 23: zeigt das Absorptionsspektrum vom Nachweisreagenz **5** auf Ethylenglykoldimethacrylat / 2-hydroxyethylmethacrylat polymerisiertem Glas (**SM4**).

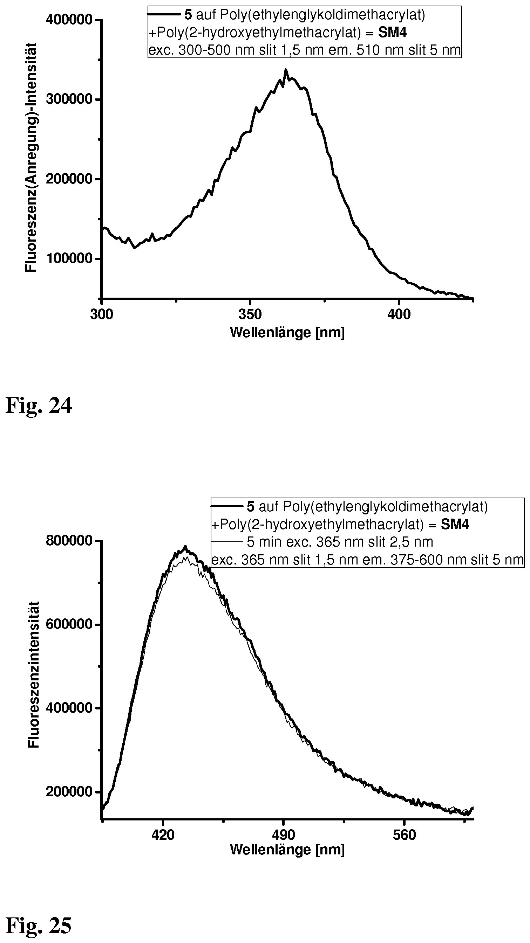

- Fig. 24: zeigt das Fluoreszenzanregungsspektrum vom Nachweisreagenz **5** auf Ethylenglykoldimethacrylat / 2-hydroxyethylmethacrylat polymerisiertem Glas (**SM4**).
- Fig. 25: zeigt das Fluoreszenzspektrum vom Nachweisreagenz **5** auf Ethylenglykoldimethacrylat / 2-hydroxyethylmethacrylat polymerisiertem Glas (**SM4**) vor und nach Dauerbestrahlung bei höherer Anregungsintensität;
- Fig. 26: zeigt die Löschung (links) mit 1,9 ng TNT Wischprobe, die am Messkopf des Handgeräts auf 150 °C geheizt wird und die Zunahme des Fluoreszenzsignals (obere Linie) des Sensormaterials **SM1** bei Regeneration (rechts), gemessen im Handgerät. Rot markiert ist die 5 %-Grenze (mittlere Linie), die in 16 Sekunden erreicht wird und blau markiert die 10 %-Grenze (untere Linie) einer 10 %-igen Fluoreszenzlöschung, die in 26 Sekunden erreicht wird;
- Fig. 27: zeigt die Löschung (links) mit 2,2 ng Moschus Ambrette Wischprobe, die am Messkopf des Handgeräts auf 150 °C geheizt wird und die Zunahme des Fluoreszenzsignals (obere Linie) des Sensormaterials **SM1** bei Regeneration (rechts), gemessen im Handgerät. Rot markiert ist die 5 %-Grenze (mittlere Linie) und blau markiert die 10 %-Grenze (untere Linie), die nicht erreicht werden;
- Fig. 28: zeigt die Löschung (links) mit DMDNB bei Raumtemperatur in der Luft und die Zunahme des Fluoreszenzsignals (obere Linie) des Sensormaterials **SM3** bei Regeneration (rechts) mit Wasserdampf, der durch Verdampfen von 2 µL Wasser am 120 - 150 °C heißem Messkopf gebildet wurde, gemessen im Handgerät. Rot markiert ist die 25 %-Grenze (mittlere Linie), die in 13 Sekunden erreicht wird und blau markiert die 50 %-Grenze (untere Linie) einer 50 %-igen Fluoreszenzlöschung, die in 35 Sekunden erreicht wird;
- Fig. 29: zeigt im Fluoreszenzspektrum die Abnahme der Fluoreszenzintensität des Sensormaterials **SM1**, umfassend die Fluoreszenzsonde **4** auf einem apolaren Polymerfilm, hergestellt ausgehend von Benzylmethacrylat (BMA) vor und nach der 84 %-igen Löschung der Fluoreszenz durch eine TNT gesättigten Luftatmosphäre bei Raumtemperatur innerhalb von 10 Minuten. Anregung erfolgte mittels UV (370 nm), das Emissionssignal wurde im Wellenlängenbereich 380 - 600 nm erfasst;

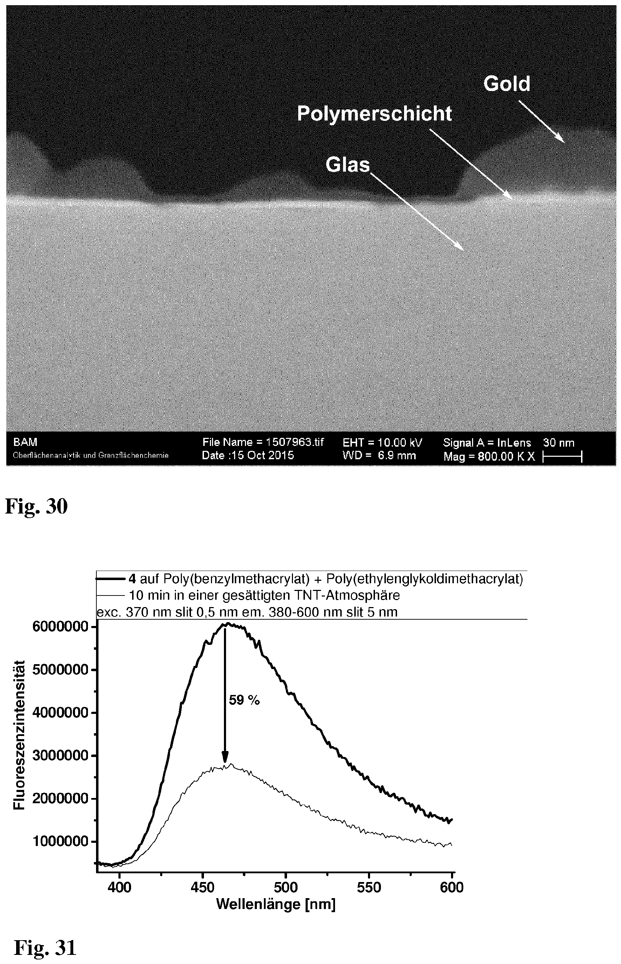

- Fig. 30: zeigt die Querschnittsaufnahme eines mit Gold bedampften Sensormaterials **SM1** unter einem Rasterelektronenmikroskop. In der Abbildung ist die Struktur des gebrochenen Trägersubstrates (Glas) (unten) mit dem darauf angeordneten Polymerfilm (Mitte) und der Goldschicht (oben). Die Schichtdicke des Polymerfilms beträgt < 5 nm;
- Fig. 31: zeigt im Fluoreszenzspektrum die Abnahme der Fluoreszenzintensität der Fluoreszenzsonde **4** auf einem apolaren Polymerfilm, hergestellt ausgehend von Benzylmethacrylat (BMA) und Ethylenglykoldimethacrylat (EDMA) vor und nach der 59 %-igen Löschung der Fluoreszenz durch eine TNT gesättigten Luftatmosphäre bei Raumtemperatur innerhalb von 10 Minuten. Anregung erfolgte mittels UV (370 nm), das Emissionssignal wurde im Wellenlängenbereich 380 - 600 nm erfasst;

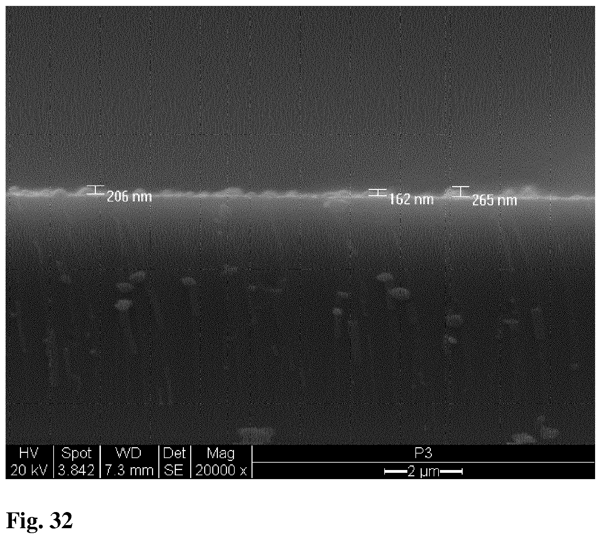

- Fig. 32: zeigt die Querschnittsaufnahme des mit dem Nachweisreagenz **4** beschichteten Polymerfilms hergestellt aus Benzylmethacrylat (BMA) und Ethylenglycoldimethacrylat (EDMA) unter einem Rasterelektronenmikroskop. In der Abbildung ist die Struktur des gebrochenen Trägersubstrates (Glas) (unten) mit dem darauf angeordneten Polymerfilmpartikeln mit dem Durchmesser 160 - 265 nm zu sehen. Beim Brechen des Glassubstrates mit der Analyt-sensitiven Schicht gelangten Polymerpartikel aus der Analyt-sensitiven Schicht auf die Bruchfläche des Glassubstrates;

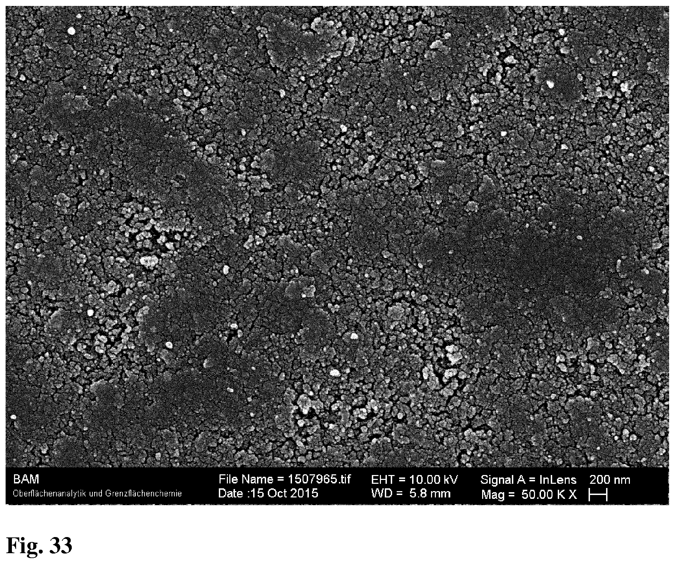

- Fig. 33: zeigt die Oberfläche eines mit Gold bedampften Sensormaterials **SM3** unter einem Rasterelektronenmikroskop. In der Abbildung ist die Struktur der Poren zu sehen, die die Diffusion des Analyten in das Polymer erlauben;

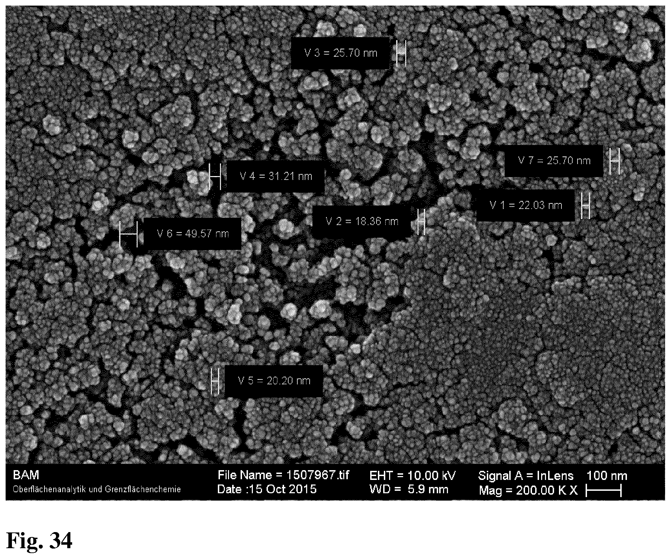

- Fig. 34: zeigt die Oberfläche eines mit Gold bedampften Sensormaterials **SM3** unter einem Rasterelektronenmikroskop bzw. die Vergrößerung der Abbildung in Fig. 33. In der Abbildung sind die Polymerpartikeln sowie die Struktur der Poren zu sehen. Die Durchmesser der Polymerpartikeln beträgt 18 - 25 nm. Die Porengröße beträgt 30 - 50 nm;
- Fig. 35: zeigt die Querschnittsaufnahme eines mit Gold bedampften Sensormaterials **SM3** unter einem Rasterelektronenmikroskop. In der Abbildung ist die Struktur des gebrochenen Trägersubstrates (Glas) (rechts) mit dem darauf angeordneten Polymerfilm. Die Schichtdicke des Polymerfilms beträgt 135 - 150 nm;
- Fig. 36: zeigt die Querschnittsaufnahme eines mit Gold bedampften Sensormaterials **SM4** unter einem Rasterelektronenmikroskop. In der Abbildung ist die Struktur des gebrochenen Trägersubstrates (Glas) (unten) mit dem darauf angeordneten Polymerfilm. Die Schichtdicke des Polymerfilms beträgt 160 - 325 nm;
- Fig. 37: zeigt die Löschung der Fluoreszenz des Sensormaterials **SM1** im Handgerät bei zwei Messungen von Wasserdampf 1 cm über der Wasseroberfläche bei einer Wassertemperatur von 65,5 °C und einer Messkopftemperatur von 120 °C um maximal 2,7 %. Rot markiert ist die 2,5 %-Grenze (mittlere Linie) und blau markiert die 5 %-Grenze (untere Linie), die nicht erreicht werden;

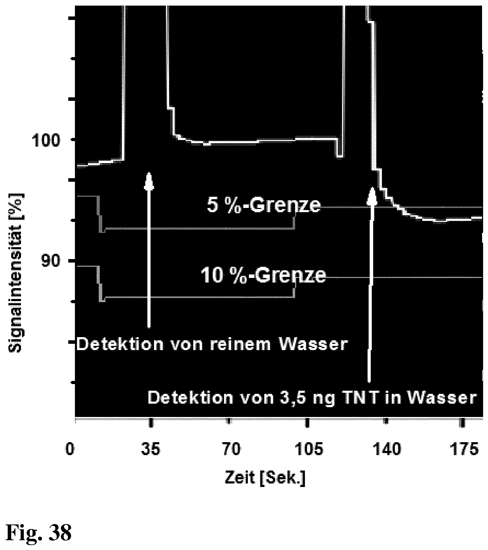

- Fig. 38: zeigt das Signal des Sensormaterials **SM1** im Handgerät vor und nach der Messung von jeweils 3µL wässriger Proben ohne und mit TNT (1,18mg/L, c = 5,2 µM), die auf dem auf 150 °C geheizten Messkopf des Handgeräts verdampft werden. Bei der Messung von reinem Wasser wird eine Fluoreszenzverstärkung beobachtet, die wieder in den Ausgangszustand zurückkehrt. Im Falle von TNT kontaminierten Proben wird zunächst ebenfalls eine Fluoreszenzverstärkung beobachtet, wobei jedoch bei der Erholung des Signals dieser nach dem Erreichen des Ausgangszustandes weiter bis zur 5 %-TNT-Nachweisgrenze fällt und diesen in 39 Sekunden erreicht.
- Fig. 39: zeigt das Fluoreszenzsignal des Sensormaterials **SM1** im Handgerät vor und nach der Messung über einer wässrigen TNT-Probe (1,18mg/L, c = 5,2 µM). Die von TNT verursachte Fluoreszenzlöschung erreicht dabei 5 %-TNT-Nachweisgrenze in 32 Sekunden und die 10 %-TNT-Nachweisgrenze in 58 Sekunden.
- Fig. 40: zeigt Ergebnisse bzw. das Fluoreszenzspektrum von Untersuchungen zur Luftstabilität des Nachweisreagenz **4** auf Poly(benzylmethacrylat) beschichtetem Glas (**SM1**) vor und nach 4 Tagen an der Luft bei Raumtemperatur;

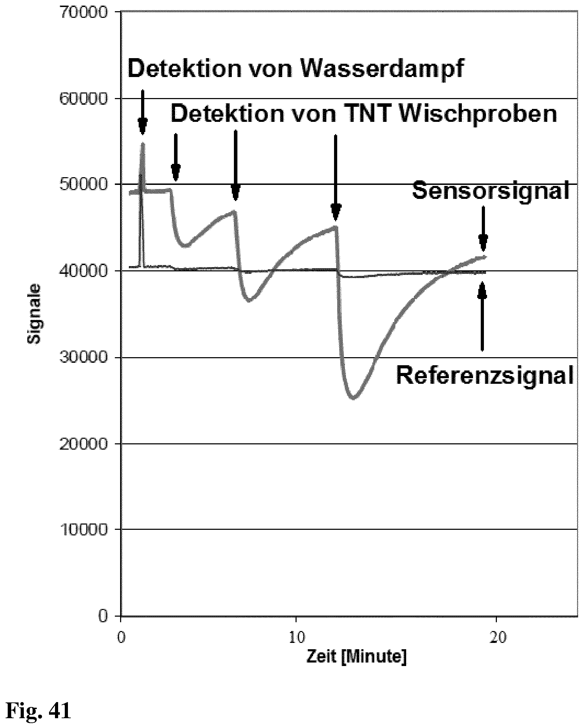

- Fig. 41: zeigt in einem dualen Messsystem, bei dem das Sensormaterial **SM1** und das entsprechende Referenzmaterial im Handgerät gleichmäßig vom Analyten benetzt werden, die Änderungen der Fluoreszenzsignale in Gegenwart von Wasserdampf und TNT. Zu sehen ist die Zunahme des Fluoreszenzsignals (links) des Sensormaterials **SM1** und des entsprechenden Referenzmaterials verursacht durch Verdampfen von einem Tropfen Wasser am Messkopf des Handgeräts, der auf 150 °C geheizt wird. Beim Ausheizen von drei TNT Wischproben unterschiedlicher Konzentration am Messkopf des Handgeräts nimmt zunächst das Fluoreszenzsignal des Sensormaterials **SM1** in 18 Sekunden bis zu 13 - 44 % ab und nimmt in der Regenerierungsphase wieder zu. Das Referenzmaterial zeigt dagegen beim Ausheizen der drei TNT Wischproben eine maximale Fluoreszenzlöschung von 2 %.

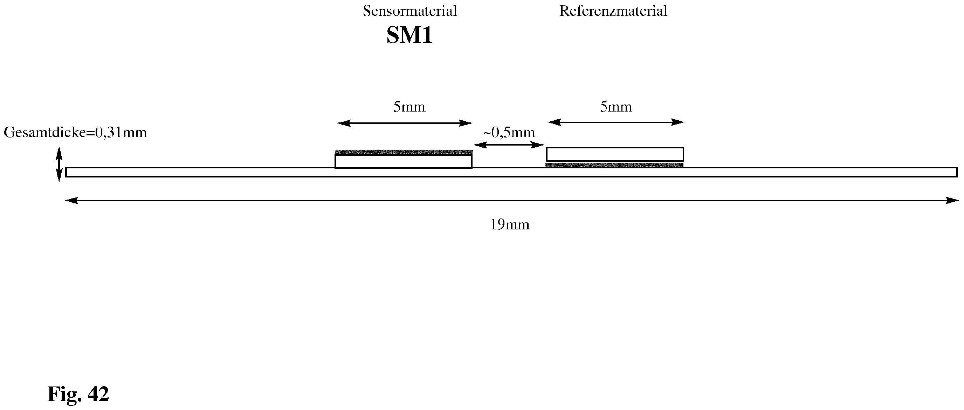

- Fig. 42: zeigt das Sensormaterial **SM1** auf einem Glassubstrat (∅ 5 mm, 0,13 mm Schichtdicke), das auf ein Glassubstrat (∅ 19 mm, 0,13 mm Schichtdicke) mit einem optischen Kleber aufgeklebt wurde. Das entsprechende Referenzmaterial befindet sich zwischen einem ∅ 5 mm und einem ∅ 19 mm Glassubstrat (0,13 mm Schichtdicke).

### Detaillierte Beschreibung des Messverfahrens

Das vorgeschlagene Nachweisverfahren basiert auf der Bereitstellung eines Nachweisreagenz, das an fester Phase adsorbiert vorliegt. Bevorzugt ist die feste Phase ein homogener Polymerfilm konstanter Dicke auf einem Glassubstrat. Der Polymerfilm ist mit einer fluoreszierenden, molekularen Sonde beschichtet, die unter den Messbedingungen als spezifisches Nachweisreagenz für praktisch relevante NOₓ-Explosivstoffe und Markerstoffe (z.B. für TNT und DMDNB) dient. Die Fluoreszenzsonde umfasst einen Triphenylamin-Kern und eine kovalent mit dem Kern in *para*-Position über eine Dreifachbindung verknüpfte elektronenziehende Phenyl-Einheit. Unter einer Fluoreszenzsonde wird in diesem Zusammenhang ein durch spezifische Fluoreszenzeigenschaften das Vorhandensein eines Explosivstoffes anzeigendes Molekül, vorliegend also ein Triphenylamin-Derivat verstanden, das in einer speziell für die Komplexierung des entsprechenden Explosivstoffes angepassten Farbstoff-Polymer-Komposition vorliegt.

Unter einer Rezeptor-Einheit wird in diesem Zusammenhang ein spezifisch mit dem nachzuweisenden NOₓ-Explosivstoff in Wechselwirkung tretendes Motiv, umfassend ein Phenylamino-Derivat verstanden, das durch Abgabe von einem Elektron an den Akzeptor (NOₓ-Explosivstoff) zur Ausbildung von Radikalkationen fähig ist. Die Rezeptor-Einheit, umfassend die Phenylamino-Gruppe ist so ausgewählt, dass sie das Radikalkation stabilisieren kann. Die beiden unsubstituierten Phenyl-Reste der Phenylamino-Gruppe erlauben sterisch eine schnelle Wechselwirkung mit dem Explosivstoff und erhöhen zugleich die Fluoreszenzquantenausbeute der molekularen Sonde.

Die Rezeptor-Einheit der molekularen Sonde ist weiterhin so angepasst, dass sie einerseits als Donor ein Elektron an den Explosivstoff abgibt und andererseits in Abhängigkeit von der Flüchtigkeit des Explosivstoffes bzw. von dessen Verweilzeit auf der Sensoroberfläche dieses wieder aufnimmt. Somit wird die stattgefundene Bindung des Explosivstoffes zur Rezeptor-Einheit mit hoher Empfindlichkeit an Hand einer Veränderung fluoreszenzoptischer, insbesondere fluoreszenzspektroskopischer Charakteristika der Fluoreszenzsonde nachgewiesen, wobei sich typischerweise die Lage des Absorptionsmaximums der gebunden im besagten Abschnitt vorliegenden Fluoreszenzsonde für eine Anregungsstrahlung nicht signifikant ändert. Das erleichtert das Auslesen der Messwerte mit einem bei fester Anregungswellenlänge (z.B. einer LED) arbeitenden, üblicherweise kostengünstigen und robusten portablen Lesegerät ("Handgerät").

Bevorzugt entspricht eine je Zeiteinheit (bei gegebener Temperatur) von der Sonde gebundene Menge der NOₓ-Verbindung einer definierten Konzentration des Explosivstoffes in der Luft oder als Wasserprobe oder Wischprobe mit zunächst noch unbekannter Konzentration des Explosivstoffes einer definierten Probenmasse mit einem zunächst noch unbekannten Gehalt des Explosivstoffes. Naturgemäß hat die Temperatur einen gewissen Einfluss auf die Gleichgewichtseinstellung auf molekularer Ebene. Durch eine geeignete Kalibrierung können eventuell störende Einflüsse, wie die temperaturabhängige Regenerierung der Sensorschichten den Messbedingungen angepasst werden. Damit sind die Fluoreszenzsonden für die vorgeschlagene Detektion von Explosivstoffen in einem Temperaturbereich 0 - 130 °C problemlos einsetzbar.

Die vorgeschlagenen Fluoreszenzsonden **SM1** und **SM2** sind besonders für den Nachweis folgender Explosivstoffe und Marker geeignet: TNT, DNT, Tetryl, PETN, NG, EGDN, RDX, HMX und NH₄NO₃.

Die vorgeschlagene Fluoreszenzsonde **SM3** ist besonders für den Nachweis folgender Explosivstoffe und Markerstoffe geeignet: DNT, DMDNB, PETN, NG, NH₄NO₃ und EGDN.

Die vorgeschlagene Fluoreszenzsonde **SM4** ist besonders für den Nachweis folgender Explosivstoffe und Marker geeignet: TNT, DNT, DMDNB, PETN, NG, NH₄NO₃ und EGDN.

Dementsprechend wird ein Nachweisverfahren zum quantitativen und qualitativen Nachweis dieser Explosivstoffe in der Luft, als Wischproben von Oberflächen und in Wasserproben vorgeschlagen. Das Nachweisverfahren zeichnet sich insbesondere dadurch aus, dass es problemlos auch vom nicht speziell geschulten Anwender durchführbar ist und auf teure, laborgebundene Messtechnik verzichtet werden kann.

Wie am Beispiel der Verbindungen **4, 5** und **6** gezeigt, kann die Luftstabilität und die Sensitivität der Indikatoren durch das Polymermaterial modifiziert und beispielsweise die Selektivität des nachzuweisenden NOₓ-Explosivstoff erhöht werden. Bei leichtflüchtigen Nitroverbindungen wie dem Marker DMDNB ist die einzige Konsequenz, dass dieser auf einer dünnen Polymeroberfläche wie Poly(benzylmethacrylat) nicht angereichert wird, weil dazu die Poren fehlen. Daher ist die Verweilzeit von DMDNB auf diesem Polymerfilm sehr kurz was die geringe Fluoreszenzlöschung und damit die schwache Wechselwirkung mit den molekularen Sonden erklärt.

Die Zusammensetzung des Polymermaterials zur Detektion der schwerflüchtigen Explosivstoffe wie TNT und RDX ist anscheinend von untergeordneter Bedeutung. Besser geeignet zur Kontrolle der Sensitivität einer Analyt-sensitiven Schicht ist die Schichtdicke des Polymerfilms bzw. die Schichtdicke der molekularen Sonde(n). Nach gegenwärtigem Verständnis wird eine gesteigerte Selektivität durch die Reduzierung der Konzentration des Indikators und durch eine höhere Temperatur auf dem Sensorfilm erreicht. Ergebnisse sind in der nachfolgenden Tabelle 4 zusammengefasst.

**Tab. 4**

| **Messkopftemp. [°C]** | **Konz. [µM]** | **TNT (5%) [sek.**] | **MA (5 %) [sek.**] | **TNT (10 %) [sek.**] | **MA (10 %) [sek.**] |
|---|---|---|---|---|---|
| 115 | 556 | 41 | 61 | 63 | 123 |
| 115 | 278 | 59 | 101 | 91 | e. |
| 115 | 222 | 55 | 201 | 85 | e. |
| 115 | 111 | 68 | n.e. | 102 | n.e. |
| 115 | 56 | 91 | n.e. | n.e. | n.e. |
| 150 | 556 | 29 | 52 | 42 | 159 |
| 150 | 278 | 29 | 91 | 52 | e. |
| 150 | 222 | 31 | 74 | 47 | 269 |
| 150 | 111 | 46 | 185 | 78 | e. |
| 150 | 56 | 71 | e. | n.e. | n.e. |

### Erläuterungen:

Messkopftemp. = Messkopftemperatur bzw. die Temperatur am Luftprobeneinlass am Handgerät. Der Luftprobeneinlass bzw. Auslass des verwendeten Handgerätes ist trichterförmig und auf eine wahlweise einstellbare Temperatur beheizbar. Die hier typischerweise verwendete Temperatur des Messkopfes betrug 150 °C. Das ermöglicht die Resublimation von schwerflüchtigem Probenmaterial auf der Analyt-sensitiven Schicht.
Konz. [µM] = Konzentrationen von Nachweisreagenz **4** in 2-Propanol, jeweils mittels Spin-Coating auf das jeweilige Polymer aufgetragen.
TNT (5 %) = Fluoreszenzlöschung des Anfangssignals um 5 % bei der Messung von einem
TNT-Prüfkörper bei Raumtemperatur mit einer Konzentration in der Luft im ppb-Bereich.
MA (5 %) = Fluoreszenzlöschung des Anfangssignals um 5 % bei der Messung von einem Moschus Ambrette-Prüfkörper bei Raumtemperatur mit einer Konzentration in der Luft im ppb-Bereich.
TNT (10 %) = Fluoreszenzlöschung des Anfangssignals um 10 % bei der Messung von einem
TNT-Prüfkörper bei Raumtemperatur mit einer Konzentration in der Luft im ppb-Bereich.
MA (10 %) = Fluoreszenzlöschung des Anfangssignals um 10 % bei der Messung von einem Moschus Ambrette-Prüfkörper bei Raumtemperatur mit einer Konzentration in der Luft im ppb-Bereich.
e. = die Nachweisgrenze von 5 % bzw. 10 % wurde nach 5 Minuten erreicht.
n.e. = die Nachweisgrenze von 5 % bzw. 10 % wurde auch nach 5 Minuten nicht erreicht.

Die Verweildauer der Explosivstoffe auf der Analyt-sensitiven Schicht kann auch über die durch Heizen einstellbare Temperatur des Sensormaterials gesteuert werden. Unter den für das jeweils verwendete Messgerät optimierten Bedingungen kann TNT die Fluoreszenz der Analyt-sensitiven Schicht stetig löschen, während strukturverwandte Moschus-Verbindungen auf denselben Analyt-sensitiven Schichten **(SM1** und **SM2)** ein schnelles Adsorptions-Desorptions-Gleichgewicht erreichen und damit den Zielwert für die Fluoreszenzlöschung nicht erreichen. Auf diese Art und Weise ist ein spezifischer Zuschnitt der molekularen Sonde möglich. Vorteilhaft weisen die hier vorgeschlagenen Nachweisreagenzien durch das Fehlen sterisch anspruchsvoller Gruppen (z.B. *tert*-Butyl-Reste) eine molekulare Konfiguration auf, die eine erleichterte Wechselwirkung mit den Analyten zulässt. Bei den konjugierten Polymeren ist der Einbau sterisch anspruchsvoller Gruppen nötig, um die Selbstlöschung der Fluoreszenz durch die Aggregatbildung zu verhindern. Im Falle der Sensormaterialien **SM1** - **SM4** übernimmt das nicht fluoreszierende Polymer diese Aufgabe. Daher ist der Einbau sterisch anspruchsvoller Gruppen in die Farbstoffstruktur der Nachweisreagenzien **4 - 6** nicht erforderlich. Auf die Selektivität der Sensormaterialien haben solche sterisch anspruchsvollen Gruppen nur wenig Einfluss, sodass die vorgeschlagene Verwendung des Polymerfilms die Kosten zum Erhalt einer Analyt-sensitiven Schicht, beispielsweise durch verminderten Syntheseaufwand, drastisch reduziert. Weitere Vorteile der Sensormaterialien **SM1** - **SM4** gegenüber den AFPs ergeben sich daraus, dass durch einfache Modifizierung der Molekülstruktur der Nachweisreagenzien, durch Herstellung von Copolymeren oder adsoptiv an das Polymer gebundenen Reagenzien bzw. Katalysatoren und durch unterschiedliche Schichtdicken (1 - 2000 nm) die Eigenschaften der Sensormaterialien an die Analyteigenschaften angepasst werden können, um dessen Effizienz zu erhöhen.

Wie ersichtlich, beruht das auf die vergleichsweise hydrophoben Fluoreszenzindikatoren **4, 5** und **6** gestützte Nachweisverfahren auf einer als Elektronendonor wirkenden Rezeptor-Einheit der Nachweisreagenzien für den betreffenden Explosivstoff in Form eines Triphenylamin-Motivs, welches, die im Farbstoffmolekül gebildeten (radikalischen) Kationen stabilisieren kann und dadurch die Fluoreszenzlöschung begünstigt.

Für das Aufbringen der Sonden auf die homogene Oberfläche des entsprechenden Polymermaterials können verschiedene Verfahren genutzt werden. Beispielsweise können die entsprechenden Mengen der gelösten Substanzen in einem geeigneten Lösungsmittelgemisch mit einem Spin-Coater, Spray-Coater, piezoelektrischen Dosiersystem, einem Nanoplotter oder mit einem angepassten Tintenstrahl-Drucker auf den Träger aufgetragen werden. Ebenso liefern handelsübliche Einzeltropfen-Dosiersysteme reproduzierbare Ergebnisse. Analog können die Farbstoffe auch durch ein geeignetes Stempel-Techniken oder Kontakt-Druckverfahren aufgetragen werden.

Vorteilhafterweise geht das Trägermaterial im Kontakt mit der Luft, die den nachzuweisenden Explosivstoff enthält, keine nachteilige Wechselwirkung mit dem Explosivstoff ein, ist also inert.

Das inerte Trägermaterial ist bevorzugt farbloses, transparentes Polymer auf einem festen Substrat, beispielsweise farblosem Glas, sodass Anregungslicht (360 - 370 nm) nicht absorbiert wird. Die Form des einsatzbereiten Trägermaterials ist frei wählbar, bevorzugt ist seine Form und Größe an eine Haltevorrichtung und diese an den verwendeten Reader angepasst. Vorteilhafterweise ist die Größe so gewählt, dass die mit den NOx-Verbindungen kontaminierte Luftströmung die ganze Sensorschicht erfasst.

Gemäß praktischen Ausführungsformen werden typischerweise in der Mikroskopie verwendete Deckgläser als inertes Trägermaterial verwendet. Beispielsweise können handelsübliche runde Deckgläser mit einem Durchmesser von 3 - 20 mm als inertes Substrat verwendet werden. Bevorzugt ist die Oberfläche des Substrates plan. Jedoch kann das Substrat zumindest abschnittsweise eine gekrümmte Oberfläche aufweisen und einen Hohlraum umschließen, der zumindest eine Eingangsöffnung für eine Zufuhr des Analyten und zumindest eine Ausgangsöffnung für die Abfuhr des Analyten aufweist. Vorteilhaft wird ein Polymerfilm auf der inneren Oberfläche, bzw. einem Hohlraumabschnitt ausgebildet. Es können ebenso abschnittsweise unterschiedliche Polymerschichten benachbart zueinander angeordnet werden, sodass das Substrat in mehrere Zonen geteilt ist. Gemäß einer weiteren Ausführungsform kann eine ansonsten homogene Polymerschicht auf dem Substrat (plan oder Hohlrauminnenfläche) in mehrere Zonen geteilt sein, indem unterschiedliche Nachweisreagenzien benachbart zueinander auf dem Träger aufgebracht werden. So werden auf einem einstückigen Substrat sensitive Schichten mit unterschiedlichen Eigenschaften ausgebildet. Deren Anordnung kann vorteilhaft so gewählt sein, dass die Strömung des zu analysierenden Mediums (Analyten, bzw. Luft, die nur möglicherweise den Analyten enthält...) durch geometrische Anordnung in einer bestimmten Reihenfolge und / oder mit einer bestimmter Strömungsgeschwindigkeit und / oder bei einem bestimmten Druck über bzw. durch diese Zonen erfolgt. Vorteilhaft kann so die Verweilzeit des Analyten in weiten Grenzen variiert werden, um einen sicheren Nachweis zu gewährleisten.

Die Schichtdicke des Trägermaterials, d.h., die Schichtdicke der auf dem inerten Substrat angeordneten Polymerschicht, kann zum Steuern einer Mess-Empfindlichkeit des Nachweisverfahrens dienen. Durch den Einsatz von Quervernetzern kann die Schichtdicke von 1 bis 5 nm (Fig. 30) auf > 1 bis 2 µm gesteigert werden.

Gemäß einer beispielhaften Ausführungsform kann eine Analyt-sensitive Schicht einzelne kovalent am Substrat gebundene Polymerketten in Form eines Polymerteppichs (brushes) umfassen, wobei die kovalent gebundenen Polymerketten im Durchschnitt von 18 bis 26, typischerweise von 20 bis 24, bevorzugt 22 ± 1 Benzylmethacrylat-Einheiten umfassen.

Um die Selektivität der Sensormaterialien **SM1-SM4** für TNT, DNT, Tetryl, PETN, NG, EGDN, RDX, HMX, NH₄NO₃ und DMDNB zu untersuchen, wurden mit einem mobilen Messgerät (hier als "Handgerät" bezeichnet) Messungen der Lösungen der Explosivstoffe und von einigen strukturverwandten Moschus-Verbindungen durchgeführt. Insbesondere zeigen **SM1** und **SM2** mit TNT sowie **SM3** mit DMDNB eine Fluoreszenzlöschung (Fig. 26 und 28). Bei der Untersuchung der Querempfindlichkeit für Moschus Ambrette, der nicht zu den Explosiv- bzw. Markerstoffen zählt, jedoch mit TNT vergleichbare Wechselwirkungen mit den Analyt-sensitiven Schichten zeigt, wurde die Nachweisgrenze für Explosivstoffe nicht überschritten.

Es ist bekannt, dass molekulare Sonden allein nicht zwischen den gesuchten Explosivstoffen und Stoffen, die ebenfalls fluoreszenzlöschende Eigenschaften aufweisen, unterscheiden können. Jedoch ist die Wahrscheinlichkeit, derartige Stoffe in der Umwelt zu finden, typischerweise sehr gering. Ausnahmen stellen die zahlreichen Moschus-Verbindungen dar, die als Bestandteile verschiedene Parfüms, Kosmetikprodukte und Pflanzenschutzmittel, im Grundwasser auftreten können. Die Sonde kann daher abhängig vom Polymermaterial, Polymerschichtdicke, Farbstoffschichtdicke, Messmodus (Wischprobe, Wasserprobe oder Luft), Signalmuster der Fluoreszenzlöschung (oder Fluoreszenzverstärkung) und der Signatur der Regenerierungsphase effektiv für den selektiven Nachweis von Explosivstoffen auf NOₓ-Basis eingesetzt werden. Weiterhin besteht selbstredend die Möglichkeit, Messungen einer Probe, mit mindestens zwei Sonden durchzuführen, um eine genauere Aussage über die Zusammensetzung der Probe treffen zu können.

Fig. 38 zeigt das Signal des Sensormaterials **SM1** im Handgerät vor und nach der Messung von jeweils 3uL wässriger Proben ohne und mit TNT (1,18 mg/L, c = 5,2 µM), die mit dem auf 150 °C geheizten Messkopf des Handgeräts verdampft werden. Bei der Messung von reinem Wasser wird eine Fluoreszenzverstärkung beobachtet, die wieder in den Ausgangszustand zurückkehrt. Im Falle von TNT kontaminierten Proben wird zunächst ebenfalls eine Fluoreszenzverstärkung beobachtet, wobei jedoch bei der Erholung des Signals dieser nach dem Erreichen des Ausgangszustandes weiter bis zur 5 %-TNT-Nachweisgrenze fällt. Um die Kontaminierung des Handgeräts durch Wasserproben zu vermeiden können die Messungen auch über der wässrigen TNT-Probe (1,18 mg/L, c = 5,2 µM) durchgeführt werden. Im Vergleich zu den Wasserproben wird eine Fluoreszenzverstärkung, verursacht durch hohe Wassermengen auf dem Sensormaterial, nicht beobachtet. Die von TNT verursachte Fluoreszenzlöschung erreicht dabei die 5 %-TNT-Nachweisgrenze in 32 Sekunden und die 10 %-TNT-Nachweisgrenze in 58 Sekunden. (Fig. 39).

Ausgehend von den vorliegenden Ergebnissen liegen die hier beschriebenen fluoreszierenden Moleküle am Polymer adsorbiert vor. Im Ergebnis des erreichten Zustandes kann eine NOx-Verbindung von der Triphenylamino-Gruppe der auf der Polymeroberfläche befindlichen Sonde ein Elektron reversibel abstrahieren und dessen Fluoreszenz löschen. Die Empfindlichkeit der Detektion hängt dabei von der Schichtdicke der molekularen Sonde im Polymerfilm und von der Aufnahmefähigkeit des Polymers des jeweiligen Explosivstoffes ab. Abhängig von dessen Flüchtigkeit kann der Explosivstoff wieder desorbiert werden, da die Fluoreszenzantwort reversibel ist. Daraus ergibt sich, dass das Polymer den Explosivstoff adsorbiert bzw. dessen Verweildauer in der Nähe der Sonde erhöht und die Abstraktion eines Elektrons von der Rezeptoreinheit Triphenylamin ermöglicht.

Gemäß den insgesamt hier vorgeschlagenen Ausführungsformen wurde ein Sondenkonzept entwickelt, wobei eine reversible physikochemische Wechselwirkung einer NOx-Verbindung an ein Nachweisreagenz, bevorzugt umfassend ein Triphenylamin, zu einem spektroskopisch messbaren Signalverlauf einer Fluoreszenz des Nachweisreagenz führt. Weiterhin wird gemäß den beispielhaft vorgeschlagenen Ausführungsformen die Einbettung der Fluoreszenzsonden in eine Polymer-Matrix, beispielsweise in eine Polymer-Matrix umfassend Poly(benzylmethacrylat), Poly(benzylacrylat), Polystyrol oder Poly(ethylenglykoldimethacrylat) / Poly(2-hydroxyethylmethacrylat) zur Detektion von schwerflüchtigen Explosivstoffen wie TNT und Poly(1,4-bisacryloylpiperazin) und dessen Derivate, Poly(1,4-bisacryloylpiperazin) / Poly(2-hydroxyethylmethacrylat) oder Poly(ethylenglykoldimethacrylat) / Poly(2-hydroxyethylmethacrylat) zur Detektion des Markerstoffes DMDNB vorgeschlagen. Diese Einbettung erlaubt es, den fluoreszenzspektroskopischen Nachweis von TNT in gelöstem, festem oder gasförmigem Zustand sogar in gemischten Phasen gleichzeitig zu führen. Durch den Einsatz von Poly(1,4-bisacryloylpiperazin) können auch sehr geringe Mengen des Markers DMDNB aus der Luft in den Poren der Polymer-Matrix angereichert und gleichzeitig mit der molekularen Sonde detektiert werden. Zur Regenerierung der fluoreszenzoptischen Eigenschaften der Analyt-sensitiven Schicht wird am 150°C heißen Messkopf eine definierte Menge Wasser (1 µL bis 2 µL) Wasser verdampft, welches dann den Analyten (Explosivstoff, Marker) aus den Polymerporen verdrängt. Nach dem Abdampfen des Wassers aus den Polymerporen nimmt das vom Messgerät gemessene Emissionssignal schnell zu und erreicht erneut den Ausgangswert. Weiterhin wird eine interne Referenzierung vorgeschlagen, welche es erlaubt, die Zuverlässigkeit des Assays zu steigern.

Zur Nachweisführung der Explosivstoffe in der Luft bzw. als Wischprobe wird die Analyt-sensitive Schicht im Messgerät mit einem geheizten Luftstrom beaufschlagt, wobei der (beheizte) Lufteinlass an die Probe bzw. an eine Wischprobe gehalten wird. Dazu kann beispielsweise ein geeigneter Messkopf, umfassend den Lufteinlass auf eine Temperatur >150°C geheizt werden. Bei Erreichen der Nachweisgrenze in einer bestimmten Zeit unter bekannten Umwelteinflüssen (Luftfeuchtigkeit und Temperatur) wird das Vorhandensein einer NOₓ-Verbindung als Fluoreszenzlöschung der Analyt-sensitiven Schicht angezeigt. Durch den kombinierten Einsatz verschieden spezifischer Analyt-sensitiver Schichten (z.B. SM3 für den Markerstoff DMDNB) kann die Zusammensetzung des entsprechenden Explosivstoffes eingegrenzt werden. Über die Messung der Regenerierungsdauer des Messsignals können zusätzliche Informationen über die Flüchtigkeit des jeweiligen Analyten gewonnen werden. Danach kann die Analyt-sensitive Schicht entweder bei höherer Temperatur und ggf. zusätzlich mit Wasserdampf regeneriert werden. Die Verwendung von Wasser verkürzt die Wartezeit im Falle von **SM3** (zuvor kontaminiert mit dem Marker DMDNB) bis zur erneuten Messung deutlich (vgl. Fig. 28). Der mittels Messkopf auf die Analyt-sensitive Schicht geleitete Wasserdampf verdrängt Analyte aus der Polymerschicht. Durch vollständiges Trocknen der Analyt-sensitiven Schicht erfolgt deren Regeneration, was an einer vollständigen Wiederherstellung der charakteristischen Fluoreszenz des jeweiligen Nachweisreagenz ersichtlich ist.

Die Einsatzfähigkeit von vorbekannten AFPs als molekulare Sonden für NOₓ-Explosivstoffe ist unter praxisrelevanten Messbedingungen bis auf wenige Ausnahmen extrem eingeschränkt. Ein Grund dafür sind falsch-positive Befunde bei Luftfeuchtigkeitsänderungen und eine dadurch verursachte hohe Fehlalarmrate. Das wird vor allem dem Selbstlöschungs-Effekt der hydrophoben AFPs durch deren Zusammenrücken in Gegenwart von Wasser zugeschrieben. Daher ist nur eine geringe Anzahl von Sensoren bekannt, die die Explosivstoffe in der Luft, als Wischproben, aus Wasser- und organischen Lösungsmittel-Proben gleichzeitig detektieren können. Weitere, auf Stickoxid reagierende Sensoren beruhen auf der thermischen Zersetzung der Explosivstoffe als Wischproben und NOₓ-Verbindungen. Neben dem irreversiblen Charakter dieser Reaktion ist jedoch die geringe Selektivität nachteilig und schränkt die Anwendbarkeit dieses Nachweisverfahren unter vor-Ort-Bedingungen erheblich ein.

Deshalb ist der Bedarf an einem einfachen und kostengünstigen Nachweisverfahren für NOₓ-Explosivstoffe unabhängig von jeweiligen Umweltbedingungen immer noch sehr hoch.

Indikatorsubstanzen - wie die hier vorgeschlagenen - haben gegenüber AFPs den Vorteil, dass sie prinzipiell sehr selektiv sind und Querempfindlichkeiten minimiert werden können. Der Nachteil ist i.d.R. eine unzureichende Breitband-Detektion.

Gemäß den vorstehend beschriebenen Prinzipien wird die Querempfindlichkeit von Analyt-sensitiven Schichten für Explosivstoffe und Markerstoffe auf NOx-Basis reduziert. Weiterhin wird an Hand einer fluoreszenzoptischen Messung mehr Information über die Zusammensetzung einer Probe, umfassend zumindest einen Explosivstoff und optional zumindest einen Markerstoff erzielt.

Die Selektivität und die Sensitivität gegenüber dem Explosivstoff sowie die Stabilität der jeweiligen Analyt-sensitiven Schicht sind unter den Messbedingungen dabei über die Molekülstruktur, die Schichtdicke und die Dicke eines Polymerfilm-Trägermaterials der vorgeschlagenen Nachweisreagenzien einstellbar. Sie sind weiter steuerbar über die chemische Natur des jeweiligen Polymermaterials und die genaue Zusammensetzung und molekulare Schichtstruktur des Analyt-sensitiven Schichtaufbaus auf dem Substrat.

Vorteile der vorgeschlagenen Ausführungsformen, insbesondere der Sensormaterialien **SM1-SM4,** sind die kostengünstige und einfache Synthese der jeweiligen molekularen Sonden. Die Herstellung der Polymerfilme ist durch die beschriebenen Methoden in großen Stückzahlen problemlos reproduzierbar (d.h., standardisierbar) und kostengünstig möglich. Das Nachweisverfahren selbst ist empfindlich und liefert zuverlässig quantitative Angaben zur Konzentration der jeweiligen NOₓ-Verbindung in der untersuchten Probe (Luft, Wasser, organische Lösungsmittel, Feststoffe). Es ermöglicht, schnell Ergebnisse zu erhalten und ist damit zur Vor-Ort-Analyse für Belange der Sicherheit und des Umweltschutzes einsetzbar.

Wie vorstehend beschrieben, sind mit den Nachweisreagenzien bzw. molekularen Sonden **4, 5** und **6** abhängig vom Polymermaterial als Träger dieser Sonden Explosivstoffe und Marker auf NOₓ-Basis zuverlässig nachweisbar. Erst die hier beschriebene Immobilisierung der Sonde auf dem entsprechenden Polymerfilm sichert eine Photostabilität und Luftstabilität der Sensorschicht in einem Temperaturbereich von 0-130°C. Die hohe Stabilität der Sensorschichten erlaubt es, auch Proben aus organischen Lösungsmitteln und Wasser zu messen. Die Bereitstellung einer hochempfindlichen Fluoreszenzsonde für die Bestimmung von TNT im Grundwasser ist von unmittelbar praktischer Bedeutung, beispielsweise für den Gesundheits- und Verbraucherschutz.

Die beschriebenen, auf Triarylamin basierenden Fluoreszenzindikatoren (Nachweisreagenzien) sind mit ihrer hohen Quantenausbeute, breitbandigen Anregungsmöglichkeit, hohen Photo-, Luft- und Langzeitstabilität und einer ausgeprägten Unempfindlichkeit gegenüber Umwelteinflüssen (wie Änderungen der Luftfeuchtigkeit, der Anwesenheit von organischen und/oder wässrigen Lösungsmitteldämpfen und Sauerstoff) auf den entsprechenden Trägermaterialien, umfassend nicht fluoreszierende, apolare Polymerfilme zur Detektion von Explosivstoffen basierend auf NOx-Einheiten, zur Detektion von thermischen Zersetzungsprodukten von Explosivstoffen wie Stickoxiden, Ausgangsmaterialien zur Herstellung von Explosivstoffen wie Salpetersäure und zur Detektion von Markerstoffen wie DMDNB und DNT geeignet.

Gemäß vorstehend beschriebenen beispielhaften Ausführungsformen und unter Berücksichtigung der hier wiedergegebenen Erkenntnisse wird vorgeschlagen, die Sonden **4** und **5** auf Poly(benzylmethacrylat)-, Poly(benzylacrylat)- und Polystyrol-Polymerfilmen zur qualitativen und quantitativen Detektion der schwerflüchtigen Explosivstoffe TNT, Tetryl, RDX, HMX, PETN, Ammoniumnitrat und des Markerstoffes 2,4-Dinitrotoluol zu nutzen. Weiterhin wird vorgeschlagen, die Sonden **5** und **6** auf Polymerfilmen, umfassend Poly(1,4-bisacryloylpiperazin) und dessen Derivative und Mischfilme, umfassend Poly(1,4-bisacryloylpiperazin)/Poly(2-hydroxyethylmethacrylat) zur qualitativen und quantitativen Detektion des Markerstoffes DMDNB zu nutzen. Weiterhin wird vorgeschlagen, die Sonde **5** auf Mischfilme, umfassend Poly(ethylenglykoldimethacrylat)/Poly(2-hydroxyethylmethacrylat) zur qualitativen und quantitativen Detektion von TNT und von DMDNB zu nutzen.

Das Triphenylamin-Motiv der Nachweisreagenzien **4, 5** und **6** wird für den Nachweis von Nitroverbindungen eingesetzt, wobei die Stabilität des Farbstoffes an der Polymer-Luftgrenze von der Zusammensetzung des Polymers abhängig ist. Das Polymer steuert zusätzlich auch die Verweildauer des jeweiligen Explosivstoffes auf der Polymeroberfläche und damit die Wechselwirkung zwischen molekularer Sonde und Analyt (Explosivstoff). Nach Kalibrierung dient die Löschung des Fluoreszenzsignals der Analyt-sensitiven Schicht unter dem Einfluss des an der Rezeptor-Einheit gebundenen Explosivstoffes zur quantitativen Bestimmung dieser in der Luft, in wässriger und organischer Lösung und auf Wischproben. Ebenso kann die, beispielsweise bei Einwirkung von Wasserdampf erfolgende, Regeneration des Fluoreszenzsignals der Analyt-sensitiven Schicht zur Identifikation eines zuvor adsorbierten fluoreszenzlöschenden Analyten allein verwendet oder ergänzend hinzugezogen werden, wenn ein unbekannter NOx-haltiger Analyt bestimmt werden soll.

Insbesondere werden erfindungsgemäß die nachfolgenden Ausführungsformen vorgeschlagen.
1. Nachweisreagenz für einen eine NOₓ-Gruppe umfassenden Analyten, wobei das Nachweisreagenz ein Arylamin umfasst, und eine Strukturformel des Arylamins ausgewählt ist unter der Strukturformel 1: 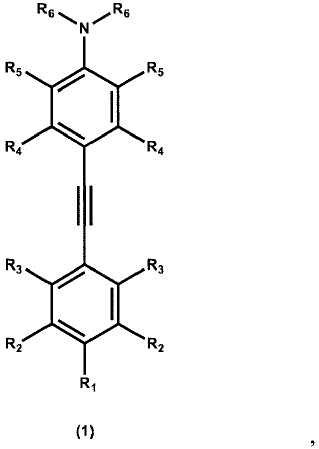 wobei
   R₁ ausgewählt ist unter CO₂X oder PhCO₂X mit X = Vinyl, Allyl, Homoallyl oder Aryl oder
   R₁ für C(O)NX₂ oder PhC(O)NX₂ steht, mit X = H, Alkyl, Perfluoralkyl, Vinyl, Allyl, Homoallyl oder Aryl;
   R₂, R₃, R₄, und/oder R₅ unabhängig voneinander ausgewählt sind unter H, F, einem Alkyl oder einem Aryl; und
   R₆ ausgewählt ist unter einem Aryl.
2. Nachweisreagenz nach Ausführungsform 1, wobei das Arylamin die Strukturformel 1 aufweist und R₁ für ein *N,N*-Dialkylamid oder für ein Phenyl *-N,N-*Dialkylamid steht.
3. Nachweisreagenz nach Ausführungsform 1 oder 2, wobei R₂, R₃, R₄ und R₅ für H stehen.
4. Nachweisreagenz nach einer der vorstehenden Ausführungsformen, wobei R₆ für eine Phenylgruppe steht und das Arylamin somit ein Triphenylamin-Motiv umfasst.
5. Nachweisreagenz nach Ausführungsform 4, wobei das Triphenylamin-Motiv in mindestens einer *para*-Position kovalent mit einer Phenylgruppe verknüpft ist, und verbleibende *para*-Positionen unsubstituiert oder methyliert vorliegen.
6. Nachweisreagenz nach Ausführungsform 5, wobei das Triphenylamin-Motiv und die Phenylgruppe über eine Dreifachbindung, über eine Doppelbindung oder über eine Einfachbindung verknüpft sind.
7. Nachweisreagenz nach Ausführungsform 4, wobei die Strukturformel des Arylamins ausgewählt ist unter den Strukturformeln **4, 5** und **6**: 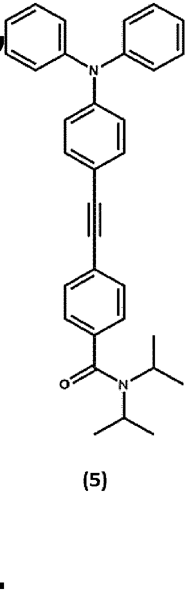 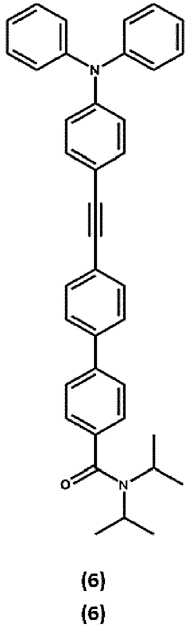 wobei die das Triphenylamin-Motiv als Donor ein Elektron an die NOₓ-Gruppe des Analyten abgibt oder als Rezeptor ein Elektron von der NOx-Gruppe des Analyten aufnimmt, bei einem Abgeben des Elektrons an die NOx-Gruppe des Analyten eine Fluoreszenzlöschung des Nachweisreagenz messbar ist, und/oder bei einem Aufnehmen des Elektrons eine Regeneration einer Fluoreszenz messbar ist, sodass der Analyt qualitativ und/oder quantitativ optisch bestimmbar ist.
8. Nachweisreagenz nach Ausführungsform 7, wobei der die NOₓ-Gruppe umfassende Analyt ausgewählt ist unter: TNT, DNT, Tetryl, PETN, NG, EGDN, DNDMB, Ammoniumnitrat, RDX und HMX.
9. Nachweisreagenz nach einer der vorstehenden Ausführungsformen, wobei der die NOx-Gruppe umfassende Analyt vorliegt in einer Probe, umfassend eine organische Lösung, eine wässrige Lösung, eine gemischt organisch-wässrigen Lösung, eine Luftprobe und/oder eine Wischprobe.
10. Verfahren zum Nachweis eines eine NOx-Gruppe aufweisenden Analyten, umfassend:
   - Bereitstellen einer Analyt-sensitiven Schicht, umfassend einen Polymerfilm auf einem Trägermaterial und ein adsorptiv am Polymerfilm gebundenes Nachweisreagenz gemäß einer der Ausführungsformen 1 bis 9;
   - Wechselwirken des die NOx-Gruppe umfassenden Analyten mit der Analytsensitiven Schicht;
   - Messen einer Fluoreszenzeigenschaft zumindest eines Abschnitts der Analytsensitiven Schicht.
   - Erwärmen und/oder Verdampfen einer definierten Probenmenge, die potentiell den die NOₓ-Gruppe umfassenden Analyten enthält;
   - Leiten eines Gases oder Gasgemisches, umfassend die erwärmte oder verdampfte definierte Probenmenge auf die Analyt-sensitive Schicht, sodass der die NOₓ-Gruppe umfassende Analyt auf, in und/oder an der Analytsensitiven Schicht angereichert wird;
   - Ermitteln einer Zusammensetzung und/oder einer Konzentration des die NOₓ-Gruppe umfassenden Analyten unter Verwendung von gespeicherten Messdaten einer Vergleichsmessung.
   - Regenerieren der Analyt-sensitiven Schicht durch Kontakt mit einem NOxfreien Fluid, durch Ausheizen und/oder durch Anströmen mit Wasserdampf.
13. Verfahren nach der Ausführungsform 10, wobei die Fluoreszenzeigenschaft ausgewählt ist unter:
   einer Fluoreszenzquantenausbeute, einer Fluoreszenzlebensdauer; einer Fluoreszenzintensität, insbesondere unter einer Fluoreszenzlöschung oder einer Fluoreszenzzunahme nach einer vorausgehenden Fluoreszenzlöschung.
14. Verfahren nach einer der Ausführungsformen 10 bis 13, wobei das Messen der Fluoreszenzeigenschaft ein direktes Erfassen eines elektrischen Signals zumindest eines Detektors oder ein Bilden eines Quotienten aus elektrischen Signalen umfasst, die bei unterschiedlichen Anregungswellenlängen von zumindest einem Detektor erfassten werden.
15. Verfahren nach einer der Ausführungsformen 10 bis 14, wobei das Messen der Fluoreszenzeigenschaft mit einem portablen, bevorzugt einem einhändig tragbaren, Messgerät erfolgt, und das Messgerät eine Scan-Vorrichtung umfasst, die eingerichtet ist zum Messen der Fluoreszenzeigenschaft bei zumindest einer fest eingestellten Wellenlänge.
16. Verfahren nach einer der Ausführungsformen 10 bis 15, wobei der Polymerfilm
   - eine Dicke von 1 nm bis 5 nm und/oder
   - eine Flächenkonzentration des Nachweisreagenz von 30 - 75 µmol / cm² Substrat aufweist.
17. Verfahren nach einer der Ausführungsformen 10 bis 16, wobei der Analyt ein Explosivstoff ist.
18. Verfahren nach einer der Ausführungsformen 10 bis 17, wobei der Polymerfilm ein Polymer umfasst, das ausgewählt ist unter: Poly(benzylmethacrylat); Poly(benzylacrylat); Polystyrol; einem Poly(arylacrylat); einem Polyacrylamid; einem Polymer umfassend alkylsubstituierte Arylacrylate; Poly(benzylmethacrylat) / Polystyrol; Poly(1,4-Bisacryloylpiperazin) und Derivative von Poly(l,4-Bisacryloylpiperazin); Poly(1,4-Bisacryloylpiperazin) / Poly(2-hydroxyethylmethacrylat); und/oder Poly(ethylenglykoldimethacrylat) / Poly(2-hydroxyethylmethacrylat).
19. Verfahren nach einer der Ausführungsformen 10 bis 18, weiterhin umfassend:
   - Komplexieren des Nachweisreagenz durch Polymerketten des Polymerfilms an einer Polymer-Luft-Grenze und/oder Anreichern des die NOₓ-Gruppe umfassenden Analyten durch den Polymerfilm.
20. Herstellungsverfahren für eine Analyt-sensitive Schicht, umfassend:
   - Bereitstellen eines Substrates;
   - Aufbauen eines Polymerfilms auf dem Substrat;
   - Auftragen auf den aufgebauten Polymerfilm eines Nachweisreagenz gemäß einer der Ausführungsformen 1 bis 9,
   wobei der Analyt eine NOₓ-Gruppe umfasst.
21. Herstellungsverfahren nach Ausführungsform 20, wobei das Substrat an seiner Oberfläche zumindest eine Art funktioneller Gruppen aufweist, die nach einer chemischen Aktivierung zu einer Verankerung des Polymerfilms auf dem Substrat verwendbar sind.
22. Herstellungsverfahren nach Ausführungsform 20 oder 21, wobei das bereitgestellte Substrat eine ebene Fläche aufweist, beispielsweise eine Scheibe ist, und das Aufbauen des Polymerfilms zumindest abschnittsweise einseitig und/oder abschnittsweise beidseitig erfolgt.
23. Herstellungsverfahren nach Ausführungsform 20 oder 21, wobei das Substrat zumindest abschnittsweise eine gekrümmte Oberfläche aufweist und einen Hohlraum umschließt, der zumindest eine Eingangsöffnung für eine Zufuhr des Analyten und zumindest eine Ausgangsöffnung für die Abfuhr des Analyten aufweist, wobei das Aufbauen des Polymerfilms auf einer im Hohlraum liegenden Substratoberfläche erfolgt.
24. Herstellungsverfahren nach einer der Ausführungsformen 20 bis 23, wobei das Auftragen durch zumindest teilweises Benetzen und/oder Besprühen erfolgt, sodass das Nachweisreagenz am Polymerfilm adsorbiert wird.
25. Herstellungsverfahren nach Ausführungsform 24, wobei das zumindest teilweise Benetzen und/ oder Besprühen mit einem Spin-Coater, einem Spray-Coater, einem piezoelektrischen Dosiersystem, einem Drucker, einem Nanoplotter, einem Tintenstrahl-Drucker, oder einem Stempel erfolgt.
26. Herstellungsverfahren zumindest nach einer der Ausführungsformen 20 bis 25, wobei das Substrat ausgewählt ist unter: einem Polymer, einem Metall, einem Flachglas oder ein Glasrohr, einer Keramik oder zumindest eines der benannten Materialien umfasst.
27. Herstellungsverfahren zumindest nach einer der Ausführungsformen 20 bis 26, wobei das Aufbauen des Polymerfilms auf dem Substrat umfasst:
   - Aktivieren des Substrats;
   - Auftragen einer Lösung des Polymers in einem Lösungsmittel;
   - kovalentes Verknüpfen des gelösten Polymers mit dem aktivierten Substrat;
   - Abtrennen des Lösungsmittels.
28. Analyt-sensitive Schicht für einen eine NOx-Gruppe umfassenden Analyten, umfassend ein Substrat, eine auf dem Substrat angeordnete Polymerschicht und zumindest ein an der Polymerschicht adsorbiertes Nachweisreagenz gemäß einer der Ausführungsformen 1 bis 9, wobei eine Fluoreszenzintensität des Nachweisreagenz in Gegenwart des Analyten gegenüber einer Fluoreszenzintensität des Nachweisreagenz in Abwesenheit des Analyten vermindert ist, wobei der auf dem Substrat aufgebaute Polymerfilm mehrere Zonen umfassend unterschiedliche Nachweisreagenzien aufweist, sodass die Analyt-sensitive Schicht Abschnitte unterschiedlicher Sensitivität für unterschiedliche, eine NOx-Gruppe umfassende, Analyten aufweist.
30. Analyt-sensitive Schicht nach einer der Ausführungsform 28 wobei
   - der Polymerfilm zumindest eine Schicht eines hydrophoben Polymers, umfassend einzelne kovalent gebundene Polymerketten umfasst, wobei die kovalent gebundenen Polymerketten im Durchschnitt Benzylmethacrylat-Einheiten einer Anzahl ausgewählt unter: 18 bis 26, 20 bis 24 und 22 ± 1 umfassen,
   - der die NOx-Gruppe umfassende Analyt ausgewählt ist unter TNT, DNT, Tetryl, PETN, NG, EGDN, NH₄NO₃, RDX und HMX.
31. Verwendung eines Nachweisreagenz gemäß einer der Ausführungsformene 1 bis 9 und/oder eines Verfahrens gemäß einer der Ausführungsformen 10 bis 19 und/oder einer Analyt-sensitiven Schicht gemäß den Ausführungsformen 28 bis 30 zur Überwachung eines Grenzwertes eines Explosivstoffes.

### Referenzen:

[1] Xu F., Peng L., Orita A., Otera J. (2012) Dihalo-Substituted Dibenzopentalenes: Their Practical Synthesis and Transformation to Dibenzopentalene Derivatives. Org. Lett. 14, 3970-3973;
[2] Schröder N., Wencel-Delord J., Glorius F. (2012) High-Yielding, Versatile, and Practical [Rh(III)Cp*]-Catalyzed Ortho Bromination and Iodination of Arenes. J. Am. Chem. Soc. 134, 8298-8301;
[3] Yang Y-S., Swager T. M. (1998) Fluorescent Porous Polymer Films as TNT Chemosensors: Electronic and Structural Effects. J. Am. Chem. Soc. 120, 11864-11873;
[4] Yang Y-S., Swager T. M. (1998) Porous Shape Persistent Fluorescent Polymer Films: An Approach to TNT Sensory Materials, J. Am. Chem. Soc. 120, 5321-5322;
[5] Sanchez J.C., Trogler W.C. J. (2008) Efficient Blue-emitting Silafluorene-fluoreneconjugated Copolymers: Selective Turn-off/Turn-on Detection of Explosives. Mater. Chem., 18, 3143-3156;
[6] Che Y., Gross D.E., Huang H., Yang D., Yang X., Discekici E., Xue Z., Zhao H., Moore J. S., Zang L., (2012) Diffusion-Controlled Detection of Trinitrotoluene: Interior Nanoporous Structure and Low Highest Occupied Molecular Orbital Level of Building Blocks Enhance Selectivity and Sensitivity. J. Am. Chem. Soc. 134, 4978-4982;
[7] Thomas III, S.W.; Amara J.P., Bjork R.E., Swager T.M. (2005) Amplifying Fluorescent Polymer Sensors for the Explosives Taggant 2,3-Dimethyl-2,3-dinitrobutane (DMNB). Chem. Commun., 4572-4574;
[8] Mardelli M., Olmsted J. (1977) Calorimetric Determination of the 9,10-Diphenyl-Anthracene Fluorescence Quantum Yield. Journal of Photochemistry, 7, 277-285;

## Patentansprüche

1. Nachweisreagenz für einen eine NOₓ-Gruppe umfassenden Analyten, wobei das Nachweisreagenz ein Arylamin umfasst, und eine Strukturformel des Arylamins ausgewählt ist unter der Strukturformel 1: 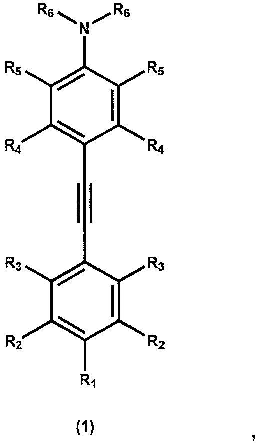 wobei,
R₁ ausgewählt ist unter CO₂X oder PhCO₂X mit X = Vinyl, Allyl, Homoallyl oder Aryl oder
R₁ für C(O)NX₂ oder PhC(O)NX₂ steht, mit X = H, Alkyl, Perfluoralkyl, Vinyl, Allyl, Homoallyl oder Aryl;
R₂, R₃, R₄, und/oder R₅ unabhängig voneinander ausgewählt sind unter H, F, einem Alkyl oder einem Aryl; und
R₆ ausgewählt ist unter einem Aryl.

2. Nachweisreagenz nach Anspruch 1, wobei das Arylamin die Strukturformel 1 aufweist und R₁ für ein *N,N*-Dialkylamid, oder für ein Phenyl-*N,N*-Dialkylamid steht.

3. Nachweisreagenz nach einem der vorstehenden Ansprüche, wobei R₆ für eine Phenylgruppe steht und das Arylamin somit ein Triphenylamin-Motiv umfasst.

4. Nachweisreagenz nach Anspruch 3, wobei das Triphenylamin-Motiv in mindestens einer *para*-Position kovalent mit einer Phenylgruppe verknüpft ist, und verbleibende *para*-Positionen unsubstituiert oder methyliert vorliegen.

5. Nachweisreagenz nach Anspruch 3, wobei die Strukturformel des Arylamins ausgewählt ist unter den Strukturformeln **5** und **6:**

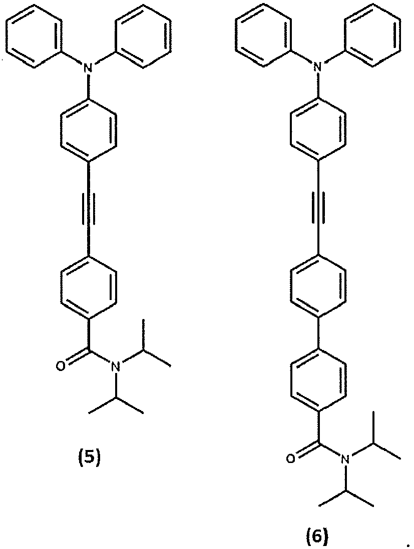

6. Nachweisreagenz nach Anspruch 5, wobei der die NOₓ-Gruppe umfassende Analyt ausgewählt ist unter: TNT, DNT, Tetryl, PETN, NG, EGDN, DNDMB, Ammoniumnitrat, RDX und HMX.

7. Verfahren zum Nachweis eines eine NOₓ-Gruppe aufweisenden Analyten, umfassend:
- Bereitstellen einer Analyt-sensitiven Schicht, umfassend einen Polymerfilm auf einem Trägermaterial und ein adsorptiv am Polymerfilm gebundenes Nachweisreagenz gemäß einem der Ansprüche 1 bis 6;
- Wechselwirken des die NOx-Gruppe umfassenden Analyten mit der Analyt-sensitiven Schicht;
- Messen einer Fluoreszenzeigenschaft zumindest eines Abschnitts der Analyt-sensitiven Schicht;
- Erwärmen und/oder Verdampfen einer definierten Probenmenge, die potentiell den die NOₓ-Gruppe umfassenden Analyten enthält;
- Leiten eines Gases oder Gasgemisches, umfassend die erwärmte oder verdampfte definierte Probenmenge auf die Analyt-sensitive Schicht, sodass der die NOₓ-Gruppe umfassende Analyt auf, in und/oder an der Analyt-sensitiven Schicht angereichert wird;
- Ermitteln einer Zusammensetzung und/oder einer Konzentration des die NOₓ-Gruppe umfassenden Analyten unter Verwendung von gespeicherten Messdaten einer Vergleichsmessung; und optional
- Regenerieren der Analyt-sensitiven Schicht durch Kontakt mit einem NOₓ-freien Fluid, durch Ausheizen und/oder durch Anströmen mit Wasserdampf.

8. Verfahren nach Anspruch 7, wobei die Fluoreszenzeigenschaft ausgewählt ist unter: einer Fluoreszenzquantenausbeute, einer Fluoreszenzlebensdauer; einer Fluoreszenzintensität, insbesondere unter einer Fluoreszenzlöschung oder einer Fluoreszenzzunahme nach einer vorausgehenden Fluoreszenzlöschung.

9. Verfahren nach einem der Ansprüche 7 oder 8, wobei das Messen der Fluoreszenzeigenschaft ein direktes Erfassen eines elektrischen Signals zumindest eines Detektors oder ein Bilden eines Quotienten aus elektrischen Signalen umfasst, die bei unterschiedlichen Anregungswellenlängen von zumindest einem Detektor erfassten werden.

10. Verfahren nach einem der Ansprüche 7 bis 9, wobei das Messen der Fluoreszenzeigenschaft mit einem portablen, bevorzugt einem einhändig tragbaren, Messgerät erfolgt, und das Messgerät eine Scan-Vorrichtung umfasst, die eingerichtet ist zum Messen der Fluoreszenzeigenschaft bei zumindest einer fest eingestellten Wellenlänge.

11. Verfahren nach einem der Ansprüche 7 bis 10, wobei der Analyt ein Explosivstoff ist und der Polymerfilm ein Polymer umfasst, das ausgewählt ist unter:
Poly(benzylmethacrylat); Poly(benzylacrylat); Polystyrol; einem Poly(arylacrylat);
einem Polyacrylamid; einem Polymer umfassend alkylsubstituierte Arylacrylate;
Poly(benzylmethacrylat) / Polystyrol;
Poly(1,4-Bisacryloylpiperazin) und Derivative von Poly(1,4-Bisacryloylpiperazin);
Poly(1,4-Bisacryloylpiperazin) / Poly(2-hydroxyethylmethacrylat); und/oder
Poly(ethylenglykoldimethacrylat) / Poly(2-hydroxyethylmethacrylat).

12. Herstellungsverfahren für eine Analyt-sensitive Schicht, umfassend:
- Bereitstellen eines Substrates;
- Aufbauen eines Polymerfilms auf dem Substrat;
- Auftragen auf den aufgebauten Polymerfilm eines Nachweisreagenz gemäß einem der Ansprüche 1 bis 6,
wobei der Analyt eine NOₓ-Gruppe umfasst.

13. Herstellungsverfahren nach Anspruch 12, wobei das bereitgestellte Substrat eine ebene Fläche aufweist, beispielsweise eine Scheibe ist, und das Aufbauen des Polymerfilms zumindest abschnittsweise einseitig und/oder abschnittsweise beidseitig erfolgt, oder das Substrat zumindest abschnittsweise eine gekrümmte Oberfläche aufweist und einen Hohlraum umschließt, der zumindest eine Eingangsöffnung für eine Zufuhr des Analyten und zumindest eine Ausgangsöffnung für die Abfuhr des Analyten aufweist, wobei das Aufbauen des Polymerfilms auf einer im Hohlraum liegenden Substratoberfläche erfolgt.

14. Analyt-sensitive Schicht für einen eine NOx-Gruppe umfassenden Analyten, umfassend ein Substrat, eine auf dem Substrat angeordnete Polymerschicht und zumindest ein an der Polymerschicht adsorbiertes Nachweisreagenz gemäß einem der Ansprüche 1 bis 6, wobei eine Fluoreszenzintensität des Nachweisreagenz in Gegenwart des Analyten gegenüber einer Fluoreszenzintensität des Nachweisreagenz in Abwesenheit des Analyten vermindert ist, wobei der auf dem Substrat aufgebaute Polymerfilm mehrere Zonen umfassend unterschiedliche Nachweisreagenzien aufweist, sodass die Analyt-sensitive Schicht Abschnitte unterschiedlicher Sensitivität für unterschiedliche, eine NOx-Gruppe umfassende, Analyten aufweist.

15. Verwendung eines Nachweisreagenz gemäß einem der Ansprüche 1 bis 6 und/oder eines Verfahrens gemäß einem der Ansprüche 7 bis 10 und/oder einer Analyt-sensitiven Schicht gemäß Anspruch 14 zur Überwachung eines Grenzwertes eines Explosivstoffes.

## Claims

1. A detection reagent for an analyte comprising an NOₓ group, wherein the detection reagent comprises an arylamine and a structural formula of the arylamine is selected from structural formula **1:** 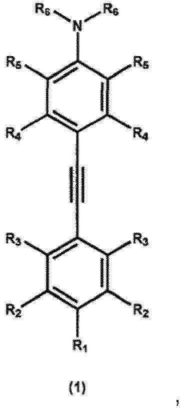 wherein
R₁ is selected from CO₂X or PhCO₂X with X = vinyl, allyl, homoallyl or aryl or R₁ stands for C(O)NX₂ or PhC(O)NX₂, with X = H, alkyl, perfluoroalkyl, vinyl, allyl, homoallyl or aryl;
R₂, R₃, R₄, and/or R₅ are selected independently of one another from H, F, an alkyl or an aryl; and
R₆ is selected from an aryl.

2. The detection reagent according to claim 1, wherein the arylamine has structural formula **1** and R₁ stands for an *N,N*-dialkylamide, or for a phenyl-*N,N*-dialkylamide.

3. The detection reagent according to one of the preceding claims, wherein R₆ stands for a phenyl group and the arylamine thus comprises a triphenylamine motif.

4. The detection reagent according to claim 3, wherein the triphenylamine motif is covalently linked to a phenyl group in at least one *para* position, and remaining para positions are unsubstituted or methylated.

5. The detection reagent according to claim 3, wherein the structural formula of the arylamine is selected from structural formulas **5** and **6:**

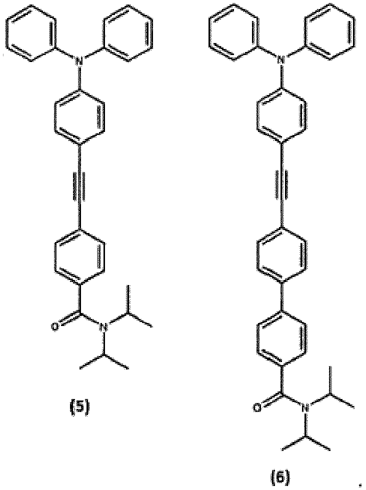

6. The detection reagent according to claim 5, wherein the analyte comprising the NOₓ group is selected from: TNT, DNT, tetryl, PETN, NG, EGDN, DNDMB, ammonium nitrate, RDX and HMX.

7. A method for detecting an analyte comprising an NOₓ group, comprising the steps of:
- providing an analyte-sensitive layer, comprising a polymer film on a carrier material and a detection reagent according to one of claims 1 to 6 adsorptively bound to the polymer film;
- interacting the analyte comprising the NOₓ group with the analyte-sensitive layer;
- measuring a fluorescence property of at least a portion of the analyte-sensitive layer;
- heating and/or vaporising a defined sample volume, which potentially contains the analyte comprising the NOₓ group;
- conducting a gas or gas mixture, comprising the heated or vaporised defined sample volume, to the analyte-sensitive layer, such that the analyte comprising the NOₓ group is enriched in and/or on the analyte-sensitive layer;
- determining the composition and/or concentration of the analyte comprising the NOₓ group with the use of stored measurement data of a comparative measurement; and optionally
- regenerating the analyte-sensitive layer by contact with an NOₓ-free fluid, by bake-out and/or by incident flow with water vapour.

8. The method according to claim 7, wherein the fluorescence property is selected from: a fluorescence quantum yield, a fluorescence lifetime; a fluorescence intensity, in particular from a fluorescence quenching or a fluorescence increase after a previous fluorescence quenching.

9. The method according to one of claims 7 or 8, wherein the measurement of the fluorescence property comprises a direct detection of an electrical signal of at least one detector or a forming of a quotient from electrical signals which are detected at different excitation wavelengths by at least one detector.

10. The method according to one of claims 7 to 9, wherein the fluorescence property is measured using a portable measuring device, preferably a portable measuring device that is manageable using one hand, and the measuring device comprises a scanning apparatus, which is designed to measure the fluorescence property at least at one fixed wavelength.

11. The method according to one of claims 7 to 10, wherein the analyte is an explosive and the polymer film comprises a polymer which is selected from: poly(benzyl methacrylate); poly(benzyl acrylate); polystyrene; a poly(aryl acrylate); a polyacrylamide; a polymer comprising alkylsubstituted aryl acrylates; poly(benzyl methacrylate)/polystyrene; poly(1,4-bisacryloylpiperazine) and derivatives of poly(1,4-bisacryloylpiperazine); poly(1,4-bisacryloylpiperazine)/poly(2-hydroxyethylmethacrylate); and/or poly(ethylene glycol dimethacrylate)/poly(2-hydroxyethyl methacrylate).

12. A method for producing an analyte-sensitive layer, comprising:
- providing a substrate;
- constructing a polymer film on the substrate;
- applying a detection reagent according to one of claims 1 to 6 to the constructed polymer film,
wherein the analyte comprises an NOₓ group.

13. The production method according to claim 12, wherein the provided substrate has a flat surface, for example is a plate, and the polymer film is constructed at least in portions on one side and/or at least in portions on both sides, or the substrate has a curved surface at least in portions and surrounds a cavity which has at least one inlet opening for feeding the analyte and at least one outlet opening for discharging the analyte, wherein the polymer film is constructed on a substrate surface located in the cavity.

14. An analyte-sensitive layer for an analyte comprising an NOₓ group, comprising a substrate, a polymer layer arranged on the substrate and at least one detection reagent according to one of claims 1 to 6 adsorbed on the polymer layer, wherein a fluorescence intensity of the detection reagent in the presence of the analyte is reduced compared to a fluorescence intensity of the detection reagent in the absence of the analyte, wherein the polymer film constructed on the substrate has a plurality of zones comprising different detection reagents, such that the analyte-sensitive layer has portions of different sensitivity for different analytes comprising an NOₓ group.

15. Use of a detection reagent according to one of claims 1 to 6 and/or of a method according to one of claims 7 to 10 and/or of an analyte-sensitive layer according to claim 14 to monitor a limit value of an explosive.

## Revendications

1. Réactif de détection d'un analyte comprenant un groupe NOₓ, le réactif de détection comprenant une arylamine, et une formule développée de l'arylamine étant choisie parmi la formule développée I : 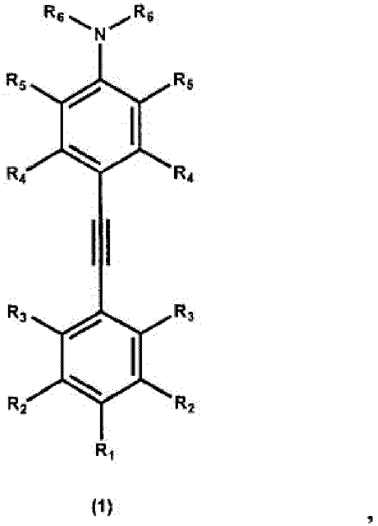 dans laquelle
R₁ est choisi parmi CO₂X ou PhCO₂X, avec X = vinyle, allyle, homoallyle ou aryle, ou
R₁ représente C(O)NX₂ ou PhC(O)NX₂, avec X = H, alkyle, perfluoroalkyle, vinyle, allyle, homoallyle ou aryle ;
R₂, R₃, R₄ et/ou R₅ sont indépendamment choisis parmi H, F, un alkyle ou un aryle ; et
R₆ est choisi parmi un aryle.

2. Réactif de détection selon la revendication 1, dans lequel l'arylamine présente la formule développée 1, et R₁ représente un *N,N-*dialkylamide, ou un phényl-*N,N*-dialkylamide.

3. Réactif de détection selon l'une des revendications précédentes, dans lequel R₆ représente un groupe phényle, et l'arylamine comprend ainsi un motif triphénylamine.

4. Réactif de détection selon la revendication 3, dans lequel le motif triphénylamine est lié par de manière covalente à un groupe phényle dans au moins une position para, et les positions para restantes sont non substituées ou méthylées.

5. Réactif de détection selon la revendication 3, dans lequel la formule développée de l'arylamine est choisie parmi les formules développées 5 et 6 :

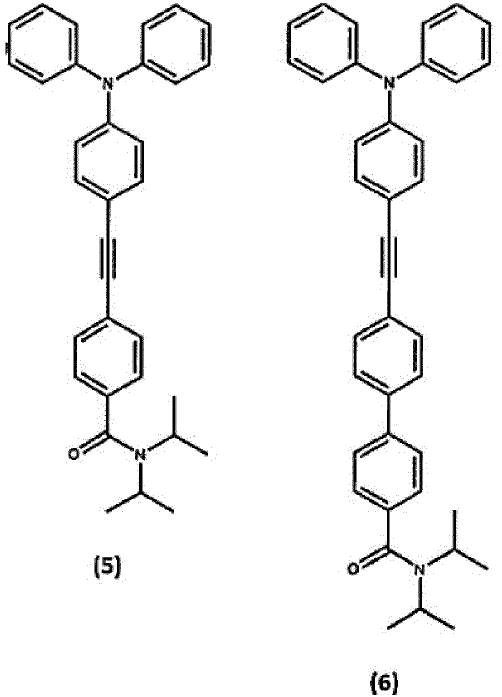

6. Réactif de détection selon la revendication 5, dans lequel l'analyte comprenant le groupe NOₓ est choisi parmi : le TNT, le DNT, le tétryle, le PETN, le NG, l'EGDN, le DNDMB, le nitrate d'ammonium, le RDX et le HMX.

7. Procédé pour la détection d'un analyte présentant un groupe NOₓ, comprenant :
- la fourniture d'une couche sensible à un analyte, comprenant un film polymère sur un matériau support et un réactif de détection lié par adsorption au film polymère, selon l'une des revendications 1 à 6 ;
- l'interaction de l'analyte comprenant le groupe NOₓ avec la couche sensible à un analyte ;
- la mesure d'une propriété de fluorescence d'au moins une partie de la couche sensible à un analyte ;
- le chauffage et/ou la vaporisation d'une quantité définie d'un échantillon qui contient potentiellement l'analyte comprenant le groupe NOₓ;
- l'envoi d'un gaz ou d'un mélange de gaz comprenant la quantité définie d'échantillon chauffée ou vaporisée sur la couche sensible à un analyte de telle sorte que l'analyte comprenant le groupe NOₓ soit enrichi sur, dans et/ou au niveau de la couche sensible à un analyte ;
- la détermination d'une composition et/ou d'une concentration de l'analyte comprenant le groupe NOₓ, par utilisation de données de mesure mémorisées d'une mesure comparative ; et en option
- la régénération de la couche sensible à un analyte par contact avec un fluide exempt de NOₓ, par chauffage et/ou par circulation de vapeur d'eau.

8. Procédé selon la revendication 7, dans lequel la propriété de fluorescence est choisie parmi : un rendement quantique de fluorescence, une durée de vie de fluorescence ; une intensité de fluorescence, en particulier parmi une extinction de fluorescence ou une augmentation de fluorescence après une extinction de fluorescence préalable.

9. Procédé selon l'une des revendications 7 ou 8, dans lequel la mesure de la propriété de fluorescence comprend une détection directe d'un signal électrique provenant d'au moins un détecteur ou une formation d'un quotient de signaux électriques détectés à différentes longueurs d'onde d'excitation provenant d'au moins un détecteur.

10. Procédé selon l'une des revendications 7 à 9, dans lequel la mesure de la propriété de fluorescence est effectuée avec un dispositif de mesure portable, de préférence pouvant être porté d'une main, et le dispositif de mesure comprenant un dispositif de balayage conçu pour mesurer la propriété de fluorescence à au moins une longueur d'onde fixe.

11. Procédé selon l'une des revendications 7 à 10, dans lequel l'analyte est une substance explosive, et le film polymère comprend un polymère choisi parmi : le poly(méthacrylate de benzyle) ; le poly(acrylate de benzyle) ; le polystyrène ; un poly(acrylate d'aryle) ; un polyacrylamide ; un polymère comprenant des acrylates d'aryle à substitution alkyle ; le poly(méthacrylate de benzyle)/polystyrène ; le poly(1,4-bisacryloylpipérazine) et les dérivés du poly(1,4-bisa&cryloylpipérazine) ; le poly(1,4-bisacryloylpipérazine)/poly(méthacrylate de 2-hydroxyéthyle) ; et/ou le poly(diméthacrylate d'éthylèneglycol)/poly(méthacrylate de 2-hydroxyéthyle).

12. Procédé de fabrication d'une couche sensible à un analyte, comprenant :
- la fourniture d'un substrat ;
- la constitution d'un film polymère sur le substrat ;
- l'application sur le film polymère constitué d'un réactif de détection selon l'une des revendications 1 à 6,
dans lequel l'analyte comprend un groupe NOₓ.

13. Procédé de fabrication selon la revendication 12, dans lequel le substrat fourni présente une surface plane, par exemple un disque, et le film polymère est constitué au moins par segments sur une face et/ou par segments sur les deux faces, ou le substrat comprend au moins par segments une surface incurvée et entoure une cavité, qui présente au moins une ouverture d'entrée pour l'amenée de l'analyte et au moins une ouverture de sortie pour l'évacuation de l'analyte, le film polymère étant constitué sur une surface de substrat se trouvant dans la cavité.

14. Couche sensible à un analyte pour un analyte comprenant un groupe NOₓ, comprenant un substrat, une couche polymère disposée sur le substrat, et au moins un réactif de détection adsorbé par la couche polymère selon l'une des revendications 1 à 6, une intensité de fluorescence du réactif de détection en présence de l'analyte étant réduite par rapport à une intensité de fluorescence du réactif de détection en l'absence de l'analyte, le film polymère constitué sur le substrat présentant de multiples zones comprenant différents réactifs de détection de telle sorte que la couche sensible à un analyte présente des segments ayant différentes sensibilités pour différents analytes comprenant un groupe NOₓ.

15. Utilisation d'un réactif de détection selon l'une des revendications 1 à 6 et/ou d'un procédé selon l'une des revendications 7 à 10 et/ou d'une couche sensible à un analyte selon la revendication 14, pour surveiller une valeur limite d'une substance explosive.
